# EUROPEAN PATENT APPLICATION

(11) **EP 1 369 130 A1**
(43) Date of publication of application: **10.12.2003**
(21) Application number: 02705171.3
(22) Date of filing: 14.03.2002
(51) Int. Cl.: A61K 45/00, A61K 31/4178, A61K 31/41, A61K 31/4184, A61K 31/519, A61K 9/00, A61K 9/08, A61K 9/50, A61K 47/02, A61K 47/34, A61P 43/00, A61P 9/00, A61P 9/12

(54) **PROCESS FOR PRODUCING SUSTAINED RELEASE PREPARATION**

(30) Priority: 16.03.2001 JP 2001077157; 19.11.2001 JP 2001353757
(71) Applicant: Takeda Chemical Industries, Ltd., Osaka-shi, Osaka 541-0045 (JP)
(72) Inventor: SHIRAISHI, Keiko, Ikeda-shi, Osaka 563-0026 (JP); YAMAGATA, Yutaka, Kobe-shi, Hyogo 654-0122 (JP); HATA, Yoshio, Shikabe-cho, Kayabe-gun, Hokkaido 041-14 (JP)
(74) Representative: Teipel, Stephan, Dr.
(86) International application number: JP0202404
(87) International publication number: WO02074340

(57) **Abstract**

The present invention relates to a process for producing a sustained-release preparation comprising removing a solvent from an organic solvent solution containing a poorly water-soluble nonpeptidic physiologically active compound, a polyvalent metal compound and a biodegradable polymer, said physiologically active compound dissolved in an amount exceeding the solubility thereof in the organic solvent solution of biodegradable polymer in the absence of the polyvalent metal compound; a sustained-release preparation obtained by the process; a sustained-release solid pharmaceutical composition comprising a nonpeptidic physiologically active substance and a biodegradable polymer, wherein about 0.05% by weight or more, based on the weight of the composition, of a polyvalent metal is present on the surface of the composition; etc. In the sustained-release preparation, the poorly water-soluble nonpeptidic physiologically active compound is uniformly distributed and the sustained-release effect of the poorly water-soluble nonpeptidic physiologically active compound can be stably achieved. Moreover, a pharmaceutical composition comprising a nonpeptidic physiologically active substance wherein the initial burst release of the physiologically active substance is efficiently controlled, a process for producing the same, etc. are provided.

## Description

### Technical Field

The present invention relates to a process for producing a sustained-release preparation of a poorly water-soluble nonpeptidic physiologically active compound. Further, the present invention relates to a sustained-release solid pharmaceutical composition which efficiently suppresses the initial burst release of a nonpeptidic physiologically active substance, and a process for producing the same.

### Background Art

During a process for preparing a sustained-release preparation using a biodegradable polymer, a physiologically active compound is added to a solution in which the biodegradable polymer is dissolved. There are advantages that, upon this, when a physiologically active compound is dissolved in the solution, uniformity (uniform distribution) of the physiologically active compound in a preparation is ensured and, when a preparation is for injection, sterilization by filtration of the solution with a filter becomes possible. Regarding a water-soluble physiologically active compound, a process for producing microspheres by an O/W type in-solution drying method is disclosed in JP-A 04-046115 and JP-A 04-046116. Regarding a poorly water-soluble physiologically active compound, a process for producing bromperidol-containing microspheres is disclosed, for example, in USP 5,656,299.

In addition, there has been a problem that, in a sustained-release preparation of a physiologically active substance using a biodegradable polymer, the considerable release of the physiologically active substance occurs frequently at an initial period of administration. Regarding a sustained-release preparation of a peptidic physiologically active substance, Journal of Controlled Release, vol. 52, pp 311-320 (1998) discloses that the initial burst release of ovalbumin is suppressed by converting a surfactant in an outer water phase from polyvinyl alcohol into polyvinylpyrrolidone. On the other hand, regarding a nonpeptidic physiologically active substance, although a process for producing bromperidol-containing microspheres is reported in USP 5,656,299, a method of suppressing the initial burst release of a drug is not disclosed.

In spite of the aforementioned methods, regarding a poorly water-soluble physiologically active compound, there is no method for dissolving a drug in an amount higher than the solubility of the physiologically active compound in a biodegradable polymer solution and, when a solubility is very low, in order to increase the concentration of a drug higher than the concentration sufficient for the industrial production process, it is required to adopt an S/O/W type in-solution drying method in which a fine powder of the drug is dispersed. In particular, when prepared for injection via an S/O type emulsion, it is required to use a sterile bulk of physiologically active compound. Under these backgrounds, there is a demand for a method for dissolving a poorly water-soluble nonpeptidic physiologically active compound in an organic solvent solution of a biodegradable polymer in an amount higher than the solubility of the compound.

Furthermore, although there is a report that the initial burst release of ovalbumin is suppressed under the extremely limited condition, a sufficient method for suppressing the initial burst release of a nonpeptidic physiologically active substance having a greatly different property is not disclosed. Especially, there is a demand for an effective method for suppressing the initial burst release of the physiologically active substance, in a sustained-release preparation containing a nonpeptidic physiologically active substance.

### Disclosure of the Invention

In order to solve the aforementioned problems, the present inventors have studied intensively and, as a result, found that addition of a polyvalent metal compound allows a poorly water-soluble nonpeptidic physiologically active compound to be solved in an organic solvent solution of a biodegradable polymer in an amount higher than the solubility of the compound.

Further, the present inventors have found that, in a composition containing a nonpeptidic physiologically active substance and a biodegradable polymer, when about 0.05% by weight, based on the weight of the composition, or more of a polyvalent metal is present on the surface of the composition, the initial burst release of the nonpeptidic physiologically active substance can be unexpectedly suppressed. In addition, when the present inventors have first practiced a method for removing an organic solvent from an emulsion obtained by mixing an organic solvent solution or suspension containing a nonpeptidic physiologically active substance and a biodegradable polymer, and a water phase having about 0.1 to about 80 mM of a polyvalent metal ion, it has been found that this method is unexpectedly suitable as a method for producing the aforementioned composition. Based on these findings, the present invention was completed.

That is, the present invention relates to:
(1) A process for producing a sustained-release preparation, which comprises removing a solvent from an organic solvent solution containing a poorly water-soluble nonpeptidic physiologically active compound, a polyvalent metal compound and a biodegradable polymer, said physiologically active compound dissolved in an amount exceeding the solubility thereof in the organic solvent solution of biodegradable polymer in the absence of the polyvalent metal compound;
(2) The process according to the above (1), wherein a molecular weight of the poorly water-soluble nonpeptidic physiologically active compound is about 2,000 or lower;
(3) The process according to the above (1), wherein the poorly water-soluble nonpeptidic physiologically active compound has a functional group which can form a complex salt with the polyvalent metal compound;
(4) The process according to the above (3), wherein the functional group is a group which can donate a covalent electron pair;
(5) The process according to the above (3), wherein the functional group is a group having one or more hetero atom(s) selected from an oxygen atom, a nitrogen atom and a sulfur atom;
(6) The process according to the above (3), wherein the functional group is a group selected from (i) a carboxyl group, (ii) an imidazolyl group, (iii) a mercapto group, (iv) an amino group, (v) a tetrazolyl group, (vi) a trifluoromethanesulfonamido group, (vii) a phosphono group, (viii) a sulfo group and (ix) an optionally substituted 5 to 7-membered monocyclic heterocyclic residue, which has one or more hetero atom(s) selected from an oxygen atom, a nitrogen atom and a sulfur atom;
(7) The process according to the above (1), wherein the poorly water-soluble nonpeptidic physiologically active compound has a functional group having a pKa of about 2.5 to about 6;
(8) The process according to the above (1), wherein the poorly water-soluble nonpeptidic physiologically active compound is a compound having angiotensin II antagonistic activity, a prodrug thereof, or a salt thereof;
(9) The process according to the above (8), wherein the compound having angiotensin II antagonistic activity is a compound having an oxygen atom in the molecule;
(10) The process according to the above (8), wherein the compound having angiotensin II antagonistic activity is a compound having an ether linkage or a carbonyl group;
(11) The process according to the above (8), wherein the compound having angiotensin II antagonistic activity is a compound represented by the formula (I): 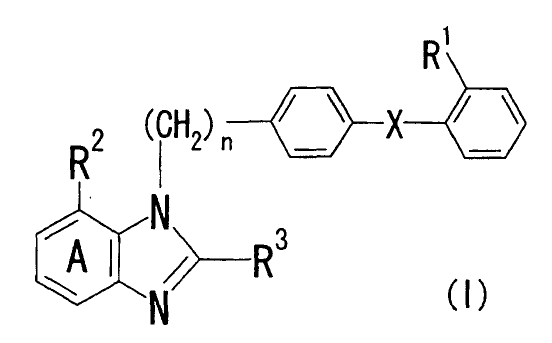 wherein R¹ denotes a group which can form an anion or a group which can be changed into such a group, X denotes a bond or a spacer having 2 or less atoms at the straight chain part thereof, n denotes 1 or 2, a ring A denotes a benzene ring which may be further substituted, R² denotes a group which can form an anion or a group which can be changed into such a group, and R³ denotes an optionally substituted hydrocarbon residue, wherein the hydrocarbon residue may bind via a hetero atom;
(12) The process according to the above (8), wherein the compound having angiotensin II antagonistic activity, a prodrug thereof, or a salt thereof is Losartan, Losartan potassium, Eprosartan, Candesartan cilexetil, Candesartan, Valsartan, Telmisartan, Irbesartan, Olmesartan, Olmesartan medoxomil or Tasosartan;
(13) The process according to the above (8), wherein the compound having angiotensin II antagonistic activity is 2-ethoxy-1-[[2'-(1H-tetrazol-5-yl)biphenyl-4-yl]methyl]benzimidazole-7-carboxylic acid;
(14) The process according to the above (8), wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate;
(15) The process according to the above (8), wherein the compound having angiotensin II antagonistic activity is 2-ethoxy-1-[[2'-(2,5-dihydro-5-oxo-1,2,4-oxadiazol-3-yl)biphenyl-4-yl]methyl]benzimidazole-7-carboxylic acid;
(16) The process according to the above (1), wherein the biodegradable polymer is an α-hydroxycarboxylic acid polymer;
(17) The process according to the above (16), wherein the α-hydroxycarboxylic acid polymer is a lactic acid-glycolic acid copolymer;
(18) The process according to the above (17), wherein a molar ratio of lactic acid and glycolic acid in the lactic acid-glycolic acid copolymer is 100/0 to 40/60;
(19) The process according to the above (16), wherein a weight average molecular weight of the polymer is 3,000 to 50,000;
(20) The process according to the above (1), wherein the sustained-release preparation is for injection;
(21) The process according to the above (1), wherein the polyvalent metal is zinc;
(22) The process according to the above (21), wherein the polyvalent metal compound comprises one or two of zinc acetate and zinc oxide;
(23) The process according to the above (1), wherein a molar ratio of the polyvalent metal compound relative to the poorly water-soluble nonpeptidic physiologically active compound in the organic solvent solution is 1/10 to 10/1;
(24) The process according to the above (1), wherein the concentration of the poorly water-soluble nonpeptidic physiologically active compound in the organic solvent solution is about 0.5 to about 70% by weight;
(25) The process according to the above (1), wherein the concentration of the biodegradable polymer in the organic solvent solution is about 0.5 to about 70% by weight;
(26) A sustained-release preparation obtainable by the process according to the above (1);
(27) The sustained-release preparation according to the above (26), wherein the poorly water-soluble nonpeptidic physiologically active compound is a compound having angiotensin II antagonistic activity, a prodrug thereof, or a salt thereof;
(28) A medicine comprising the sustained-release preparation according to the above (26);
(29) The medicine according to the above (28), which is an agent for preventing or treating circulatory diseases;
(30) The medicine according to the above (28), which is an agent for preventing or treating hypertension, abnormality of diurnal blood pressure variation or organ dysfunction;
(31) An organic solvent solution, which comprises a poorly water-soluble nonpeptidic physiologically active compound, a polyvalent metal compound and a biodegradable polymer, said physiologically active compound dissolved in an amount exceeding the solubility thereof in the organic solvent solution of a biodegradable polymer in the absence of the polyvalent metal compound;
(32) Use of a polyvalent metal compound for increasing the solubility of a poorly water-soluble nonpeptidic physiologically active compound in an organic solvent solution of a biodegradable polymer;
(33) A method for increasing the solubility of a poorly water-soluble nonpeptidic physiologically active compound in an organic solvent solution containing a biodegradable polymer, an organic solvent and the poorly water-soluble nonpeptidic physiologically active compound, by using a polyvalent metal compound;
(34) A sustained-release solid pharmaceutical composition comprising a nonpeptidic physiologically active substance and a biodegradable polymer, wherein about 0.05% by weight based on the weight of the composition, or more of a polyvalent metal is present on the surface of the composition;
(35) The sustained-release solid pharmaceutical composition according to the above (34), wherein the initial burst release of the nonpeptidic physiologically active substance is suppressed at about 60% or less as compared with the absence of the polyvalent metal on the surface;
(36) The sustained-release solid pharmaceutical composition according to the above (34), wherein a molecular weight of the nonpeptidic physiologically active substances is about 2,000 or less;
(37) The sustained-release solid pharmaceutical composition according to the above (34), wherein the nonpeptidic physiologically active substance is a poorly water-soluble compound;
(38) The sustained-release solid pharmaceutical composition according to the above (34), wherein the nonpeptidic physiologically active substance is a compound having angiotensin II antagonistic activity, a prodrug thereof, or a salt thereof;
(39) The sustained-release solid pharmaceutical composition according to the above (38), wherein the compound having angiotensin II antagonistic activity is a compound having an oxygen atom in a molecule;
(40) The sustained-release solid pharmaceutical composition according to the above (38), wherein the compound having angiotensin II antagonistic activity is a compound having an ether linkage or a carboxyl group;
(41) The sustained-release solid pharmaceutical composition according to the above (38), wherein the compound having angiotensin II antagonistic activity is a compound represented by the formula (I): 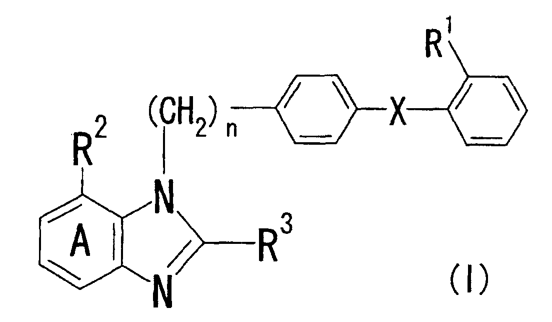 wherein R¹ denotes a group which can form an anion or a group which can be changed into such a group, X denotes a bond or a spacer having 2 or less atoms at the straight chain part thereof, n denotes 1 or 2, a ring A denotes a benzene ring which may be further substituted, R² denotes a group which can form an anion or a group which can be changed into such a group, and R³ denotes an optionally substituted hydrocarbon residue, wherein the hydrocarbon residue may bind via a hetero atom;
(42) The sustained-release solid pharmaceutical composition according to the above (38), wherein the compound having angiotensin II antagonistic activity, a prodrug thereof, or a salt thereof is Losartan, Losartan potassium, Eprosartan, Candesartan cilexetil, Candesartan, Valsartan, Telmisartan, Irbesartan, Ormesartan, Ormesartan medoxomil or Tasosartan;
(43) The sustained-release solid pharmaceutical composition according to the above (38), wherein the compound having angiotensin II antagonistic activity is 2-ethoxy-1-[[2'-(1H-tetrazol-5-yl)biphenyl-4-yl]methyl]benzimidazole-7-carboxylic acid;
(44) The sustained release solid pharmaceutical composition according to the above (38), wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate;
(45) The sustained-release solid pharmaceutical composition according to the above (38), wherein the compound having angiotensin II antagonistic activity is 2-ethoxy-1-[[2'-(2,5-dihydro-5-oxo-1,2,4-oxadiazol-3-yl)biphenyl-4-yl]methyl]benzimidazole-7-carboxylic acid;
(46) The sustained-release solid pharmaceutical composition according to the above (34), wherein the biodegradable polymer is an α-hydroxycarboxylic acid polymer;
(47) The sustained-release solid pharmaceutical composition according to the above (46), wherein the α-hydroxycarboxylic acid polymer is a lactic acid-glycolic acid copolymer;
(48) The sustained-release solid pharmaceutical composition according to the above (47), wherein a molar ratio of lactic acid and glycolic acid in the lactic acid-glycolic acid copolymer is 100/0 to 40/60;
(49) The sustained-release solid pharmaceutical composition according to the above (46), wherein a weight average molecular weight of the polymer is about 3,000 to about 50,000;
(50) The sustained-release solid pharmaceutical composition according to the above (34), the polyvalent metal is a divalent metal;
(51) The sustained-release solid pharmaceutical composition according to the above (50), wherein the divalent metal is zinc;
(52) The sustained-release solid pharmaceutical composition according to the above (34), which is for injection;
(53) The sustained-release solid pharmaceutical composition according to the above (34), which is microcapsules;
(54) A process for producing the sustained-release solid pharmaceutical composition according to the above (34), which comprises removing an organic solvent from an emulsion obtained by mixing an organic solvent solution or suspension containing a nonpeptidic physiologically active substances and a biodegradable polymer, and a water phase containing polyvalent metal ion in concentration of about 0.1 to about 80 mM;
(55) The process for producing the sustained-release solid pharmaceutical composition according to the above (34), which comprises removing an organic solvent from an emulsion obtained by mixing an organic solvent solution containing a nonpeptidic physiologically active substance, a polyvalent metal and a biodegradable polymer, said physiologically active substance dissolved in an amount exceeding the solubility thereof in the organic solvent solution of the biodegradable polymer in the absence of the polyvalent metal, and a water phase containing polyvalent metal ion in concentration of about 0.1 to about 80mM;
(56) The process according to the above (54) or (55), wherein the polyvalent metal ion is zinc;
(57) The process according to the above (55), wherein the polyvalent metal is used as one or two of polyvalent metal compound(s) selected from zinc acetate and zinc oxide;
(58) A sustained-release solid pharmaceutical composition obtainable by the process according to the above (54) or (55);
(59) The sustained-release solid pharmaceutical composition according to the above (38), which is an agent for preventing or treating circulatory disease;
(60) The sustained-release solid pharmaceutical composition according to the above (38), which is an agent for preventing or treating hypertension, abnormality of diurnal blood pressure variation or organ dysfunction;
(61) A method of suppressing the initial burst release of a nonpeptidic physiologically active substance, which comprises, in a solid pharmaceutical composition containing a nonpeptidic physiologically active substance and a biodegradable polymer, allowing about 0.05% by weight, based on the weight of the composition, or more of a polyvalent metal to be present on the surface of the composition; and the like.

### A. Regarding nonpeptidic physiologically active substances

A physiologically active compound in the present invention means a physiologically active compound effective to prophylaxis and therapeutics for diseases and illness of mammals (e.g., human being, cattle, pigs, dogs, cats, mice, rats, rabbits and the like), and a nonpeptidic compound is preferable. Inter alia, a physiologically active compound having a molecular weight of about 200 to about 3000 is preferable, a physiologically active compound having a molecular weight of about 2000 or smaller is more preferable and, particularly, a physiologically active compound having a molecular weight of about 300 to about 2000 is preferable.

In addition, a physiologically active compound in the present invention is preferably a "poorly water-soluble nonpeptidic physiologically active compound".

As used herein, "poorly water-soluble" means that the amount of water or a physiological saline solution required for dissolving a solute of 1 g within 30 minutes under conditions of strongly stirring for 30 seconds every 5 minutes at 20 ± 5°C is at least 100 mL or more, preferably 1,000 mL or more, more preferably 10,000 mL or more.

The "nonpeptidic physiologically active compound" used in the present invention may be any compounds which are pharmacologically useful, preferably synthetic organic compounds.

The "nonpeptidic physiologically active compound" includes compounds having a receptor agonistic or antagonistic activity, an enzyme inhibiting activity, a transporter promoting or inhibiting activity, or the like.

The receptor to which the "nonpeptidic physiologically active compound" exhibits an agonistic or antagonistic activity may be present on the surface of cells or inside of cells. As the receptors on the surface of cells, there are ion channel coupled type, G protein coupled type and enzyme coupled type. As the kinds of ligands for the receptor, there are small peptides, proteins, amino acids, nucleotides, steroids, fatty acid derivatives, nitrogen monoxide, carbon monoxide and the like. Examples of the receptor include luteinizing hormone-releasing hormone (LH-RH) receptor, thyrotropin releasing hormone (TRH) receptor, corticotropin releasing hormone (CRF) receptor, endorphin receptor, Substance P receptor, neurotensin receptor, thyroid-stimulating hormone (TSH) receptor, lactation hormone (PRL) receptor, follicle-stimulating hormone (FSH) receptor, luteinizing hormone (LH) receptor, adrenocorticotropic hormone (ACTH) receptor, PPAR-α receptor, PPAR-γ receptor, testosterone receptor and the like.

The enzyme which is inhibited by the "nonpeptidic physiologically active compound" includes blood coagulation system enzymes, fibrinogenolysis system enzymes, digestive enzymes, phosphorylation enzymes, metabolism enzymes, antioxidant enzymes and the like. Examples of the enzyme include monoamine oxidase (MAO), angiotensin converting enzyme, HMG-CoA reductase, cholesterol esterification enzyme (ACAT), cycloxygenase (COX), trypsin, α-chymotrypsin, kallikrein, β-glactosidase, elastase, thrombomodulin, thrombin, blood coagulation factors (factor I to factor X), Protein C, Protein S, plasmin, plasminogen activator, urokinase, Protein Kinase C, tyrosine kinase, cytochromes p450 (3A4, 1A, 2C, 2D, etc.) and superoxide dismutase (SOD), and the like. The enzyme which is inhibited by the "nonpeptidic physiologically active compound" includes enzymes derived from human cells, bacteria, phages, viruses and the like. The nonpeptidic physiologically active compound exhibiting the inhibiting activity is expected to have an antibacterial or anti-virus activity. Examples of the enzyme include cross-linking forming enzyme transpeptidase, penicillin binding proteins (PBP-1A, PBP-1B, PBP-2, PBP-3, PBP-4, PBP-5, PBP-6), neuraminidase, aminopeptidase A, aminopeptidase B, α-amylase, β-lactamase, reverse transcriptase inhibiting agent and the like.

The transporter which is promoted or inhibited by the "nonpeptidic physiologically active compound" includes passive or active ion channel, glucose transporter, peptide transporter, p-glycoprotein and the like. Examples of the transporter include voltage-dependent sodium channel, potassium-dependent sodium channel, potassium-dependent calcium channel, potassium channel, chloro ion channel, gastric mucosal proton pump (H⁺, K⁺-ATPase), glucose transporters (GLUT1, GLUT2, GLUT3, GLUT4), PEPT 1, MDR1, MDR2, MRP, cMOAT, ACT1 and the like.

The "nonpeptidic physiologically active compound" used in the present invention has a functional group which can form a complex salt with a polyvalent metal compound (polyvalent metal ion). The reaction between a polyvalent metal compound and an organic compound has been investigated, for example, in detail in the protein purification field, and details are found in Purification of proteins by IMCA (authored by Eugene Sulkowski, Trends in Biotechnology, Vol3, No. 1, pp 1 to 7 (1985)), etc. According to this publication, there is described that a functional group which can donate a covalent electron pair to a polyvalent metal ion such as zinc, copper, cobalt, nickel and the like can form coordinate bond with the polyvalent metal. Examples of atoms constituting a functional group which can donate a covalent electron pair include a nitrogen atom, a sulfur atom and an oxygen atom. That is, as the functional group, a group having 1 or more (preferably 1 to 5) hetero atom(s) selected from a nitrogen atom, a sulfur atom and an oxygen atom is preferable. Examples of the functional group which can donate a covalent electron pair include (1) a carboxyl group, (2) an imidazolyl group, (3) a mercapto group (-SH), (4) an amino group, (5) a tetrazolyl group, (6) a trifluoromethanesulfonamido group (-NHSO₂CF₃), (7) a phosphate group, (8) a sulfonate group, and (9) an optionally substituted 5- to 7-membered (preferably 5- to 6-membered) monocyclic heterocyclic residue having 1 or more (preferably 1 to 5) hetero atom(s) selected from an oxygen atom, a nitrogen atom and a sulfur atom, and the like.

The "optionally substituted 5- to 7-membered monocyclic heterocyclic residue having 1 or more hetero atom(s) selected from an oxygen atom, a nitrogen atom and a sulfur atom" includes the same residue as an "optionally substituted 5- to 7-membered (preferably 5- to 6-membered) monocyclic heterocyclic residue containing 1 or more of N, S and O " defined with respect to R¹ of a compound represented by the formula (I) as described hereinafter.

A pKa of the functional group possessed by the "nonpeptidic physiologically active compound" is preferably about 2.5 to about 6, more preferably about 3 to about 5.

The "nonpeptidic physiologically active compound" used in the present invention preferably includes a compound having angiotensin II antagonistic activity (compound having angiotensin II receptor antagonistic activity), a prodrug thereof, or a salt thereof.

In the present specification, the angiotensin II antagonistic activity is to inhibit competitively or non-competitively binding of angiotensin II to the angiotensin II receptors on a cellular membrane so as to reduce potent vasoconstrictive action or vascular smooth muscle proliferation action induced by angiotensin II and to ameliorate the symptom of hypertension.

As the compound having angiotensin II antagonistic activity used in the present invention, a nonpeptidic compound having the antagonistic activity, which has an advantage of a long-acting time, is preferable. As the compound having angiotensin II antagonistic activity, a compound having an oxygen atom in the molecule is preferable. Inter alia, a compound having an ether linkage or a carbonyl group (the carbonyl group may form a hydroxy group by resonation) is preferable, a compound having an ether linkage or a ketone derivative is more preferable, and an ether derivative is particularly preferable. In addition, as the compound having angiotensin II antagonistic activity, a compound which can form an anion or a group which can be changed into such a group is preferable.

The nonpeptidic compound having angiotensin II antagonistic activity is not particularly limited, but imidazole derivatives are disclosed in JP-A 56-71073, JP-A 56-71074, JP-A 57-98270, JP-A 58-157768, USP 4,355,040 and USP 4,340,598, improved imidazole derivatives are disclosed in EP-253310, EP-291969, EP-324377, EP-403158, WO-9100277, JP-A 63-23868 and JP-A 1-117876, pyrrole, pyrazole and triazole derivatives are disclosed in USP 5,183,899, EP-323841, EP-409332 and JP-A 1-287071, benzimidazole derivatives are disclosed in USP 4,880,804, EP-0392317, EP-0399732, EP-0400835, EP-425921, EP-459136 and JP-A 3-63264, azaindene derivatives are disclosed in EP-399731, pyrimidone derivatives are disclosed in EP-407342, quinazoline derivatives are disclosed in EP-411766, xanthine derivatives are disclosed in EP-430300, condensed imidazole derivatives are disclosed in EP-434038, pyrimidinedione derivatives are disclosed in EP-442473, thienopyridone derivatives are disclosed in EP-443568, and heterocyclic compounds are disclosed in EP-445811, EP-483683, EP-518033, EP-520423, EP-588299, and EP-603712. Further, representatives of the above compounds are described in Journal of Medicinal Chemistry, vol. 39, No. 3, pp625-656, 1996. As the nonpeptidic compound having angiotensin II antagonistic activity or a salt thereof, in addition to compounds described in the aforementioned known publications, any nonpeptidic compounds having the angiotensin II antagonistic activity may be used. Inter alia, Losartan (DuP753), Losartan potassium, Eprosartan (SK & F108566), Candesartan cilexetil (TCV-116), Valsartan (CGP-48933), Telmisartan (BIBR277), Irbesartan (SR47436), Tasosartan (ANA-756), Olmesartan medoxomil and metabolically active substances thereof (Candesartan, Olmesartan etc.) are preferably used.

In addition, as the nonpeptidic compound having angiotensin II antagonistic activity, for example, a benzimidazole derivative represented by the formula (I): 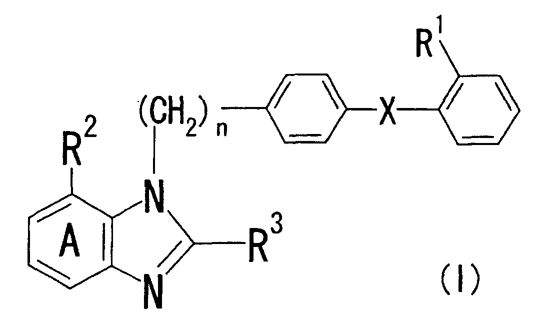 wherein R¹ denotes a group which can form an anion or a group which can be changed into such a group, X denotes a bond or a spacer having 2 or less atoms at the straight chain part thereof, n denotes 1 or 2, a ring A denotes a benzene ring which may be further substituted, R² denotes a group which can form an anion or a group which can be changed into such a group, and R³ denotes an optionally substituted hydrocarbon residue, wherein the hydrocarbon residue may bind via a hetero atom (a hydrocarbon residue which may bind via a hetero atom and may be substituted; preferably, a hydrocarbon residue which may be substituted and binds via an oxygen atom)), or a salt thereof is preferably used.

Examples of the group which can form an anion as R¹ (group having a hydrogen atom which can be released as a proton) in the above formula (I) include (1) a carboxyl group, (2) a tetrazolyl group, (3) a trifluoromethanesulfonamido group (-NHSO₂CF₃), (4) a phosphate group, (5) a sulfonate group, and (6) an optionally substituted 5 to 7-membered (preferably 5 to 6-membered) monocyclic heterocyclic residue containing 1 or more of N, S and O.

Examples of the "optionally substituted 5 to 7-membered (preferably 5 to 6-membered) monocyclic heterocyclic residue containing 1 or more of N, S and O" include: 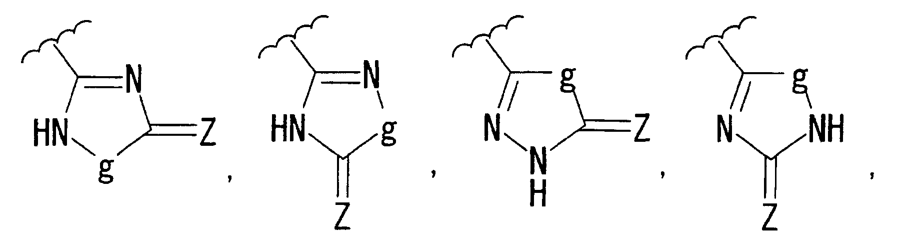 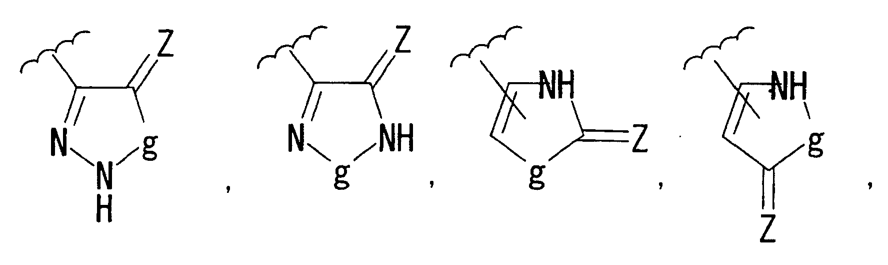 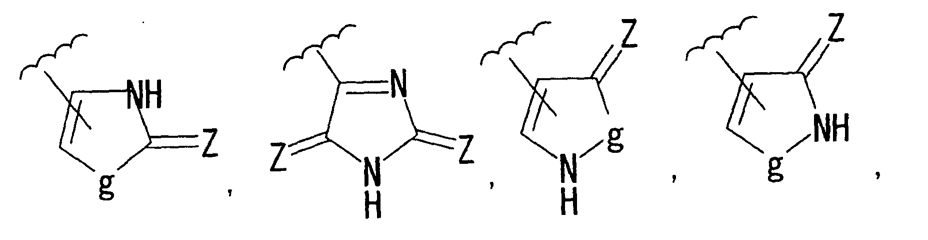 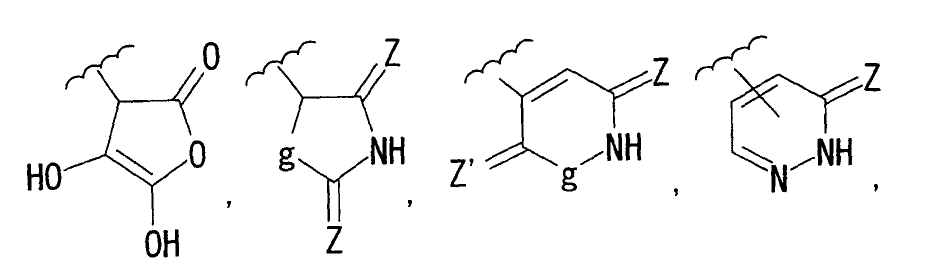 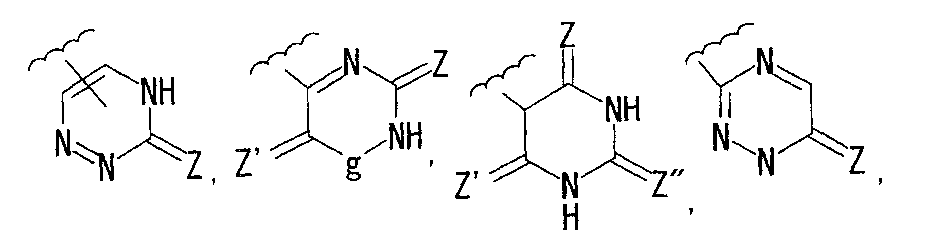 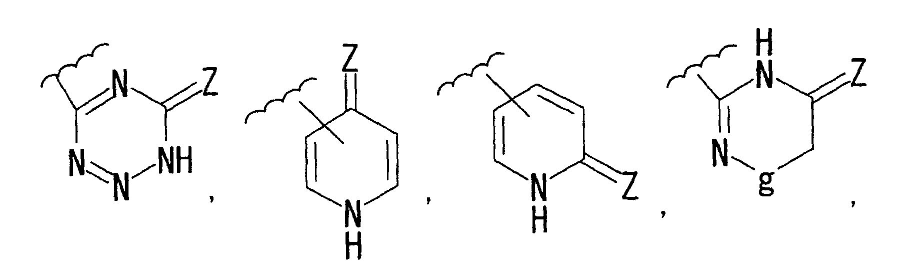 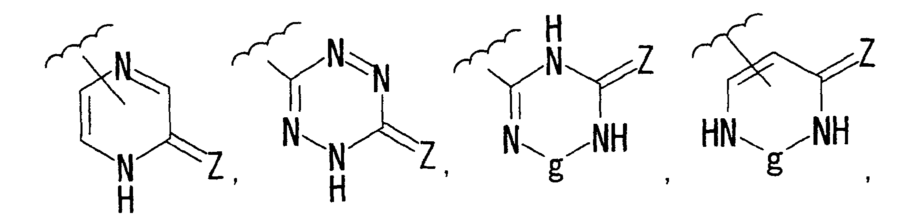 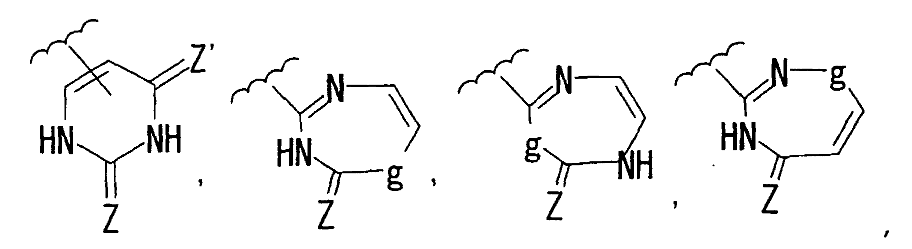 etc. The chemical bond between the heterocyclic ring residue represented by R¹ and the phenyl group to which said heterocyclic ring residue binds may be a carbon-carbon bond as shown above, or a nitrogen-carbon bond via one of the several nitrogen atoms when the symbol g is -NH-, etc. in the above formulas.

For example, when R¹ is represented by the formula: 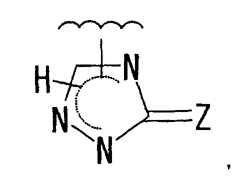 its specific embodiments are 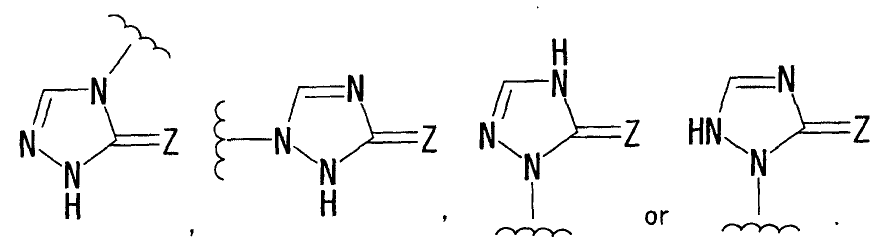 Other examples of R¹ binding through a nitrogen atom include 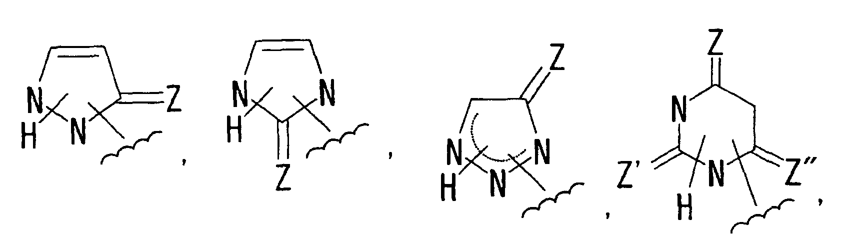 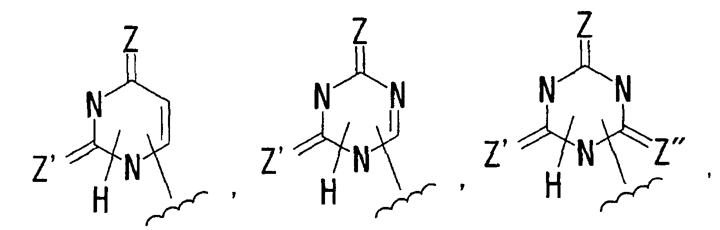 etc.

In the above formulas, g denotes -CH₂-, -NH-, -O- or -S(O)ₘ-, > = Z, >=Z' and >=Z" denote a carbonyl group, a thiocarbonyl group and an optionally oxidized sulfur atom (e.g. S, S(O), S(O)₂, etc.) (preferably a carbonyl or thiocarbonyl group, more preferably a carbonyl group), respectively, and m denotes an integer of 0, 1 or 2.

Preferred examples of the heterocyclic ring residue represented by R¹ include a heterocyclic ring residue simultaneously having -NH- or -OH group as proton donor and a carbonyl group, a thiocarbonyl group, a sulfinyl group, etc. as proton acceptor, such as an oxadiazolone ring, an oxadiazolothione ring or an thiadiazolone ring, etc. While the heterocyclic ring residue represented by R¹ may form a condensed ring by connecting the substituents on the heterocyclic ring, it is preferably 5- to 6-membered ring residue, more preferably 5-membered ring residue.

As a heterocyclic residue represented by R¹, a group represented by the formula: 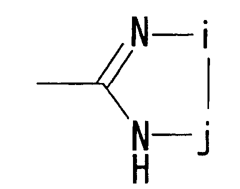 wherein i denotes -O- or -S-, j denotes >= O, >= S or >=S(O)ₘ, and m is as defined above (inter alia, 2,5-dihydro-5-oxo-1,2,4-oxadiazol-3-yl, 2,5-dihydro-5-thioxo-1,2,4-oxadiazol-3-yl, 2,5-dihydro-5-oxo-1,2,4-thiadiazol-3-yl, particularly, 2,5-dihydro-5-oxo-1,2,4-oxadiazol-3-yl) is preferable.

Further, the following tautomeric isomers are present in the above heterocyclic ring residue (R¹). For example, in 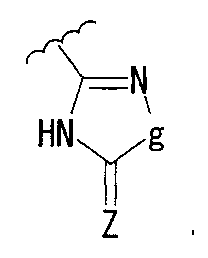 when Z is 0 and g is 0, 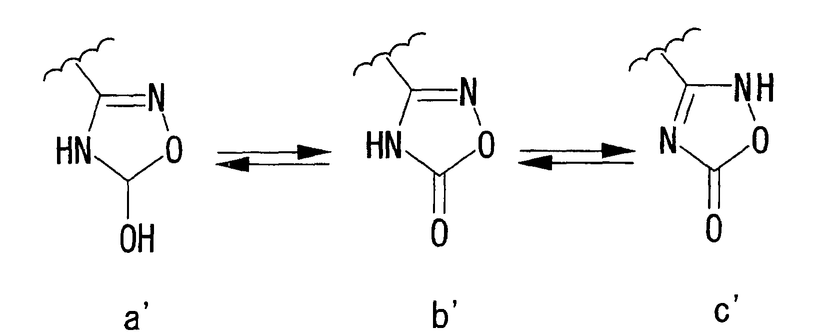 the three tautomeric isomers a', b' and c' exist and a group of the formula: 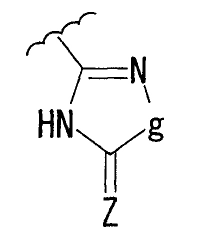 include all of the above a', b' and c'.

The group capable of forming an anion as R¹ may be protected by an optionally substituted lower (C₁₋₄) alkyl group, an acyl group (e.g., lower (C₂₋₅) alkanoyl, benzoyl, etc.), etc. at its possible position.

Examples of the optionally substituted lower (C₁₋₄) alkyl group include (1) a lower (C₁₋₄) alkyl group optionally substituted with one to three phenyl groups which may have halogen atom, nitro, lower (C₁₋₄) alkyl, lower (C₁₋₄) alkoxy, etc. (e.g., methyl, triphenylmethyl, p-methoxybenzyl, p-nitrobenzyl, etc.); (2) a lower (C₁₋₄) alkoxy-lower (C₁₋₄) alkyl group (e.g., methoxymethyl, ethoxymethyl, etc.); (3) a group of the formula: -CH(R⁴)-OCOR⁵ wherein R⁴ is (a) a hydrogen, (b) a straight or branched lower C₁₋₆ alkyl group (e.g., methyl, ethyl, n-propyl, isopropyl, n-butyl, isobutyl, t-butyl, n-pentyl, isopentyl, neopentyl, etc.), (c) a straight or branched lower C₂₋₆ alkenyl group or (d) a C₃₋₈ cycloalkyl group (e.g., cyclopentyl, cyclohexyl, cycloheptyl, etc.), and R⁵ is (a) a straight or branched lower C₁₋₆ alkyl group (e.g., methyl, ethyl, n-propyl, isopropyl, n-butyl, isobutyl, sec-butyl, t-butyl, n-pentyl, isopentyl, neopentyl, etc.), (b) a straight or branched lower C₂₋₆ alkenyl group, (c) a lower C₁₋₃ alkyl group substituted with a C₃₋₈ cycloalkyl group (e.g., cyclopentyl, cyclohexyl, cycloheptyl, etc.) or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as benzyl, p-chlorobenzyl, phenethyl, cyclopentylmethyl, cyclohexylmethyl, etc., (d) a lower C₂₋₃ alkenyl group substituted with a C₃₋₈ cycloalkyl or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as cinnamyl, etc. having an alkenyl moiety such as vinyl, propenyl, allyl, isopropenyl, etc., (e) an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as phenyl, p-tolyl, naphthyl, etc., (f) a straight or branched lower C₁₋₆ alkoxy group (e.g., methoxy, ethoxy, n-propoxy, isopropoxy, n-butoxy, isobutoxy, sec-butoxy, t-butoxy, n-pentyloxy, isopentyloxy, neopentyloxy, etc.), (g) a straight or branched lower C₂₋₈ alkenyloxy group (e.g., allyloxy, isobutenyloxy, etc.), (h) a C₃₋₈ cycloalkyloxy group (e.g., cyclopentyloxy, cyclohexyloxy, cycloheptyloxy, etc.), (i) a lower C₁₋₃ alkoxy group substituted with a C₃₋₈ cycloalkyl (e.g., cyclopentyl, cyclohexyl, cycloheptyl, etc.) or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as benzyloxy, phenethyloxy, cyclopentylmethoxy, cyclohexylmethoxy, etc. having an alkoxy moiety such as methoxy, ethoxy, n-propoxy, isopropoxy, etc., etc.), (j) a lower C₂₋₃ alkenyloxy group substituted with a C₃₋₈ cycloalkyl (e.g., cyclopentyl, cyclohexyl, cycloheptyl, etc.) or an optionally substituted aryl group (e.g., a phenyl group or a naphthyl group, etc., optionally having a halogen atom, a nitro, a lower (C₁₋₄) alkyl, a lower (C₁₋₄) alkoxy, etc.) such as cinnamyloxy, etc. having an alkenyloxy moiety such as vinyloxy, propenyloxy, allyloxy, isopropenyloxy, etc. or (k) an optionally substituted aryloxy group (e.g., a phenoxy group, a naphthoxy group, etc., optionally having a halogen atom, a nitro, a lower (C₁₋₄) alkyl, a lower (C₁₋₄) alkoxy, etc.) such as phenoxy, p-nitrophenoxy, naphthoxy, etc.; etc.

The group capable of forming an anion as R¹ may be substituted, in addition to the above protective group such as an optionally substituted lower (C₁₋₄) alkyl group or an acyl group (e.g., lower (C₂₋₅) alkanoyl, benzoyl, etc.), etc., with an optionally substituted lower (C₁₋₄) alkyl group (e.g. an optionally substituted lower (C₁₋₄) alkyl group similar to the "optionally substituted lower (C₁₋₄) alkyl group" exemplified as a protective group for the above group capable of forming an anion as R¹), a halogen atom, a nitro, a cyano, a lower (C₁₋₄) alkoxy, an amino optionally substituted with 1 to 2 lower (C₁₋₄) alkyl groups, etc., at the possible position.

In the above formula, the group which can be changed into the group capable of forming an anion (a group having a hydrogen atom capable of leaving as proton) as R¹ may be a group convertible into a group capable of forming an anion under biological or physiological conditions (for example, in vivo reaction, etc. such as oxidation, reduction, hydrolysis, etc. by in vivo enzyme, etc.) [so called prodrug], or the group convertible into a group capable of forming an anion represented by R¹ may be a group chemically convertible into a group capable of forming an anion, such as cyano, N-hydroxycarbamimidoyl group (-C(=N-OH)-NH₂), a group selected from the class consisting of (1) a carboxyl group, (2) a tetrazolyl group, (3) a trifluoromethanesulfonamide group (-NHSO₂CF₃), (4) a phosphono group, (5) a sulfo group and (6) an optionally substituted monocyclic 5- to 7-membered (preferably 5- to 6-membered) monocyclic heterocyclic ring residue which contains one or more of N, S and O, each of which is protected with an optionally substituted lower (C₁₋₄) alkyl group or an acyl group, etc. [so called synthetic intermediate].

As the group R¹, carboxyl, tetrazolyl or 2,5-dihydro-5-oxo-1,2,4-oxadiazol-3-yl (preferably, tetrazolyl), each of which may be protected with an optionally substituted lower (C₁₋₄) alkyl (e.g., methyl, triphenylmethyl, methoxymethyl, ethoxymethyl, p-methoxybenzyl, p-nitrobenzyl, etc.) or an acyl group (e.g., lower (C₂₋₅) alkanoyl, benzoyl, etc.), or cyano or N-hydroxycarbamimidoyl (preferably cyano) is preferable with tetrazolyl being particularly preferable.

In the above formula, X denotes a bond or a spacer having 2 or less atoms at the straight chain part thereof (preferably a bond). Examples of the spacer having 2 or less atoms at the straight chain part thereof include any divalent chains in which the number of atoms constituting the straight chain part is 1 or 2 and which may have a side chain, and specifically lower (C₁₋₄) alkylene in which the number of atoms constituting the straight chain part is 1 or 2, -CO-, -O-, -S-, -NH-, -CO-NH-, -O-CH₂-, -S-CH₂-, -CH=CH-, etc.

In the above formula, n denotes an integer of 1 or 2 (preferably 1).

In the above formula, the ring A may have, in addition to the group R², another substituent, for example, (1) halogen (e.g., F, Cl, Br, etc.), (2) cyano, (3) nitro, (4) an optionally substituted lower (C₁₋₄) alkyl, (5) a lower (C₁₋₄) alkoxy, (6) an optionally substituted amino group (e.g., amino, N-lower (C₁₋₄) alkylamino (e.g., methylamino, etc.), N,N-di-lower (C₁₋₄) alkylamino (e.g., dimethylamino, etc.), N-arylamino (e.g., phenylamino, etc.), alicyclic amino (e.g., morpholino, piperidino, piperazino, N-phenylpiperazino, etc.), etc.), (7) a group of the formula: -CO-D' wherein D' is a hydroxy group or a lower (C₁₋₄) alkoxy whose alkyl moiety may be substituted with a hydroxy group, a lower (C₁₋₄) alkoxy, a lower (C₂₋₆) alkanoyloxy (e.g., acetoxy, pivaloyloxy, etc.), a lower (C₁₋₆) alkoxy-carbonyloxy (e.g., methoxycarbonyloxy, ethoxycarbonyloxy, etc.) or a lower (C₃₋₆) cycloalkoxycarbonyloxy (e.g., cyclohexyloxycarbonyloxy, etc.), or (8) tetrazolyl, trifluoromethanesulfonamide group, phosphono group or sulfo group, each of which may be protected with an optionally substituted lower (C₁₋₄) alkyl ("an optionally substituted lower (C₁₋₄) alkyl group" similar to that exemplified as a protective group for the above group capable of forming an anion represented by R¹, etc.) or acyl (e.g., lower (C₂₋₅) alkanoyl, benzoyl, etc.), etc.

Of these substituents, one or two may simultaneously be present at any possible position on the benzene ring, in addition to the group R², and preferred examples of the substituents for the benzene ring represented by A include an optionally substituted lower (C₁₋₄) alkyl (e.g., a lower (C₁₋₄) alkyl, etc. optionally substituted with a hydroxy group, a carboxyl group, a halogen, etc.), a halogen, etc. As the ring A, a benzene ring having no substituent besides the group R² is preferable.

In the above formula, examples of the group capable of forming an anion (a group having a hydrogen atom capable of leaving as proton) as R² include (1) an optionally esterified or amidated carboxyl group, (2) a tetrazolyl group, (3) a trifluoromethanesulfonamide group (-NHSO₂CF₃), (4) a phosphono group, (5) a sulfo group, etc., each of which may be protected with an optionally substituted lower alkyl group (e.g. an optionally substituted lower (C₁₋₄) alkyl group similar to the "optionally substituted lower (C₁₋₄) alkyl group" exemplified as a protective group for the above group capable of forming an anion as R¹) or an acyl group (e.g., lower (C₂₋₅) alkanoyl, benzoyl, etc.), or any one of the groups capable of converting thereinto under biological or physiological conditions (for example, in vivo reaction, etc. such as oxidation, reduction, hydrolysis, etc. by in vivo enzyme, etc.), or chemically.

Examples of the optionally esterified or amidated carboxyl as R² include a group of the formula: -CO-D wherein D is (1) a hydroxy group, (2) an optionally substituted amino (for example, amino, N-lower (C₁₋₄) alkylamino, N,N-di-lower (C₁₋₄) alkylamino, etc.) or (3) an optionally substituted alkoxy [e.g., (i) a lower (C₁₋₆) alkoxy group whose alkyl moiety is optionally substituted with a hydroxy group, an optionally substituted amino (e.g., amino, N-lower (C₁₋₄) alkylamino, N,N-di-lower (C₁₋₄) alkylamino, piperidino, morpholino, etc.), a halogen, a lower (C₁₋₆) alkoxy, a lower (C₁₋₆) alkylthio, a lower (C₃₋₈) cycloalkoxy or an optionally substituted dioxolenyl (e.g., 5-methyl-2-oxo-1,3-dioxolen-4-yl, etc.), or (ii) a group of the formula: -O-CH(R⁶)-OCOR⁷ wherein R⁶ is (a) a hydrogen, (b) a straight or branched C₁₋₆ lower alkyl group (e.g., methyl, ethyl, n-propyl, isopropyl, n-butyl, isobutyl, t-butyl, n-pentyl, isopentyl, neopentyl, etc.), (c) a straight or branched C₂₋₆ lower alkenyl group or (d) a C₃₋₈ cycloalkyl group (e.g., cyclopentyl, cyclohexyl, cycloheptyl, etc.), and R⁷ is (a) a straight or branched C₁₋₆ lower alkyl group (e.g., methyl, ethyl, n-propyl, isopropyl, n-butyl, isobutyl, sec-butyl, t-butyl, n-pentyl, isopentyl, neopentyl, etc.), (b) a straight or branched C₂₋₆ lower alkenyl group, (c) a lower C₁₋₃ alkyl group substituted with a C₃₋₈ cycloalkyl group (e.g., cyclopentyl, cyclohexyl, cycloheptyl, etc.) or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as benzyl, p-chlorobenzyl, phenethyl, cyclopentylmethyl, cyclohexylmethyl, etc., (d) a lower C₂₋₃ alkenyl group substituted with a C₃₋₈ cycloalkyl or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as cinnamyl, etc. having an alkenyl moiety such as vinyl, propenyl, allyl, isopropenyl, etc., (e) an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as phenyl, p-tolyl, naphthyl, etc., (f) a straight or branched lower C₁₋₆ alkoxy group (e.g., methoxy, ethoxy, n-propoxy, isopropoxy, n-butoxy, isobutoxy, sec-butoxy, t-butoxy, n-pentyloxy, isopentyloxy, neopentyloxy, etc.), (g) a straight or branched lower C₂₋₈ alkenyloxy group (e.g., allyloxy, isobutenyloxy, etc.), (h) a C₃₋₈ cycloalkyloxy group (e.g., cyclopentyloxy, cyclohexyloxy, cycloheptyloxy, etc.), (i) a lower C₁₋₃ alkoxy group substituted with a C₃₋₈ cycloalkyl (e.g., cyclopentyl, cyclohexyl, cycloheptyl, etc.) or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as benzyloxy, phenethyloxy, cyclopentylmethoxy, cyclohexylmethoxy, etc. having an alkoxy moiety such as methoxy, ethoxy, n-propoxy, isopropoxy, etc., etc.), (j) a lower C₂₋₃ alkenyloxy group substituted with a C₃₋₈ cycloalkyl (e.g., cyclopentyl, cyclohexyl, cycloheptyl, etc.) or an optionally substituted aryl group (e.g., a phenyl group or a naphthyl group, etc., optionally having a halogen atom, a nitro, a lower (C₁₋₄) alkyl, a lower (C₁₋₄) alkoxy, etc.) such as cinnamyloxy, etc. having an alkenyloxy moiety such as vinyloxy, propenyloxy, allyloxy, isopropenyloxy, etc. or (k) an optionally substituted aryloxy group (e.g., a phenoxy group, a naphthoxy group, etc., optionally having a halogen atom, a nitro, a lower (C₁₋₄) alkyl, a lower (C₁₋₄) alkoxy, etc.) such as phenoxy, p-nitrophenoxy, naphthoxy, etc.], etc.

As R², an optionally esterified carboxyl is preferable, and its specific examples include -COOH and a salt thereof, -COOMe, -COOEt, -COOtBu, -COOPr, pivaloyloxymethoxycarbonyl, 1-(cyclohexyloxycarbonyloxy)ethoxycarbonyl, 5-methyl-2-oxo-1,3-dioxolen-4-ylmethoxycarbonyl, acetoxymethoxycarbonyl, propionyloxymethoxycarbonyl, n-butyryloxymethoxycarbonyl, isobutyryloxymethoxycarbonyl, 1-(ethoxycarbonyloxy)ethoxycarbonyl, 1-(acetoxy)-ethoxycarbonyl, 1-(isobutyryloxy)ethoxycarbonyl, cyclohexylcarbonyloxymethoxycarbonyl, benzoyloxymethoxycarbonyl, cinnamyloxycarbonyl, cyclopentylcarbonyloxymethoxycarbonyl, etc. The group R² may be any one of the groups capable of forming an anion under biological or physiological conditions (for example, in vivo reaction, etc. such as oxidation, reduction, hydrolysis, etc. by in vivo enzyme, etc.), the groups capable of chemically forming an anion (e.g., COO⁻, its derivative, etc.) or the groups capable of changing thereinto. The group R² may be a carboxyl group or its prodrug.

Preferred examples of the group R² include a group of the formula: -CO-D wherein D is (1) a hydroxy group or (2) a lower (C₁₋₄) alkoxy whose alkyl moiety is optionally substituted with a hydroxy group, an amino, a halogen, a lower (C₂₋₆) alkanoyloxy (e.g., acetoxy, pivaloyloxy , etc.), a lower (C₃₋₈) cycloalkanoyloxy, a lower (C₁₋₆) alkoxy-carbonyloxy (e.g., methoxycarbonyloxy, ethoxycarbonyloxy, etc.), a lower (C₃₋₈) cycloalkoxycarbonyloxy (e.g., cyclohexyloxycarbonyloxy, etc.), a lower (C₁₋₄) alkoxy or a lower (C₃₋₈) cycloalkoxy. Among others, carboxyl is preferable.

In the above formula, examples of the "hydrocarbon residue" in the "optionally substituted hydrocarbon residue, wherein the hydrocarbon residue may bind via a hetero atom" represented by R³ include (1) an alkyl group, (2) an alkenyl group, (3) an alkynyl group, (4) an cycloalkyl group, (5) an aryl group, (6) an aralkyl group, etc. Among others, an alkyl group, an alkenyl group and a cycloalkyl group are preferable.

Examples of the alkyl group of the above mentioned (1) include straight or branched lower alkyl group having about 1-8 carbon atoms such as methyl, ethyl, propyl, isopropyl, butyl, isobutyl, sec-butyl, t-butyl, pentyl, i-pentyl, hexyl, heptyl, octyl, etc.

Examples of the alkenyl group of the above mentioned (2) include straight or branched lower alkenyl group having about 2-8 carbon atoms such as vinyl, propenyl, 2-butenyl, 3-butenyl, isobutenyl, 2-octenyl, etc.

Examples of the alkynyl group of the above mentioned (3) include straight or branched lower alkynyl group having about 2-8 carbon atoms such as ethynyl, 2-propynyl, 2-butynyl, 2-pantynyl, 2-octynyl, etc.

Examples of the cycloalkyl group of the above (4) include a lower cycloalkyl having about 3-6 carbon atoms, such as cyclopropyl, cyclobutyl, cyclopentyl, cyclohexyl, etc.

Each of the above-mentioned alkyl group, alkenyl group, alkynyl group and cycloalkyl group may be substituted with a hydroxy group, an optionally substituted amino group (e.g., amino, N-lower (C₁₋₄) alkylamino, N,N-di-lower (C₁₋₄) alkylamino, etc.), halogen , a lower (C₁₋₄) alkoxy group, a lower (C₁₋₄) alkylthio group, etc.

Examples of the aralkyl group of the above (5) include a phenyl-lower (C₁₋₄) alkyl, etc., such as benzyl, phenethyl, etc. Examples of the aryl group of the above (6) include phenyl, etc.

Each of the above-mentioned aralkyl group and aryl group may be substituted, at any possible position on the benzene ring, with a halogen (e.g., F, Cl, Br, etc.), a nitro, an optionally substituted amino group (e.g., amino, N-lower (C₁₋₄) alkylamino, N,N-di-lower (C₁₋₄) alkylamino, etc.), a lower (C₁₋₄) alkoxy (e.g., methoxy, ethoxy, etc.), a lower (C₁₋₄) alkylthio (e.g., methylthio, ethylthio, etc.), a lower (C₁₋₄) alkyl (e.g., methyl, ethyl, etc.), etc.

Preferred examples of the "optionally substituted hydrocarbon residue" in the "optionally substituted hydrocarbon residue, wherein the hydrocarbon residue may bind via a hetero atom" represented by R³ include an optionally substituted alkyl or alkenyl group (e.g., a lower (C₁₋₅) alkyl or a lower (C₂₋₅) alkenyl group, each of which may be substituted with a hydroxy group, an amino group, a halogen, a lower (C₁₋₄) alkoxy group, etc.). Among others, a lower (C₁₋₅) alkyl (more preferably, ethyl) is preferable.

Preferred examples of the "hetero-atom" in the "optionally substituted hydrocarbon residue, wherein the hydrocarbon residue may bind via a hetero atom" represented by R³ include -O-, -S(O)m- [m is an integer of 0 to 2], -NR'- [R' is a hydrogen atom or a lower (C₁₋₄) alkyl], etc. Among others, -O- is preferable.

Among others, as R³, a lower (C₁₋₅) alkyl or a lower (C₂₋₅) alkenyl group, each of which may be substituted with a substituent selected from the class consisting of a hydroxy group, an amino group, a halogen and a lower (C₁₋₄) alkoxy group and which may bind via -O-, -S(O)m- [m is an integer of 0 to 2] or -NR'- [R' is a hydrogen atom or a lower (C₁₋₄) alkyl], etc. is preferable and a lower (C₁₋₅) alkyl or lower (C₁₋₅) alkoxy (in particular, ethoxy) is more preferable.

Among the non-peptide compounds having angiotensin II antagonistic activity and represented by the formula (I), a benzimidazole-7-carboxylic acid derivative of the formula (I') : 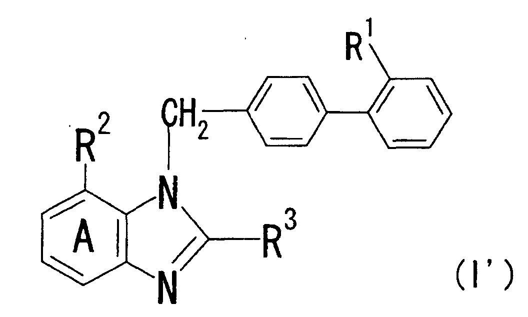 wherein R¹ is (1) carboxyl group, (2) tetrazolyl group or (3) a group of the formula: 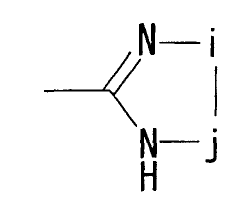 wherein i is -O- or -S-, j is >=O, >=S or >=S(O)m, and m is as defined above; the ring A is a benzene ring having an optional substituent selected from the class consisting of an optionally substituted lower (C₁₋₄) alkyl (e.g., a lower (C₁₋₄) alkyl optionally substituted with a hydroxy group, a carboxyl group, a halogen, etc.) and a halogen, in addition to the group R² (preferably, a benzene ring having no substituent besides the group R²); R² is a group of the formula: -CO-D wherein D is (1) a hydroxy group or (2) a lower (C₁₋₄) alkoxy whose alkyl moiety may be substituted with a hydroxy group, an amino, a halogen, a lower (C₂₋₆) alkanoyloxy (e.g., acetoxy, pivaloyloxy, etc.), a lower (C₃₋₈) cycloalkanoyloxy, a lower (C₁₋₆) alkoxy-carbonyloxy (e.g., methoxycarbonyloxy, ethoxycarbonyloxy, etc.), a lower (C₃₋₈) cycloalkoxy-carbonyloxy (e.g., cyclohexyloxycarbonyloxy, etc.), a lower (C₁₋₄) alkoxy or a lower (C₃₋₈) cycloalkoxy; R³ is a lower (C₁₋₅) alkyl or a lower (C₂₋₅) alkenyl group, each of which may bind via -O-, -S(O)m- [m is an integer of 0 to 2] or -NR'- [R' is a hydrogen atom or a lower (C₁₋₄) alkyl] and which may be substituted with a substituent selected from the class consisting of a hydroxy group, an amino group, a halogen and a lower (C₁₋₄) alkoxy group (preferably, a lower (C₁₋₅) alkyl or a lower (C₁₋₅) alkoxy; more preferably, ethoxy), etc. or a pharmaceutically acceptable salt thereof is preferable. Among others, 2-ethoxy-1-[[2'-(1H-tetrazol-5-yl)biphenyl-4-yl]methyl]benzimidazole-7-carboxylic acid [Candesartan], 1-(cyclohexyloxycarbonyloxy)ethyl 2-ethoxy-1-[[2'-(1H-tetrazol-5-yl)biphenyl-4-yl]methyl]-benzimidazole-7-carboxylate [Candesartan cilexetil], pivaloyloxymethyl 2-ethoxy-1-[[2'-(1H-tetrazol-5-yl)biphenyl-4-yl]methyl]benzimidazole-7-carboxylate, 2-ethoxy-1-[[2'-(2,5-dihydro-5-oxo-1,2,4-oxadiazol-3-yl)biphenyl-4-yl]methyl]benzimidazole-7-carboxylic acid, etc. or a salt thereof are preferable.

The above mentioned benzimidazole derivative can be produced by known methods described in, for example, EP-425921, EP-459136, EP-553879, EP-578125, EP-520423, EP-668272, etc. or a method analogous thereto. When Candesartan cilexetil is used for the present invention, a stable C-type crystal described in EP-459136 is preferably used.

In the present invention, the compound having angiotensin II antagonistic activity or a prodrug thereof may be used as such or in the form of any possible pharmaceutically acceptable salts thereof. Examples of said salts include a salt with inorganic bases (e.g., alkaline metals such as sodium, potassium, etc.; alkaline earth metals such as calcium, magnesium, etc.; transition metal such as zinc, iron, copper, etc.; etc.); organic bases (e.g., organic amines such as trimethylamine, triethylamine, pyridine, picoline, ethanolamine, diethanolamine, triethanolamine, dicyclohexylamine, N,N'-dibenzylethylenediamine, etc.; basic amino acids such as arginine, lysine, ornithine, etc.; etc.); etc., in case that the compound having angiotensin II antagonistic activity has an acidic group such as a carboxyl group, etc.

The prodrug of the compound having angiotensin II antagonistic activity [hereinafter, referred to as All antagonist] means a compound which is converted to All antagonist under the physiological condition or with a reaction due to an enzyme, an gastric acid, etc. in a living body, that is, a compound which is converted to All antagonist with oxidation, reduction, hydrolysis, etc. according to an enzyme; a compound which is converted to AII antagonist with gastric acid, etc.; etc. Examples of the prodrug of the All antagonist include a compound wherein an amino group of the All antagonist is acylated, alkylated or phosphorylated (e.g. a compound wherein an amino group of the All antagonist is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, tert-butylated, etc.); a compound wherein an hydroxy group of the AII antagonist is acylated, alkylated, phosphorylated or borated (e.g. a compound wherein an hydroxy group of the AII antagonist is acetylated, palmitoylated, propanoylated, pivaloylated, succinylated, fumarylated, alanylated, dimethylaminomethylcarbonylated, etc.); a compound wherein a carboxyl group of the All antagonist is esterified or amidated (e.g. a compound wherein a carboxyl group of the All antagonist is ethyl esterified, phenyl esterified, carboxymethyl esterified, dimethylaminomethyl esterified, pivaloyloxymethyl esterified, ethoxycarbonyloxyethyl esterified, phthalidyl ester, (5-methyl-2-oxo-1,3-dioxolen-4-yl)methyl esterified, cyclohexyloxycarbonylethyl esterified, methyl amidated, etc.); etc. These prodrugs can be produced by per se known method from the All antagonist.

In addition, the prodrug of the All antagonist may be a compound which is converted into the All antagonist under the physiological conditions as described in "Pharmaceutical Research and Development", Vol. 7 (Drug Design), pages 163-198 published in 1990 by Hirokawa Publishing Co. (Tokyo, Japan).

Further, the AII antagonist may be either of hydrates or non-hydrates.

### B. Regarding polyvalent metal (1)

The following will illustrate in detail the polyvalent metal used in the process of the present invention using an organic solvent solution containing a poorly water-soluble nonpeptidic physiologically active compound, a polyvalent metal compound and a biodegradable polymer, wherein the physiologically active compound is dissolved in an amount exceeding the solubility thereof in the solution of biodegradable polymer and organic solvent in the absence of the polyvalent metal compound.

The polyvalent metal to be used in the present invention may be any metal, as long as it is a metal that does not adversely affect a living body. Examples of the metal include polyvalent metals such as a divalent metal (e.g., zinc, calcium, magnesium, iron, copper, aluminum, tin, manganese, etc.), a trivalent metal (e.g., iron, aluminum, manganese, etc.), a tetravalent metal (e.g., tin, etc.), etc.

The polyvalent metal to be used in the present invention may be used in the form of a "polyvalent metal compound" such as a compound with an inorganic or organic substance, a complex compound, or a metal oxide. In addition, the "polyvalent metal compound" may have bound water or crystallization water.

Preferable examples of the polyvalent metal include zinc, calcium, magnesium and the like. A particularly preferable example of the polyvalent metal is zinc.

As the organic substance, organic acids such as an aliphatic carboxylic acid and an aromatic acid, and acetylacetone are used. As the aliphatic carboxylic acid, preferably, aliphatic C₁₋₉ carboxylic acids (e.g. aliphatic monocarboxylic acid, aliphatic dicarboxylic acid, aliphatics tricarboxylic acid etc.) are used. The aliphatic carboxylic acid may be saturated or unsaturated.

As the aliphatic monocarboxylic acid, for example, saturated aliphatic C₁₋₉ monocarboxylic acids (e.g. carbonic acid, formic acid, acetic acid, propionic acid, butyric acid, valeric acid, caproic acid, enanthic acid, caprylic acid, pelargonic acid, capric acid etc.) and unsaturated aliphatic C₂₋₉ monocarboxylic acids (e.g. acrylic acid, propiolic acid, methacrylic acid, crotonic acid, isocrotonic acid etc.) are used.

As the aliphatic dicarboxylic acid, for example, saturated aliphatic C₂₋₉ dicarboxylic acids (e.g. malonic acid, succinic acid, glutaric acid, adipic acid, pimelic acid etc.) and unsaturated aliphatic C₂₋₉ dicarboxylic acids (e.g. maleic acid, fumaric acid, citraconic acid, mesaconic acid etc.) are used.

As the aliphatic tricarboxylic acid, for example, saturated aliphatic C₂₋₉ tricarboxylic acids (e.g. tricarballylic acid, 1,2,3-butanetricarboxylic acid etc.) are used.

The aforementioned aliphatic carboxylic acid may have 1 or 2 hydroxy group(s), and examples thereof include glycolic acid, lactic acid, glyceric acid, tartronic acid, malic acid, tartaric acid, citric acid and the like.

The aliphatic carboxylic acid is preferably aliphatic monocarboxylic acid, more preferably aliphatic C₂₋₉ monocarboxylic acid. A particularly preferable example of the aliphatic carboxylic acid is acetic acid.

As the aromatic acid, for example, benzoic acid, salicylic acid, and phenolsulfonic acid are used.

Specific examples of the metal compound include:
salts of zinc and inorganic acids [e.g. zinc halides (e.g. zinc chloride, zinc bromide, zinc iodide, zinc fluoride etc.), zinc sulfate, zinc nitrate, zinc thiocyanate etc.], salts of zinc and organic acids [e.g. zinc aliphatic carboxylates (e.g. zinc carbonate, zinc acetate, zinc glycolate, zinc lactate, zinc tartarate etc.), aromatic zinc salts (e.g. zinc benzoate, zinc salicylate, zinc phenolsulfonate etc.)], zinc acetylacetonate,
salts of calcium and inorganic acids [e.g. calcium halides (e.g. calcium chloride, calcium bromide, calcium iodide, calcium fluoride etc.) calcium sulfate, calcium nitrate, calcium thiocyanate etc.], salts of calcium and organic acids [e.g. calcium aliphatic carboxylates (e.g. calcium carbonate, calcium acetate, calcium propionate, calcium oxalate, calcium tartarate, calcium lactate, calcium citrate, calcium gluconate etc.), aromatic calcium salts (e.g. calcium benzoate, calcium salicylate etc.)], calcium acetylacetonate,
salts of magnesium and inorganic acids [e.g. magnesium halides (e.g. magnesium chloride, magnesium bromide, magnesium iodide, magnesium fluoride etc.), magnesium sulfate, magnesium nitrate, magnesium thiocyanate etc.], salts of magnesium and organic acids [e.g. magnesium aliphatic carboxylates (e.g. magnesium carbonate, magnesium acetate, magnesium propionate, magnesium oxalate, magnesium tartalate, magnesium lactate, magnesium citrate, magnesium gluconate etc.), aromatic magnesium salts (e.g. magnesium benzoate, magnesium salicylate etc.)], and magnesium acetylacetonate,
salts of iron and inorganic acids [e.g. iron halide (e.g. iron chloride, iron bromide, iron iodide, iron fluoride etc.), iron sulfate, iron nitrate, iron thioncyanate etc.], salts of iron and organic acids [e.g. iron aliphatic carboxylates (e.g. iron carbonate, iron acetate, iron glycolate, iron lactate, iron tartarate etc.), aromatic iron salts (e.g. iron benzonate, iron salicylate, iron phenolsulfonate etc.)], and iron acetylacetonate, and
metal oxides (e.g. zinc oxide, iron oxide, calcium oxide, magnesium oxide, aluminium oxide, copper oxide, manganese oxide etc.).

As the polyvalent metal compound, preferably, zinc acetate, zinc oxide, calcium acetate, magnesium acetate, iron chloride, iron acetylacetonate, zinc acetylacetonate, calcium acetylacetonate, magnesium acetylacetonate and the like are used, more preferably, zinc acetate or/and zinc oxide, that is, either one of zinc acetate and zinc oxide, or a combination thereof is used.

When zinc acetate and zinc oxide are used as the polyvalent metal compound, they may be used alone or by mixing them, and a ratio of zinc acetate/zinc oxide (molar ratio) is 0/100 to 100/0, preferably 10/90 to 70/30, most preferably 25/75 to 55/45.

In the present invention, a part of the polyvalent metal contained in the sustained-release preparation may form a metal salt (e.g. zinc salt or heterogeneous metal salt) with a biodegradable polymer. This metal salt of a biodegradable polymer can be prepared, for example, by the method described in JP-A 09-221420 or an analogous method. Alternatively, a part of the polyvalent metal contained in the sustained-release preparation may form a complex with a poorly water-soluble nonpeptidic physiologically active compound.

A mixing ratio of the metal compound used in present invention relative to the poorly water-soluble nonpeptidic physiologically active compound is varied depending on the particular metal compound and poorly water-soluble nonpeptidic physiologically active compound and their optimal amounts to be added are different. For example, when the metal compound is divalent and the poorly water-soluble nonpeptidic physiologically active compound is an All antagonistic compound, a ratio of metal compound/poorly water-soluble nonpeptidic physiologically active compound (molar ratio) is preferably 1/10 to 10/1, more preferably 1/5 to 5/1, most preferably 1/2 to 2/1.

### C. Regarding biodegradable polymer

Examples of the biodegradable polymer to be used in the present invention include a polymer, a copolymer, or a mixture thereof which is synthesized from one or more of α-hydroxycarboxylic acids (e.g., glycolic acid, lactic acid, etc.), hydroxydicarboxylic acids (e.g., malic acid, etc.), hydroxytricarboxylic acids (e.g., citric acid, etc.), etc. and which has a free carboxyl group; poly-α-cyanoacrylic acid esters; polyamino acids (e.g., poly-g-benzyl-L-glutamic acid, etc.); maleic anhydride copolymer (e.g., styrene-maleic acid copolymer, etc.), etc.

The mode of polymerization may be any of random, block and graft. When the above α-hydroxycarboxylic acids, hydroxydicarboxylic acids and hydroxytricarboxylic acids have optical active centers in their molecules, any one of D-isomers, L-isomers and DL-isomers may be used. Among others, α-hydroxycarboxylic acid polymer (preferably lactic acid-glycolic acid copolymer), its ester, poly-α-cyanoacrylic acid esters, etc. are preferable, and lactic acid-glycolic acid copolymer, its ester are more preferable.

When the lactic acid-glycolic acid copolymer is used as the biodegradable polymer, the molar ratio (mole %) ranges preferably from 100/0 to 40/60 and more preferably from 100/0 to 50/50.

Usually, the weight-average molecular weight of the above lactic acid-glycolic acid copolymer ranges from about 3,000 to about 50,000, preferably about 4,000 to about 40,000, and more preferably about 5,000 to about 30,000. Further, a dispersity (weight average molecular weight/number average molecular weight) is usually preferably about 1.2 to about 4.0, more preferably about 1.5 to 3.5.

In the present specification, the weight-average molecular weight, number-average molecular weight and dispersity mean molecular weights determined in terms of polystyrene by gel permeation chromatography (GPC) with 14 polymers of polystyrene as reference substances with weight-average molecular weights of 1,110,000, 707,000, 354,000, 189,000, 156,000, 98,900, 66,437, 37,200, 17,100, 9,830, 5,870, 2,500, 1,303 and 500, and dispersities calculated therefrom. The determination was carried out using GPC column KF804L × 2 (manufactured by Showa Denko K.K., Japan) and using chloroform as the mobile phase. To calculate number-average molecular weight, the biodegradable polymer is dissolved in a mixed solvent of acetone and methanol and the solution is titrated with alcoholic potassium hydroxide solution with using phenolphthalein as an indicator to determine the terminal carboxyl group content. This value is hereinafter referred to as number-average molecular weight by end-group determination. While the number-average molecular weight by end-group determination is an absolute value, that by GPC determination is a relative value that varies depending on various analytical conditions (for example, kind of the mobile phase, kind of the column, reference substance, selection of slice width, selection of baseline, etc.). It is therefore difficult to have an absolute numerical representation of the latter. However, for example, in the case of a polymer having a terminal carboxyl group and produced from lactic acid and glycolic acid by catalyst-free polycondensation, the number-average molecular weight by GPC and the number-average molecular weight by end-group determination almost agree with each other. The description that the number-average molecular weight by GPC and end-group determination "almost agree" here denotes that the number-average molecular weight by end-group determination falls within the range from about 0.2 to about 1.5 times, preferably about 0.3 to about 1.2 times of the number-average molecular weight by GPC.

The lactic acid-glycolic acid copolymer can be produced by, for example, catalyst-free polycondensation from lactic acid and glycolic acid (JP 61-28521 A) or ring-opening polymerization with catalyst from cyclic lactide, glycolide, etc. (Encyclopedic Handbook of Biomaterials and Bioengineering Part A: Materials, Volume 2, Marcel Dekker, Inc. (1995)).

The polymer produced by ring-opening polymerization has little or no carboxyl group, however, a polymer having a terminal carboxyl group obtained by chemically treating the former polymer (J. Controlled Release, Vol. 41, pages 249-257 (1996)) can be used for the present invention.

The above-mentioned lactic acid-glycolic acid copolymer having a terminal carboxyl group can be synthesized by a known production process (e.g. catalyst-free polycondensation described in JP 61-28521 A), without any problem. Moreover, the polymer having free carboxyl groups at unspecified position can be synthesized by a known production process (for example, WO94/15587).

Further, as the lactic acid-glycolic acid copolymer in which a terminal is converted into a free carboxyl group by chemical treatment after ring-opening polymerization, for example, polymers sold by Boehringer Ingelheim KG may be used.

These biodegradable polymers may be used alone or by mixing to or more kinds.

### D. Embodiments (1)

The following will illustrate in detail the process of the present invention using an organic solvent solution containing a poorly water-soluble nonpeptidic physiologically active compound, a polyvalent metal compound and a biodegradable polymer, wherein the physiologically active compound is dissolved in an amount exceeding the solubility thereof in the solution of biodegradable polymer and organic solvent in the absence of the polyvalent metal compound, and embodiments thereof.

The process of the present invention is characterized by using an organic solvent solution containing a poorly water-soluble nonpeptidic physiologically active compound, a polyvalent metal compound and a biodegradable polymer, wherein the physiologically active compound is dissolved in an amount exceeding the solubility thereof in the solution of biodegradable polymer and the organic solvent in the absence of the polyvalent metal compound; and is characterized by dissolving a poorly water-soluble nonpeptidic physiologically active compound in an organic solvent solution in an amount exceeding the solubility thereof in the absence of a polyvalent metal compound, even when a biodegradable polymer is not contained in the organic solvent solution. That is, the solubility of a poorly water-soluble nonpeptidic physiologically active compound in an organic solvent solution in the absence of a polyvalent metal compound is increased due to the presence of a polyvalent metal compound, and the solubility of a poorly water-soluble nonpeptidic physiologically active compound in a solution of a biodegradable polymer and an organic solvent in the absence of a polyvalent metal compound is increased due to the presence of a polyvalent metal compound.

In the present specification, an "amount exceeding the solubility" refers to about 110% or more, preferably about 300% or more, more preferably about 500% or more of the solubility of a poorly water-soluble nonpeptidic physiologically active compound in a solution of a biodegradable polymer and an organic solvent in the absence of a polyvalent metal compound.

As the organic solvent, for example, halogenated hydrocarbons (e.g. dichloromethane, chloroform, dichloroethane, trichloroethane, carbon tetrachloride etc.), ethers (e.g. ethyl ether, isopropyl ether etc.), fatty acid esters (e.g. ethyl acetate, butyl acetate etc.), aromatic hydrocarbons (e.g. benzene, toluene, xylene etc.), alcohols (e.g. ethanol, methanol etc.), and acetonitrile are used. These may be used by mixing at an appropriate ratio. Inter alia, as a halogenated hydrocarbon, dichloromethane is suitable and, as an alcohol, ethanol and methanol are suitable. These may be used by mixing at an appropriate ratio.

Additives may be added to the above-mentioned organic solvent solution. Examples of the additives include a solubilizer which maintains stability of the drug such as carbonic acid, oxalic acid, citric acid, phosphoric acid, hydrochloric acid, sodium hydroxide, arginine, lysine and their salts, etc. Further, as stabilizers of the drug, there can be added, for example, albumin, gelatin, citric acid, sodium ethylenediaminetetraacetate, dextrin, sodium hydrogensulfite, polyols such as polyethyleneglycol, etc., etc., and as preservatives there can be added, for example, conventional para-oxybenzoic acid esters (e.g., methylparaben, propylparaben, etc.), benzylalcohol, chlorobutanol, thimerosal, etc.

The concentration of the poorly water-soluble nonpeptidic physiologically active compound in the organic solvent solution is varied depending on a kind of the poorly water-soluble nonpeptidic physiologically active compound and a kind of the organic solvent, and is generally about 0.5 to about 70% by weight. For example, when the poorly water-soluble nonpeptidic physiologically active compound is an All antagonistic compound, and a mixed solution of dichloromethane and methanol is used as the organic solvent, the concentration is selected, generally, from about 0.5 to about 70% by weight, more preferably about 1 to about 60% by weight, particularly preferably about 2 to about 50% by weight.

The concentration of the biodegradable polymer in the organic solvent solution is varied depending on a molecular weight of the biodegradable polymer and a kind of the organic solvent, and is generally about 0.5 to about 70% by weight. For example, when dichloromethane is used as an organic solvent, the concentration is selected, generally, from about 0.5 to about 70% by weight, more preferably about 1 to about 60% by weight, particularly preferably about 2 to about 50% by weight.

Further, when ethanol or methanol is used as an organic solvent to be mixed with dichloromethane, a ratio relative to dichloromethane in the mixed organic solvent is generally selected from about 10 to about 90% by volume, more preferably about 15 to about 85% by volume, particularly preferably about 20 to about 80% by volume.

As the order of addition of the poorly water-soluble nonpeptidic physiologically active compound, the polyvalent metal compound and the biodegradable polymer to the organic solvent, any one may be added first, or they may be added at the same time, or an organic solvent solution of the poorly water-soluble nonpeptidic physiologically active compound and the polyvalent metal compound and an organic solvent solution of the biodegradable polymer may be mixed.

In order to dissolve the poorly water-soluble nonpeptidic active compound, the polyvalent metal compound and the biodegradable polymer in the organic solvent, they may be allowed to stand, or they may be mixed by appropriately using energy. As the energy, there are ultrasonication, physical shaking, stirring and the like. The dissolution may be carried out at any temperature as far as it is not higher than the boiling point of a solvent to be used, and it is preferably 0°C to 30°C. Time required for dissolution is varied depending on kinds of the poorly water-soluble nonpeptidic physiologically active compound, the polyvalent metal compound and the biodegradable polymer, a blending ratio, a dissolution temperature, vibration and the like, and is preferably 1 second to 7 days, more preferably 5 seconds to 3 days, most preferably 30 seconds to 1 day at room temperature.

The organic solvent solution of the poorly water-soluble nonpeptidic physiologically active compound, the polyvalent metal compound and the biodegradable polymer which is characteristics in the present invention is preferably a sterile solution, more preferably a solution prepared by filter-sterilization. When it is used as a preparation for injection for injection, there is an advantage that a sterile bulk of the poorly water-soluble nonpeptidic physiologically active compound is not needed.

Amounts of the poorly water-soluble nonpeptidic physiologically active compound and the polyvalent metal compound to be formulated in the sustained-release preparation obtained by the process of the present invention are varied depending on a kind of the physiologically active compound, the desired pharmacological effect and a duration term of the effect and the like. However, when the physiologically active compound, the polyvalent metal compound and the biodegradable polymer are used as starting materials, relative to the total amount of these three materials, the amount of the physiologically active compound is usually about 1 to about 50% by weight, more preferably 15 to 40% by weight, particularly preferably about 20 to 40% by weight and, on the other hand, the amount of the polyvalent metal compound is usually about 0.5 to about 20% by weight, more preferably about 1 to about 15% by weight, particularly preferably about 2 to about 10% by weight.

The dosage form of the sustained-release preparation of the present invention is not particularly limited, but a parenteral preparation is preferable and a transdermal preparation, a transmucous preparation, an implant and a microcapsule injectable preparation are contemplated. A preparation for injection using microcapsules which has a long sustained release term and has less burden to a patient is preferable.

The following will illustrate a process for producing the sustained-release preparation of the present invention, which comprises removing a solvent from an organic solvent solution containing a poorly water-soluble nonpeptidic physiologically active compound, a polyvalent metal compound and a biodegradable polymer, said physiologically active compound dissolved in an amount exceeding the solubility thereof in the solution of biodegradable polymer and the organic solvent in the absence of the polyvalent metal compound, by using microcapsules (hereinafter, referred to as microspheres in some cases) as an example of a preparation.

### (I) In-water drying method

The organic solvent solution of the poorly water-soluble nonpeptidic physiologically active compound, the polyvalent metal compound and the biodegradable polymer obtained by the above-mentioned method is added to a water phase to form an O (oil phase)/W (water phase) emulsion. Then, a solvent in the oil phase is evaporated to prepare microcapsules. At this time, the volume of the water phase is generally selected from about 1-fold to about 10,000-fold, more preferably about 5-fold to about 5,000-fold, particularly preferably about 10-fold to about 2,000-fold volume of the oil phase.

An emulsifying agent may be added to the outer water phase. Any emulsifying agent which can generally form a stable O/W emulsion may be added. Specifically, anionic surfactants (sodium oleate, sodium stearate, sodium laurylsulfate etc.), nonionic surfactants (polyoxyethylene sorbitan fatty acid esters [Tween 80, Tween 60, Atlas Powder], polyoxyethylene castor oil derivatives [HCO-60, HCO-50, Nikko Chemicals] etc.), polyvinylpyrrolidone, polyvinyl alcohol, carboxymethylcellulose, lecithin, gelatin and hyaluronic acid, etc., are used. These may be used alone or in combination of 2 or more thereof. Upon use, the concentration is preferably in a range of about 0.01 to 10% by weight, more preferably in a range of about 0.05 to about 5% by weight.

An osmotic pressure adjusting agent may be added to the outer water phase. Any osmotic pressure adjusting agent may be used as far as it show the osmotic pressure when formulated into an aqueous solution.

Examples of the osmotic pressure adjusting agent include polyhydric alcohols, monohydric alcohols, monosaccharides, disaccharides, oligosaccharides and amino acids, and derivatives thereof.

As the polyhydric alcohols, for example, dihydric alcohols such as glycerin and the like, pentahydric alcohols such as arabitol, xylitol, adnitol and the like, and hexahydric alcohols such as mannitol, sorbitol, dulcitol and the like are used. Inter alia, hexahydric alcohols are preferable and, in particular, mannitol is suitable.

Examples of the monohydric alcohols include methanol, ethanol, isopropyl alcohol, etc., and, among them, methanol is preferable.

As the monosaccharides, for example, pentoses such as arabinose, xylilose, ribose, 2-deoxyribose and the like, and hexoses such as glucose, fructose, galactose, mannose, sorbose, rhamnose, fucose and the like are used and, among them, hexoses are preferable.

As the oligosaccharides, trioses such as maltotriose, raffinose and the like, and tetroses such as stachyose and the like are used and, among them, trioses are preferable.

As the derivatives of monosaccharides, disaccharides and oligosaccharides, for example, glucosamine, galactosamine, glucuronic acid, galacturonic acid and the like are used.

As the amino acids, any amino acids can be used as far as they are L-isomers, such as glycine, leucin, arginine and the like. Among them, L-arginine is preferable.

These osmotic pressure adjusting agents may be used alone or by mixing them.

These osmotic pressure adjusting agents are used at such a concentration that osmotic pressure of an outer water phase becomes about 1/50-fold to about 5-fold, preferably about 1/25-fold to 3-fold osmotic pressure of physiological saline.

As a method for removing the organic solvent, a per se known method or an analogous method is used. Example thereof include a method for evaporating the organic solvent at normal pressure or by gradually evacuating while stirring with a propeller-type stirrer or a magnetic stirrer, and a method for evaporating the organic solvent while regulating a degree of vacuum using a rotary evaporator.

Thus obtained microcapsules are collected by centrifugation or filtration, the physiologically active compound, a drug retaining substance and an emulsifying agent and the like adhered to the surfaces of microcapsules are repeatedly washed with distilled water several times, and the microcapsules are dispersed in distilled water, followed by lyophilization.

During the production process, an aggregation preventing agent may be added in order to prevent aggregation of particles. As the aggregation preventing agent, for example, mannitol, lactose, glucose, water soluble polysaccharides such as starches (e.g. corn starch etc.), amino acid such as glycine, and protein such as fibrin and collagen are used. Inter alia, mannitol is suitable.

Further, after lyophilization, if necessary, moisture and the organic solvent in microcapsules may be removed by warming under reduced pressure under such conditions that microcapsules are not fused together. Preferably, warming is performed at a temperature slightly higher than the intermediate point glass transition temperature of the biodegradable polymer obtained by a differential scanning calorimeter, preferably under conditions of a temperature raising rate of 10 to 20°C per minute. More preferably, warming is performed at a temperature range from the intermediate point glass transition temperature of the biodegradable polymer to a temperature about 30°C higher than it. Inter alia, when a lactic acid-glycolic acid copolymer is used as the biodegradable polymer, warming is performed in a temperature range, preferably, from its intermediate point glass transition temperature to a temperature 10°C higher than the intermediate point glass transition temperature, more preferably in a temperature range from an intermediate point glass transition temperature to a temperature 5°C higher than the intermediate point glass transition temperature.

Heating time is varied depending on an amount of microcapsules, etc., and is generally about 12 hours to about 168 hours, preferably about 24 hours to about 120 hours, particularly preferably about 48 hours to about 96 hours after a temperature of microcapsules themselves has reached a predetermined temperature.

The warming method is not particularly limited as far as it can warm collected microcapsules uniformly.

As the warming drying method, for example, a method for warming and drying in a constant temperature bath, a fluidizing bed, a moving bath or a kiln, and a method for warming and drying with a microwave are used. Among them, a method of warming and drying in a constant temperature bath is preferable.

Alternatively, removal of moisture and the organic solvent in microcapsules may be performed by a method using a supercritical fluid (CO₂ etc.) or carbon dioxide in a high pressure gaseous state. Preferably, a method using carbon dioxide in a high pressure gaseous state is adopted.

A method for removing the solvent with carbon dioxide in a high pressure gaseous state will be illustrated in detail below.

The high pressure gas in the present invention is gas having pressure not lower than atmospheric pressure at a certain temperature and not higher than liquefying pressure at that temperature.

Examples of the high pressure gas used in the present invention include carbon dioxide, nitrous oxide, nitrogen, helium, argon, alkanes (e.g. ethane, propane etc.), alkenes (e.g. ethylene etc.), and the like. These may be used by mixing at an appropriate ratio, but it is desirable to use carbon dioxide alone.

When the temperature of high pressure gas contacting with a preparation is too high and exceeds the glass transition temperature of the biodegradable polymer used as the base of the preparation, a risk of fusing and deformation of the preparation, degradation and deterioration of the physiologically active substance and the like is increased. The glass transition temperature in the present invention refers to the intermediate point glass transition temperature obtained by raising a temperature at a warming rate of 10 or 20°C per minute using a differential scanning calorimeter (DSC). Further, when the temperature of high pressure gas is too low, the organic solvent is insufficiently removed. Desirably, the organic solvent is removed to such an extent that the concentration thereof is lower than 1,000 ppm, preferably lower than 500 ppm, more preferably lower than about 100 ppm. Therefore, the temperature which is advantageous for using carbon dioxide as the high pressure gas in the present invention is in a range of +20 to -60°C, preferably +10 to -50°C, more preferably 0 to -40°C, further preferably -5 to -30°C, most preferably -10 to -25°C on the basis of the glass transition temperature of the biodegradable polymer (usually around 20 to 60°C).

The range of pressure upon use is varied depending on selected high pressure gas but, generally, when the pressure of high pressure gas is too high, a risk of fusing and deformation of microcapsules, and increase in initial burst release immediately after administration is increased. On the other hand, when the pressure is too low, the organic solvent is insufficiently removed. In the present invention, the pressure which is advantageous for using carbon dioxide as the high pressure gas is about 1 to 7 MPa, preferably about 1 to 4 MPa, more preferably about 2 to 4 MPa.

Time for allowing to contact with high pressure gas is also varied depending on a temperature and pressure of high pressure gas, an amount of microcapsules to be treated and the like and, when carbon dioxide is used as the high pressure gas, about 5 minute to about 24 hours is preferable. About 10 minutes to about 12 hours is further preferable.

The high pressure gas treating step for treating microcapsules with carbon dioxide in a high pressure gaseous state will be illustrated in more detail below. Such a high pressure gas treating apparatus is made up, for example, of a liquefied carbonic acid bomb, a carbon dioxide supplying pump, a heat exchanger, an extracting vessel, a constant temperature bath, a detector, full automated pressure regulating valves and a recovering vessel. Microcapsules to be treated are placed in the extracting vessel, and the apparatus is sealed and warmed to a predetermined temperature. Then, liquefied carbon dioxide is fed with the carbon dioxide supplying pump from the liquefied carbon dioxide bomb to the heat exchanger, warmed to a predetermined temperature, and converted into the high pressure gas state. Then, this carbon dioxide in the high pressure gas state is blown into the extracting vessel, and a solvent in microcapsules is dissolved and extracted into the high pressure gas. The extracted solvent is recovered into the recovering vessel via the detector and the automated pressure regulating valves. The pressure to be applied to the entire system is controlled by the full automated pressure regulating valves connected to the downmost-stream. By allowing to contact with the high pressure gas for a predetermined time, the remaining organic solvent can be removed.

### (II) Phase separation method

For producing microcapsules by this method, an coacervating agent is gradually added to the organic solvent solution of the poorly water-soluble nonpeptidic physiologically active compound, the polyvalent metal compound and the biodegradable polymer obtained by the above-mentioned method with stirring to precipitate and solidify microcapsules. The volume of the coacervating agent is selected from the range of about 0.01 to about 1,000-fold, more preferably about 0.05 to about 500-fold, and in particular, about 0.1 to about 200-fold volume of the oil phase.

Any coacervating agent is acceptable as long as it is a polymer, mineral oil, vegetable oil, etc., that is miscible in the organic solvent and does not dissolve both of the physiologically active compound and the biodegradable polymer. For example, silicone oil, sesame oil, soybean oil, corn oil, cotton seed oil, coconut oil, linseed oil, mineral oil, n-hexane, n-heptane, etc. are employed. These may be used in combination of two or more thereof.

Thus-obtained microcapsules are separated, repeatedly washed with heptane, etc. to remove the coacervating agent, other than the physiologically active compound and the biodegradable polymer, and then dried under reduced pressure. Alternatively, the microcapsules may be washed by the method similar to that described in the above (I) in-water drying, and then subjected to freeze-drying, and further to removal of the solvent by warming and drying, using a supercritical fluid or carbon dioxide in a high pressure gaseous state, etc. As treatment for removing the solvent, the above-mentioned method using carbon dioxide in a high pressure gaseous state, etc. is preferably adopted.

### (III) Spray drying method

In producing microcapsules by this method, the organic solvent solution of the poorly water-soluble nonpeptidic physiologically active compound, the polyvalent metal compound and the biodegradable polymer obtained by the above-mentioned method is sprayed via a nozzle into the drying chamber of a spray drier to volatilize the organic solvent in the fine droplets in a very short period of time to prepare microcapsules. Examples of the nozzles include double-fluid nozzle, pressure nozzle, rotary disc nozzle, etc. Thereafter, if necessary, the microcapsules may be washed with the method similar to that described in the above (I) in-water drying, and then subjected to freeze-drying, and further to removal of the solvent by warming and drying, using a supercritical fluid or carbon dioxide in a high pressure gaseous state, etc.

### (IV) Air grinding method

As a dosage form other than the above-mentioned microcapsules, the organic solvent solution as described in (I) in-water drying method for producing microcapsules, which contains the poorly water-soluble nonpeptidic physiologically active compound, the polyvalent metal compound and the biodegradable polymer, said physiologically active compound dissolved in an amount exceeding the solubility thereof in the solution of biodegradable polymer and the organic solvent in the absence of the polyvalent metal compound, may be evaporated to dryness with regulating a degree of vacuum degree by using, for example, a rotary evaporator, and ground into a fine powder with a jet mill.

Further, the ground fine powder may be washed with the method similar to that described in the above (I) in-water drying method for producing microcapsules, and then subjected to freeze-drying, and further to removal of the solvent by warming and drying, using a supercritical fluid or carbon dioxide in a high pressure gaseous state, etc.

The release of the drug from the thus obtained microcapsules or fine powders can be controlled by degradation rate of the biodegradable polymer used and a kind and/or amount of the polyvalent metal compound.

The sustained-release preparation of the present invention can be administered as it is, or can be used for the production of various preparations as a raw material and administered as medicine such as injections or implants intramuscularly, subcutaneously, into organs, etc.; transmucosal preparations into nasal cavity, rectum, uterus, etc.; oral preparations (e.g., capsules (e.g., hard capsules, soft capsules, etc.), solid preparations such as granules, powders, etc., liquid preparations such as syrups, emulsions, suspensions, etc.); etc. Also, the sustained-release preparation of the present invention can be administered using a needleless injector.

For example, when the sustained-release preparation obtained by the process of the present invention is to be processed into a preparation for injection, it is dispersed together with a dispersing agent (e.g., surfactants such as Tween 80, HCO-60, etc., polysaccharides such as sodium hyaluronate, carboxymethylcellulose, sodium alginate, etc., etc.), a preservative (e.g., methylparaben, propylparaben, etc.), an isotonizing agent (e.g., sodium chloride, mannitol, sorbitol, glucose, proline, etc.), etc. to form an aqueous suspension, or it is dispersed together with vegetable oil such as sesame oil, corn oil, etc. to form an oily suspension, said suspensions being actually used as sustained-release preparations for injection.

The particle size of the sustained-release preparation obtained by the process of the present invention is selected from the range satisfying the dispersibility and needle-passability requirements when it is used as an injectable suspension. For example, the average diameter ranges from about 0.1 to about 300 µm, preferably from about 0.5 to about 150 µm, more preferably from about 1 to about 100 µm.

In order to prepare a sterile preparation of the sustained-release preparation obtained by the process of the present invention, a method for sterilizing all production steps, a method for sterilizing with γ-ray, a combined method thereof, a method for adding a preservative, etc. are employed, and there is no limitation to a specific method.

With low toxicity, the sustained-release preparation obtained by the process of the present invention can be used in mammals (e.g., human, bovine, swine, dog, cat, mouse, rat, rabbit, etc.) as safe medicine, etc.

The dose of the sustained-release preparation obtained by the process of the present invention is within the range of an effective amount of the physiologically active compound, varying depending on type, content and dosage form of the physiologically active compound as the active component; duration of release of the physiologically active compound; target disease; subject animal; etc. For example, the dose per administration of the active component, the physiologically active compound, is preferably chosen within the range from about 0.01 mg to about 10 mg/kg body weight per adult, more preferably from about 0.05 mg to about 5 mg/kg body weight per adult, when the sustained-release preparation is 1-month preparation.

The dose per administration of the sustained-release preparation is preferably chosen within the range from about 0.05 mg to about 50 mg/kg body weight per adult, more preferably from about 0.1 mg to about 30 mg/kg body weight per adult.

Number of administrations can be appropriately selected from once per a few weeks, once per a month, or once per a few months (e.g., 3 months, 4 months, 6 months, etc.), etc., depending on type, content and dosage form of the active component, the physiologically active compound, duration of release of the physiologically active compound, target diseases, subject animals, etc.

Further, the sustained-release preparation of the present invention can advantageously be used for patients who are bedridden, are suffered from dementia, pharynic and/or espophageal diseases, diseases of digestive organs, and disorder of eating and swallowing, and are difficult to be medicated by oral administration such as at the time of surgery, or the like.

In case that the physiologically active compound is a compound having All antagonistic activity, the compound possesses high safety and therefore acute increase of a blood level just after administration of it does not cause excess reduction of blood pressure. Thus, the sustained-release preparation of the present invention can be used for the diseases described below and can maintain a constant drug level in blood during day and night. Therefore, the dose and numbers of administration can be reduced, compared with administration of conventional oral preparations. Moreover, the change of blood drug level is not remarkable and condition of the patient does not change due to interruption of taking the drug, etc. Therefore, it is expected that the treatment effect of the drug become clearer by administration of the sustained-release preparation of the present invention.

As for the safety, the risk of excess decrease in blood pressure is low as compared with that in case of oral administration as described above in the situation of normal use. Even if excess pressure decrease takes place in case of an incident such as a traffic accident, or the like owing to loss of a large quantity of body fluids, pressure increase can immediately be done by intravenous administration of not only angiotensin II but also a medicine (such as catechol amine preparation) to be used usually in an emergency medical care field and continuous pressure increase is also possible by oral administration of a hypotension curing agent, so that not only acute caring treatment at the time of emergency but also long term treatment can be possible.

Examples of the objective diseases of the compound having angiotensin II antagonistic activity as the physiologically active compound include diseases which are caused or promoted to be caused by vascular contraction, proliferation, and organ dysfunction manifested through an angiotensin II receptor, existence of angiotensin II or factors induced by the presence of angiotensin II.

Examples of such diseases include systemic diseases such as hypertension, abnormality of diurnal blood pressure variation, heart diseases (e.g., cardiac hypertrophy, acute heart failure, chronic heart failure including congestive heart failure, cardiomyopathy, angina pectoris, myocarditis, arrhythmia, tachycardia, myocardial infarction, etc.), cerebrovascular disorder (e.g., asymptomatic cerebrovascular disorder, transient cerebral ischemia, cerebral apoplexy, cerebrovascular dementia, hypertensive cerebropathy, etc.), cerebral edema, cerebral circulatory disorder, recurrence and sequela of cerebrovascular disorder (neurotic symptom, psychic symptom, subjective symptom, disorder in daily living activities, etc.), ischemic peripheral circulatory disorder, myocardial ischemia, venous insufficiency, progression of cardiac insufficiency after myocardial infarction, diabetes mellitus, diabetic complications (diabetic retinopathy, diabetic nephropathy, diabetic neuropathy, etc.), renal diseases (nephritis, glomerulonephritis, glomerulosclerosis, renal failure, thrombotic vasculopathy, complications of dialysis, organ dysfunction including nephropathy by radiation damage, etc.) arteriosclerosis including atherosclerosis (aneurysm, coronary arteriosclerosis, cerebral arteriosclerosis, peripheral arteriosclerosis, etc.), vascular hypertrophy, vascular hypertrophy or obliteration and organ failure after intervention (percutaneous transluminal coronary angioplasty, stenting, coronary angioscopy, intravascular ultrasound, douche thrombolytic therapy, etc.), vascular re-obliteration and restenosis after bypass, polycythemia, hypertension, organ failure and vascular hypertrophy after transplantation, rejection after transplantation, ocular diseases (glaucoma, ocular hypertension, etc.), thrombosis, multiple organ failure, endothelial dysfunction, hypertensive tinnitus, other cardiovascular diseases (deep vein thrombosis, obstructive impairment, arteriosclerosis obliteran, obliterative thromboangiitis, transient cerebral circulation disorder, Raynaud's disease, Berger disease, etc.), metabolic and/or nutritional disorder (obesity, hyperlipemia, hypercholesterolemia, diabetes mellitus, impaired glucose tolerance, hyperuricacidemia, hyperkalemia, hypernatremia, etc.), nerve degeneration diseases (Alzheimer's disease, Parkinson's disease, amyotrophic lateral sclerosis, AIDS related cerebral symptom, etc.), central nervous system disorder (cerebral hemorrhage, cerebral infarct, their sequela, complication, head injury, spinal injury, cerebral edema, sensory malfunction, sensory functional disorder, autonomic nervous system disorder, autonomic nervous system malfunction, multiple sclerosis, etc.), dementia, defects of memory, disorder of consciousness, amnesia, anxiety symptom, catatonic symptom, disconform mental state, psychopathy, (depression, epilepsy, alcoholism, etc.), inflammatory diseases (diabetic complication such as retinopathy, nephropathy, neuropathy great vessel dysfunction; arthritis such as rheumatoid arthritis, osteoarthritis, rheumatoid myelitis, periostitis; inflammation after operation and injury; remission of swelling; pharyngitis; cystitis; pneumonia; atopic dermatitis; inflammatory intestinal diseases such as Crohn's disease, ulcerative colitis; meningitis; inflammatory ocular disease; inflammatory pulmonary diseases such as pneumonia, pulmonary silicosis, pulmonary sarcoidosis, pulmonary tuberculosis, etc.), allergic diseases (allergic coryza, conjunctivitis, gastrointestinal allergy, pollinosis, anaphylaxis, etc.), chronic obstructive pulmonary disease, interstitial pneumonia, pneumocytis carinni pneumonia, collagen diseases (e.g., systemic lupus erythematosus, scleroderma, polyarteritis, etc.), hepatic diseases (hepatitis including chronic hepatitis, hepatic cirrhosis, etc.), portal hypertension, digestive system disorder (gastritis, gastric ulcer, stomach cancer, stomach disorder after operation, dyspepsia, esophageal ulcer, pancreatitis, colon polyp, cholelithiasis, hemorrhoidal disease, etc.), blood and/or myelopoietic diseases (erythrocytosis, vascular purpura, autoimmune hemolytic anemia, disseminated intravascular coagulation, multiple myelopathy, etc.), bone diseases (fracture, refracture, osteoporosis, osteomalacia, bone Behcet's disease, sclerosing myelitis, rheumatoid arthritis, osteoarthrosis of the knee and joint tissue distruction owing to similar diseases and disorder, etc.), solid ulcer, ulcer [malignant melanoma, malignant lymphoma, cancer of digestive organs (e.g., cancer of stomach, intestine, etc.)], cancer and cachexia following cencer, metastasis cancer, endocrinopathy (Addison's disease, Cushing's syndrome, pheochromocytoma, primary aldosteronism and the like), Creutzfeldt-Jakob disease, urinary organ and/or male genital diseases (cystitis, prostatic hypertrophy, prostatic cancer, sex infectious disease, etc.), female disorder (climacteric disorder, gestosis, endometriosis, hysteromyoma, ovarian disease, breast disease, sex infectious disease, etc.), diseases and disorder related to environment and occupational factors (radiation hazard, hazard by ultraviolet, infrared, or laser beam, altitude sickness, etc.), respiratory diseases (cold syndrome, pneumonia, asthma, pulmonary hypertension, pulmonary thromboembolism, pulmonary thrombosis, etc.), infectious diseases (viral infectious disease by cytomegalovirus, influenza virus, herpes virus, etc., rikettsiosis, bacterial infectious diseases, etc.), toxemia (sepsis, sepsis shock, endotoxin shock, Gram-negative supsis, toxin shock syndrome, etc.), rhinolarygological diseases (Meniere's syndrome, tinnitus, dysgeusia, vertigo, disequilibrium, dysphagia, etc.), skin diseases (keloid, hemangioma, psoriasis, etc.), intradialytic hypotension, myasthenia gravis, chronic fatigue syndrome, and the like.

In case the physiologically active compound is a compound having angiotensin II antagonistic activity, by suppressing the action of angiotensin II for a long term, disorder and abnormality of biological and physiological functions which cause a variety of diseases accompanying adult disorder and aging can be improved, or accentuation of such disorder and abnormality can be controlled, thereby achieving primary and secondary prophylactic effects or controlling development of disease conditions caused by those diseases and symptoms. Such disorder and abnormality of biological and physiological functions include disorder and abnormality of automatic controlling capability of cerebral circulation and/or renal circulation, disorder of circulation (peripheral, cerebral, microcirculation and the like), disorder of blood-brain-barrier, insulin susceptibility deterioration, salt susceptibility deterioration, abnormal state of coagulation and fibrinolysis system, abnormal state of blood and blood cell components (accentuation of platelet agglutinability, abnormality of erythrocyte transformation, accentuation of leukocyte agglutininig function, rise of blood viscosity, etc.), production and function accentuation of growth factor and cytokine (PDGF, VEGF, FGF, interleukin, TNF-α, MCP-1, etc.), accentuation of proliferation and infiltration of inflammatory cells, accentuation of production of free radical, liposteatosis accentuation, endothelial function disorder, endothelial, cell, and organ dysfunction, cell morphogenesis change of edema, smooth muscle, and the like (morphogenesis to proliferation type), accentuation of production and function of vasoactive substance and thrombosis inducers (endothelin, thromboxane A₂, etc.), abnormal vasoconstriction, impaired glucose tolerance, metabolic disorder (serum lipid disorder, blood sugar disorder, etc.), abnormal cell propagation, vascular rebirth (including abnormal vasa vasorum formation in adventitial coat of atherosclerosis, abnormal capillary reticular formation), and the like. Among them, the compound can be advantageously used as a primary and secondary prophylactic drug for organ dysfunction accompanied with a variety of diseases (e.g. cerebrovascular disorder and organ dysfunction following the cerebrovascular disorder, organ dysfunction following cardiovascular diseases, organ dysfunction following diabetes mellitus, organ dysfunction after intervention).

In the sustained-release preparation obtained by the process of the present invention, when the physiologically active compound is a compound having angiotensin II antagonistic activity (especially, candesartan, cilexetil, candesartan, etc.), it can be advantageously used as a prophylactic and therapeutic drug for portal hypertension. It is well known that esophagovaritosis occurs frequently during night (Hepatology 1994; 19: 595-601), and since the preparation of the present invention is capable of maintaining a constant blood level all day long, the preparation of the present invention is not only capable of decreasing the dosage and the number of administration as compared with those in case of a preparation for oral administration, but also expected to realize stable lowering of portal vein pressure owing to the slight fluctuation of the blood level of the drug. The above characteristics of the preparation show usefulness as a preventive drug for rupture of varicose vein in esophagus and stomach. Further, since no symptom change is caused owing to interruption of the agent administration, it is also expected to clarify the therapeutic effect. Further, a compound having angiotensin II antagonistic activity as the physiologically active compound (especially, candesartan cilexetil, candesartan) is expected to be efficacious on the production and promotion of HGF (hepatocyte growth factor) and to attribute to liver regeneration and liver function restitution.

Further, it is expected to be able to further clarify the prophylactic and therapeutic efficacy on the cerebrovascular disorder such as cerebral infarct, etc. by maintaining a constant blood level of a compound having angiotensin II antagonistic activity as the physiologically active compound (especially, candesartan cilexetil, candesartan) all day long. In case the physiologically active compound is a compound having angiotensin II antagonistic activity, as a method for treating a patient, it can also be thought that a preparation for oral administration of the angiotensin II antagonist is firstly administered to the patient for a certain period to confirm the reaction of the patient, and then the sustained-release preparation of the present invention is administered. The angiotensin II antagonist to be used for the preparation for the oral administration may be the same as or different from the angiotensin II antagonist contained in the sustained-release preparation. In addition, a hypotensive other than the angiotensin II antagonist (e.g. calcium antagonist, diuretic, β-blocker, etc.) is firstly administered orally to confirm the reaction of a patient, and then the sustained-release preparation of the present invention can be administered. Further, a diuretic hypotensive drug (a preparation for oral administration) which is commonly used in combination with the angiotensin II antagonist may be used in combination with the sustained-release preparation of the present invention.

Further, the sustained-release preparation may be used in combination with other medical components including lipid decreasing drug or cholesterol decreasing drug, HMG-CoA reductase (3-hydroxy-3-methylglutaryl coenzyme A reductase) inhibitor, insulin sensitivity improving drug, bone disease therapeutic drug, myocardial protection drug, coronary arterial disease therapeutic drug, other hypertension therapeutic drug, chronic heart failure therapeutic drug, diabetic mellitus therapeutic drug, liver disease therapeutic drug, intestine and/or duodenal ulcer therapeutic drug, biliary disorder therapeutic drug, dysthyroidism therapeutic drug, nephrosis syndrome therapeutic drug, chronic renal failure therapeutic drug, female disease therapeutic drug, urinary organs and/or male genital disease therapeutic drug, and infection therapeutic drug. In this case, these compounds may be administered in the form of an oral preparation and in some cases, in the form of a rectal preparation or suppository. In this case, examples of components which can be used in combination include fibrates (e.g., clofibrate, benzafibrate, GM fibrozyl, etc.), nicotinic acid and its derivatives and analogues (e.g., acipimox, probucol), bile acid-conjugated resin (e.g. cholestyramine, questinol, etc.), compounds suppressing cholesterol absorption (e.g., sitosterol, neomycin, etc.) squalene epoxidase inhibitor (e.g., NB-598 and analogous compounds, etc.), and the like.

Examples of other components which can be used in combination include oxidosqualene-lanosterolcyclase such as decaline derivatives, azadecaline derivatives, and indane derivatives.

Furthermore, combinations with the following various therapeutic drugs are also possible.
Hypertension therapeutic drugs: diuretic [e.g., furosemide (Lasix), bemetanid (Lunetoron), azosemido (Diart)]; hypotensive drug [e.g., ACE inhibitor (enalapril maleate (Renivace), etc.), Ca antagonist (manidipine, amlodipine), α- or β-receptor inhibitor, etc.] and the like;
Chronic heart failure therapeutic drugs: cardiotonic [e.g., cardiotonic glycoside (Digoxin, etc.), β-receptor stimulus agent (catecholamin preparation such as denopamine and dobutamine), PDE inhibitor. etc.], diuretic agent [e.g., furosemide (Lasix), spironolactone (Aldacton), etc.], ACE inhibitor [e.g. enalapril maleate (Renivace), etc.], Ca antagonist (e.g., amlodipine), β-receptor inhibitor, and the like;
Antiarryhthmic drugs: disopyramide, lidocaine, quinidine sulfate, flecainide acetate, mexiletine hydrochloride, amiodarone, β-blocker, Ca antagonist, and the like;
Bone disease therapeutic drugs: calcium preparation (e.g. calcium carbonate, etc.), calcitonine preparation, activated type vitamin D₃ preparation [e.g., alfacalcidol (Alfarol), calcitriol (Rocaltrol), etc.], sex hormones (e.g., estrogen, estradiol, etc.), hormone preparation [e.g., estrogen conjugated (Premarin), etc.], ipriflavone preparation (Osten, etc.), vitamin K₂, vitamin K₂ preparation [e.g., menatetrenone (Glakay), etc.], bisphosphonate preparation (etidronate, etc.), prostaglandin E2, fluorine compound (e.g., sodium fluoride, etc.), bone matrix protein (BMP), fibroblast growth Factor (FGF), platelet-derived growth factor (PDGF), transforming growth factor (TGF-β), insulin-like growth factor 1 and 2 (IGF-1, -2), parathyroid hormone (PTH), compounds disclosed in EP-A1-376197, EP-A1-460488, and EP-A1-719782 [e.g., (2R,4S)-(-)-N-[4-(diethoxyphosphorylmethyl)phenyl]-1,2,4,5-tetrahydro-4-methyl-7,8-methylenedioxy-5-oxo-3-benzothiepin-2-carboxyamide, etc.], and the like;
Diabetes mellitus therapeutic drugs; Actos, rosiglitazone, Kinedak, Penfill, Humulin, Euglucon, Glimicron, Daonil, Novolin, Monotard, insulins, Glucobay, Dimelin, Rastinon, versylcone, Deamelin-S, Iszilin, and the like;
Liver disease therapeutic drugs: glycyrrhizin preparation (e.g., Strong Minophagen, etc.), liver lysosome, SH compound (e.g., glutathione, etc.), special amino acid preparation (e.g., Aminoleban, etc.), phospholipid (e.g., polyenephosphatidyl choline, etc.), vitamins (e.g., vitamin B₁, B₂, B₆, B₁₂, C, etc.), adrenocortical hormone (e.g., dexamethasone, betamethasone, etc.), interferon (e.g., interferon α, β, etc.), hepatic cerebropathy therapeutic drug (e.g., lactulose, etc.), hemostatic to be employed in esophagus and/or gastric varicosis rupture (e.g., vasopressin, somatostatin, etc.), and the like;
Gastric and/or duodenal ulcer therapeutic drugs: antiacid [e.g., histamine H2 receptor antagonists (cimetidine, etc.), proton pump inhibitor (lansoprazole, etc.), and the like;
Biliary tract therapeutic drugs: choleretic (e.g., dehydrocholic acid, etc.), cholekinetic (e.g., magnesium sulfate, etc.), and the like;
Hypothyroidism therapeutic drugs: dry thyroidea (Thyreoid), levothyroxine sodium (Thyradin-S), liothyronine sodium (Thyronin, Thyronamin), and the like;
Nephritic syndrome therapeutic drugs: for steroid therapy which is normally employed as primarily selection, prednisolon (Predonine), prednisolone sodium succinate (Predonine), methylprednisolone sodium succinate Solumedrol), betamethasone (Rinderone), and the like are used; and for anticoagulation therapy, dipyridamole (Persantin), dilazep dihydrochloride (Comelian), teclopidine, clopidogrel, antiplatelet drug such as Fxa inhibitor, etc. and anticoagulant are used;
HMG-CoA reductase inhibitors: cerivastatin, atorvastatin, provastatin, simvastatin, itavastatin, lovastatin, fluvastatin, (+)-3R,5S-7-(4-(4-fluorophenyl)-6-isopropyl-2-(N-methyl-N-methanesulfonylamino)pyridin-5-yl)-3,5-dihydroxy-6(E)-heptenoic acid, and the like;
Chronic renal failure therapeutic drugs: usable in combination with diuretic agent [e.g., furosemide (Lasix), bumetanid (Lunetoron), azosemido (Diart)], hypotensive drug [e.g., ACE inhibitor, enalapril maleate (Renivace)], calcium antagonist (manidipine) and α-adrenergic receptor antagonist, preferably by oral administration at the time of administration;
Thrombosis prophylactic drugs: anticoagulant [e.g., heparin sodium, heparin calcium, warfarin potassium (warfarin), blood coagulation factor Xa inhibitor and drugs capable of correcting balancing function of coagulation system], thrombolytic (e.g., tPA, urokinase), antiplatelet [e.g., aspirin, sulfinpyrazon (Anturan), dipyridamole (Persantin), ticropidine (Panaldine), cilostazol (Pletaal), GPIIb/IIIa antagonist (ReoPro)], and the like;
Coronary vasodilators: nifedipine, diltiazem, nicorandil, nitrite agent, and the like;
Cardioplegia drugs: opening drug for heart ATP-K, Na-H antiporters inhibitor, endothelin antagonist, urotensin antagonist, and the like;
Anti-inflammatory drugs: aspirin, acetaminophen, nonsteroidal anti-inflammatory drug (e.g., indometacine, etc.), steroid agent (e.g., dexamethasone, etc.), and the like;
Antiallergic drugs: antihistamine drug (e.g., chloropheniramine maleate, etc.), drug for stimulus therapy (e.g., bucillamine, etc.), azelastine hydrochloride, seratrodast, tranilast, oxatomide, Stronger Neo Minophagen C, tranexamic acid, ketotifen fumarate, and the like;
Antineoplastic drugs: alkylating agent, metabolic antagonist, antitumor antibiotic preparation, antitumor plant-derived component preparation, other antitumor drugs, and the like,
Central nervous system active drugs: anxiolytic, sleep sedative, anesthetic, antispasmodic, autonomic drug, drug for Parkinson's disease, drug for psychoneurosis, and the like;
Therapeutic drugs for female diseases: [e.g., therapeutic drug for climacteric disorder (estrogen conjugated, estradiol, testosterone enanthate, estradiol valerate, etc.), breast cancer therapeutic drug (tamoxifen citrate, etc.), endometriosis and/or myeoma uteri therapeutic drug (leuprorelin acetate, danazol, etc.)], and the like;
Urinary organ and/or male genital disease therapeutic drugs: [e.g., prostatic hypertrophy (tamsulosin hydrochloride, prazosin hydrochloride, chlormadinone acetate, etc.), prostatic cancer (leuprorelin acetate, goserelin acetate, chlormadinone acetate, etc.)], and the like;
Infectious disease therapeutic drugs: [e.g., antibiotic preparation (cefotiam dihydrochloride, cefozopran hydrochloride, ampicillin, etc.), chemotherapeutic drug (sulfonamido agent, synthetic antibacterial agent, antiviral substance, etc.), biological preparation (vaccines, blood derivatives such as immunogloblin, etc.), and the like]; and others.

Further, there are medical care by introduction of living body-derived various factors and their gene (e.g. ischemic disease therapeutic by vascular regeneration promoting factors such as HGF, VEGF and their gene introduction, etc.), care by decoy nucleic acids and the like.

In case of using the sustained-release preparation obtained by the process of the present invention in combination with these medical components, respective drugs may be incorporated into the single sustained-release preparation. Alternatively, the above-described medical components can be compounded with pharmacologically acceptable carriers, fillers, binders, diluent and the like to prepare respective preparations, and they are separately or simultaneously administered in combination with the sustained-release preparation of the present invention. In case preparations of these medical components are separately produced, the respective preparations can be mixed using a diluent upon administration, but the respective separate preparations can also be administered to a patient simultaneously or at certain time intervals.

### E. Regarding polyvalent metal (2)

The following will illustrate the polyvalent metal used in the "sustained-release solid pharmaceutical composition comprising a nonpeptidic physiologically active substance and a biodegradable polymer, wherein about 0.05% by weight based on the weight of the composition, or more of a polyvalent metal is present on the surface of the composition" of the present invention.

The polyvalent metal which is present on the surface of the sustained-release solid pharmaceutical composition of the present invention is not particularly limited as far as it is a compound having no adverse effect on a living body and, as a metal spices, polyvalent metals such as divalent metals (e.g. zinc, calcium, magnesium, iron, copper, aluminum, tin, manganese etc.), trivalent metals (e.g. iron, aluminum, manganese etc.), and tetravalent metals (e.g. tin etc.) (preferably, zinc etc.) are used.

The polyvalent metal which is present on the surface of the sustained-release solid pharmaceutical composition of the present invention may be used as a "polyvalent metal compound" such as a compound with inorganic or organic substances, a complex compound and a metal oxide. Further, the "polyvalent metal compound" may have bound water or crystallization water.

Preferable examples of the polyvalent metal include zinc, calcium, magnesium and the like. Particularly preferable examples of the polyvalent metal is zinc.

As the organic substance, organic acids such as aliphatic carboxylic acid and aromatic acid, and acetylacetone are used. As the aliphatic carboxylic acid, preferably, aliphatic C₁₋₉ carboxylic acids (e.g. aliphatic monocarboxylic acid, aliphatic dicarboxylic acid, aliphatic tricarboxylic acid etc.) are used. The aliphatic carboxylic acid may be saturated or unsaturated.

As the aliphatic monocarboxylic acid, for example, saturated C₁₋₉ aliphatic monocarboxylic acids (e.g. carbonic acid, formic acid, acetic acid, propionic acid, butyric acid, valeric acid, caproic acid, enanthic acid, caprylic acid, pelargonic acid, capric acid etc.) and unsaturated C₂₋₉ aliphatic monocarboxylic acids (e.g. acrylic acid, propiolic acid, methacrylic acid, crotonic acid, isocrotonic acid etc.) are used.

As the aliphatic dicarboxylic acid, for example, saturated C₂₋₉ aliphatic dicarboxylic acids (e.g. malonic acid, succinic acid, glutaric acid, adipic acid, pimelic acid etc.) and unsaturated C₂₋₉ aliphatic dicarboxylic acids (e.g. maleic acid, fumaric acid, citraconic acid, mesaconic acid etc.) are used.

As the aliphatic tricarboxylic acid, for example, saturated C₂₋₉ aliphatic tricarboxylic acids (e.g. tricarballylic acid, 1,2,3-butanetricarboxylic acid etc.) are used.

The above-mentioned aliphatic carboxylic acid may have 1 or 2 hydroxy group(s), and examples thereof include glycolic acid, lactic acid, glyceric acid, tartronic acid, malic acid, tartaric acid, citric acid and the like.

The aliphatic carboxylic acid is preferably aliphatic monocarboxylic acid, more preferably C₂₋₉ aliphatic monocarboxylic acid. Particularly preferable examples of the aliphatic carboxylic acid include acetic acid.

As the aromatic acid, for example, benzoic acid, salicylic acid, and phenolsulfonic acid are used.

Examples of inorganic substances include a halogen (e.g. fluorine, chlorine, bromine, iodine), an inorganic acid (e.g. sulfuric acid, nitric acid, thiocyanic acid), and oxygen.

Examples of the polyvalent metal compound include:
salts of zinc and inorganic acids [e.g. zinc halides (e.g. zinc chloride, zinc bromide, zinc iodide, zinc fluoride etc.), zinc sulfate, zinc nitrate, zinc thiocyanate etc.], salts of zinc and organic acids [e.g. zinc aliphatic carboxylates (e.g. zinc carbonate, zinc acetate, zinc glycolate, zinc lactate, zinc tartarate etc.), aromatic zinc salts (e.g. zinc benzoate, zinc salicylate, zinc phenolsulfonate etc.)], zinc acetylacetonate, etc.
salts of calcium and inorganic acids [e.g. calcium halides (e.g. calcium chloride, calcium bromide, calcium iodide, calcium fluoride etc.), calcium sulfate, calcium nitrate, calcium thiocyanate etc.], salts of calcium and organic acids [e.g. calcium aliphatic carboxylates (e.g. calcium carbonate, calcium acetate, calcium propionate, calcium oxalate, calcium tartarate, calcium lactate, calcium citrate, calcium gluconate etc.), aromatic calcium salts (e.g. calcium benzoate, calcium salicylate etc.)], calcium acetylacetonate, etc.
salts of magnesium and inorganic acids [e.g. magnesium halides (e.g. magnesium chloride, magnesium bromide, magnesium iodide, magnesium fluoride etc.), magnesium sulfate, magnesium nitrate, magnesium thiocyanate etc.], salts of magnesium and organic acids [e.g. magnesium aliphatic carboxylates (e.g. magnesium carbonate, magnesium acetate, magnesium propionate, magnesium oxalate, magnesium tartalate, magnesium lactate, magnesium citrate, magnesium gluconate etc.), aromatic magnesium salts (e.g. magnesium benzoate, magnesium salicylate etc.)], magnesium acetylacetonate, etc.
salts of iron and inorganic acids [e.g. iron halides (e.g. iron chloride, iron bromide, iron iodide, iron fluoride etc.), iron sulfate, iron nitrate, iron thioncyanate etc.], salts of iron and organic acids [e.g. iron aliphatic carboxylates (e.g. iron carbonate, iron acetate, iron glycolate, iron lactate, iron tartarate etc.), aromatic iron salts (e.g. iron benzonate, iron salicylate, iron phenolsulfonate etc.)], iron acetylacetonate, etc.

In addition, metal oxides (e.g. zinc oxide, iron oxide, calcium oxide, magnesium oxide, aluminium oxide, copper oxide, manganese oxide etc.) may be used.

As the polyvalent metal compound, preferably, zinc acetate, zinc sulfate, calcium acetate, magnesium acetate and the like are used. More preferably, zinc acetate or/and zinc sulfate are used.

As the polyvalent metal which is present on the surface of the sustained-release solid pharmaceutical composition of the present invention, the polyvalent metal compound used itself may be present, or the polyvalent metal in the form of a polyvalent metal ion may form a salt with the solid pharmaceutical composition (e.g. with the nonpeptidic physiologically active substance, the biodegradable polymer, or other additives in the composition; in particular, a functional group such as a carboxyl group present at a terminal of the biodegradable polymer).

In the present specification, the wordings "present on the surface" means that the polyvalent metal is present in the form of a film-like coating, a net, or a dotted form or is localized. As an aspect of the "present", the polyvalent metal may form a salt with the solid pharmaceutical composition as described above, or the polyvalent metal may be adhered or attached thereto, or may be present together with components of the solid pharmaceutical composition.

In the present invention, the "surface" refers to not only the outermost surface of the solid pharmaceutical composition, but also a site having a thickness of about 10% or less, preferably about 5% or less expressed by an average diameter from the outermost surface.

As a method for allowing the polyvalent metal to be present on the surface of the sustained-release solid pharmaceutical composition, physical, chemical, electrical or thermodynamic outer forces may be used. That is, there are methods by (1) spraying a solution containing the polyvalent metal (compound), (2) plating the polyvalent metal (compound), (3) adsorbing fine particles of the polyvalent metal (compound), (4) thermally fusing fine particles of the polyvalent metal (compound), and the like.

Among these methods, a preferable method for placing the polyvalent metal on the surface of the sustained-release solid pharmaceutical composition is a method for coating the surface of the sustained release solid-pharmaceutical composition with a solution containing the polyvalent metal (compound).

Preferably, a solvent to be used for dissolving the polyvalent metal is a poor solvent for the biodegradable polymer constituting the sustained-release solid pharmaceutical composition. Examples of a preferable solvent include distilled water, alcohol (methanol or ethanol), acetone, acetonitrile, ethyl acetate and the like. More preferable are distilled water and alcohol. These solvents may be used by mixing two or more thereof. The concentration of the polyvalent metal (compound) in a solution is preferably about 0.1 to about 80 mM, more preferably about 0.5 to about 60 mM, most preferably about 1 to about 40 mM.

Immersion of the sustained-release solid pharmaceutical composition into a solution of the polyvalent metal (compound) may be performed during preparation of the sustained-release solid pharmaceutical composition, or after molding thereof, preferably during the preparation. In particular, when the sustained-release solid pharmaceutical composition is prepared in the form of microcapsules using the in-water drying method described hereinafter, it is possible to allow the polyvalent metal to be present on the surface of the microcapsules effectively via a step for emulsifying the nonpeptidic physiologically active substance, the biodegradable polymer and the organic solvent in a water phase (outer water phase) in which the polyvalent metal (compound) is dissolved.

The amount of the polyvalent metal which is present on the surface of the sustained release solid pharmaceutical composition prepared by the above-mentioned process is about 0.05% by weight or more, preferably about 0.05% by weight to about 5% by weight, more preferably about 0.1% by weight to about 2% by weight.

The initial burst release rate of a drug upon administration of the sustained release solid pharmaceutical composition prepared by the above-mentioned process, in which the polyvalent metal is present on the surface, to a living body, is preferably suppressed to about 60% or less, more preferably about 50% or less, most preferably about 40% or less as compared with a sustained-release solid pharmaceutical composition in which the polyvalent metal is not present on the surface.

The wording "not present" of the polyvalent metal refers to the case where the amount of the polyvalent metal is less than 0.05% by weight, preferably 0.03% by weight, more preferably 0.01% by weight relative to the total weight of the sustained-release solid pharmaceutical composition.

The "initial burst release rate" refers to a ratio of an amount of a drug released for "a certain term after administration" relative to the total amount of the drug released during the "releasing term" after the sustained-release solid pharmaceutical composition is administered to a living body. The "certain term after administration" depends on the releasing period of the sustained-release solid pharmaceutical composition and, for example, when the "releasing term" is 24 hours, it is 4 hours, when the "releasing term" is 7 days, it is 0.5 day and, when the "releasing term" is 30 days or longer, it is 1 day.

As a method for determining the initial burst release rate of a drug of the sustained-release solid pharmaceutical composition, there are in vivo and in vitro calculating methods. As a method for calculating the initial burst release rate in vivo, there is a method wherein blood drug levels are measured after the sustained-release solid pharmaceutical composition is administered to a living body to obtain a drug-level vs time curve and a ratio of an area under the curve (AUC) after a certain term after administration relative to AUC during the entire releasing term is determined. Alternatively, there is a method for calculating the initial burst release rate based on an amount of a drug remaining in the sustained-release solid pharmaceutical composition after a certain term after administration and the total amount of the drug administered. In a method for calculating the initial burst release rate in vitro, the rate can be obtained by dividing an amount of a drug released into a release test solution in a predetermined term by the total amount of the drug containing in the sustained-release solid pharmaceutical composition used in the assessment. Preferably, as the release test solution, various buffers (e.g. phosphate buffer, carbonate buffer, HEPES, citrate buffer etc.) are used. Further, various surfactants (Tween 20, Tween 80, Pruronics, SDS etc.) may be added to the release test solution. The predetermined period of time depends on the period of the release test and, when the release period is 24 hours, it is 1 hour or shorter, when the release peirod is 1 week, it is about 0.2 to 0.5 day and, the release period is 1 month, it is about 1 day.

### F. Regarding internal metal

In the sustained-release solid pharmaceutical composition of the present invention, the polyvalent metal may be incorporated into a site other than the surface in addition to the surface. The polyvalent metal is not particularly limited as far as it is a compound having no adverse affect on a living body and, as a metal spices, polyvalent metals such as divalent metals (e.g. zinc, calcium, magnesium, iron, copper, aluminum, tin, manganese etc.), trivalent metals (e.g. iron, aluminum, manganese etc.), and tetravalent metals (e.g. tin etc.) (preferably, zinc etc.) are used.

The polyvalent metal which may be incorporated into a site other than the surface may be used as a "polyvalent metal compound" such as a compound with inorganic or organic substances, a complex compound and a metal oxide. Further, the "polyvalent metal compound" may have bound water or crystallization water.

Preferable examples of the polyvalent metal include zinc, calcium, magnesium and the like. Particularly preferable examples of the polyvalent metal is zinc.

As the organic substance, organic acids such as aliphatic carboxylic acid and aromatic acid, and acetylacetone are used. As the aliphatic carboxylic acid, preferably, C₁₋₉ aliphatic carboxylic acids (e.g. aliphatic monocarboxylic acid, aliphatic dicarboxylic acid, aliphatic tricarboxylic acid etc.) are used. The aliphatic carboxylic acid may be saturated or unsaturated.

As the aliphatic monocarboxylic acid, for example, saturated C₁₋₉ aliphatic monocarboxylic acids (e.g. carbonic acid, formic acid, acetic acid, propionic acid, butyric acid, valeric acid, caproic acid, enanthic acid, caprylic acid, pelargonic acid, capric acid etc.) and unsaturated C₂₋₉ aliphatic monocarboxylic acids (e.g. acrylic acid, propiolic acid, methacrylic acid, crotonic acid, isocrotonic acid etc.) are used.

As the aliphatic dicarboxylic acid, for example, saturated C₂₋₉ aliphatic dicarboxylic acids (e.g. malonic acid, succinic acid, glutaric acid, adipic acid, pimelic acid etc.) and unsaturated C₂₋₉ aliphatic dicarboxylic acids (e.g. maleic acid, fumaric acid, citraconic acid, mesaconic acid etc.) are used.

As the aliphatic tricarboxylic acid, for example, saturated C₂₋₉ aliphatic tricarboxylic acids (e.g. tricarballylic acid, 1,2,3-butanetricarboxylic acid etc.) are used.

The above-mentioned aliphatic carboxylic acid may have 1 or 2 hydroxy group(s), and examples thereof include glycolic acid, lactic acid, glyceric acid, tartronic acid, malic acid, tartaric acid, citric acid and the like.

The aliphatic carboxylic acid is preferably aliphatic monocarboxylic acid, more preferably aliphatic C₂₋₉ monocarboxylic acid. Particularly preferable examples of the aliphatic carboxylic acid include acetic acid.

As the aromatic acid, for example, benzoic acid, salicylic acid, and phenolsulfonic acid are used.

Examples of the inorganic substance include a halogen (e.g. fluorine, chlorine, bromine, iodine), an inorganic acid (e.g. sulfuric acid, nitric acid, thiocyanic acid), and oxygen.

Examples of the polyvalent metal compound which may be incorporated into a site other than the surface include:
salts of zinc and inorganic acids [e.g. zinc halides (e.g. zinc chloride, zinc bromide, zinc iodide, zinc fluoride etc.), zinc sulfate, zinc nitrate, zinc thiocyanate etc.], salts of zinc and organic acids [e.g. zinc aliphatic carboxylates (e.g. zinc carbonate, zinc acetate, zinc glycolate, zinc lactate, zinc tartarate etc.), aromatic zinc salts (e.g. zinc benzoate, zinc salicylate, zinc phenolsulfonate etc.)], zinc acetylacetonate, etc.
salts of calcium and inorganic acids [e.g. calcium halides (e.g. calcium chloride, calcium bromide, calcium iodide, calcium fluoride etc.), calcium sulfate, calcium nitrate, calcium thiocyanate etc.], salts of calcium and organic acids [e.g. calcium aliphatic carboxylates (e.g. calcium carbonate, calcium acetate, calcium propionate, calcium oxalate, calcium tartarate, calcium lactate, calcium citrate, calcium gluconate etc.), aromatic calcium salts (e.g. calcium benzoate, calcium salicylate etc.)], calcium acetylacetonate, etc.
salts of magnesium and inorganic acids [e.g. magnesium halides (e.g. magnesium chloride, magnesium bromide, magnesium iodide, magnesium fluoride etc.), magnesium sulfate, magnesium nitrate, magnesium thiocyanate etc.], salts of magnesium and organic acids [e.g. magnesium aliphatic carboxylates (e.g. magnesium carbonate, magnesium acetate, magnesium propionate, magnesium oxalate, magnesium tartalate, magnesium lactate, magnesium citrate, magnesium gluconate etc.), aromatic magnesium salts (e.g. magnesium benzoate, magnesium salicylate etc.)], magnesium acetylacetonate, etc.
salts of iron and inorganic acids [e.g. iron halides (e.g. iron chloride, iron bromide, iron iodide, iron fluoride etc.), iron sulfate, iron nitrate, iron thioncyanate etc.], salts of iron and organic acids [e.g. iron aliphatic carboxylates (e.g. iron carbonate, iron acetate, iron glycolate, iron lactate, iron tartarate etc.), aromatic iron salts (e.g. iron benzonate, iron salicylate, iron phenolsulfonate etc.)], iron acetylacetonate, etc.
metal oxides (e.g. zinc oxide, iron oxide, calcium oxide, magnesium oxide, aluminium oxide, copper oxide, manganese oxide etc.).

As the polyvalent metal (compound) which may be incorporated into a site other than the surface, preferably, zinc acetate, zinc oxide, calcium acetate, magnesium acetate, iron chloride, iron acetylacetonate, zinc acetylacetonate, calcium acetylacetonate, magnesium acetylacetonate and the like are used and, more preferably, zinc acetate or/and zinc oxide (that is, either one selected from zinc acetate and zinc oxide or a combination thereof) are used.

In the present invention, a part of the polyvalent metal contained in the sustained-release solid pharmaceutical composition may form a metal salt (e.g. zinc salt or a heterogeneous metal salt etc.) with the biodegradable polymer. This metal salt of the biodegradable polymer can be prepared by the method described, for example, in JP-A 09-221420 or an analogous method. Alternatively, a part of the polyvalent metal contained in the sustained-release solid pharmaceutical composition may form a complex with the nonpeptidic physiologically active substance.

The mixing ratio of the polyvalent metal (compound) which is present at a site other than the surface relative to the nonpeptidic physiologically active substance is varied depending on particular kinds of the polyvalent metal (compound) and the nonpeptidic physiologically active substance and their optimal amounts to be added are different. However, for example, when the polyvalent metal (compound) is divalent and the nonpeptidic physiologically active substance is an All antagonistic compound, the ratio of polyvalent metal (compound)/nonpeptidic physiologically active substance (molar ratio) is preferably about 1/10 to about 10/1, more preferably about 1/5 to about 5/1, most preferably about 1/2 to about 2/1.

### G. Embodiment (2)

The following will illustrate a process for producing the "sustained-release solid pharmaceutical composition comprising a nonpeptidic physiologically active substance and a biodegradable polymer, wherein about 0.05% by weight based on the weight of the composition, or more of a polyvalent metal is present on the surface of the composition" of the present invention and embodiments thereof.

The sustained-release solid pharmaceutical composition of the present invention can be produced, for example, by removing an organic solvent from an emulsion obtained by mixing an organic solvent solution or suspension containing the nonpeptidic physiologically active substance and the biodegradable polymer, with a water phase having the polyvalent metal ion concentration of about 0.1 to about 80 mM.

At this time, the solubility of the nonpeptidic physiologically active substance in an organic solvent solution in the absence of the polyvalent metal (compound) is increased by the presence of the polyvalent metal (compound), and the solubility of the nonpeptidic physiologically active substance in the biodegradable polymer organic solvent solution in the absence of the polyvalent metal (compound) is increased by the presence of the polyvalent metal (compound).

Therefore, in a process for producing the sustained-release solid pharmaceutical composition of the present invention, for example, there may be used an organic solvent solution containing the nonpeptidic physiologically active substance in an amount exceeding the solubility thereof in the biodegradable polymer and the an organic solvent containing no polyvalent metal (compound), the polyvalent metal (compound) and the biodegradable polymer. Even when the biodegradable polymer is not contained in the organic solvent solution, the nonpeptidic physiologically active substance may be dissolved in the organic solvent solution in such an amount exceeding the solubility thereof in the solution in the absence of the polyvalent metal (compound).

Examples of the organic solvent to be used include halogenated hydrocarbons (e.g. dichloromethane, chloroform, dichloroethane, trichloroethane, carbon tetrachloride etc.), ethers (e.g. ethyl ether, isopropyl ether etc.), fatty acid esters (e.g. ethyl acetate, butyl acetate etc.), aromatic hydrocarbons (e.g. benzene, toluene, xylene etc.), alcohols (e.g. ethanol, methanol etc.), acetonitrile, etc. These may be used by mixing at an appropriate ratio. Inter alia, as the halogenated hydrocarbon, dichloromethane is suitable and, as the alcohol, ethanol and methanol are suitable. These may be used by mixing at an appropriate ratio.

Additives may be added to the above-mentioned organic solvent solution. Examples of the additives include a solubilizer which maintains stability of the drug such as acetic acid, carbonic acid, oxalic acid, citric acid, phosphoric acid, hydrochloric acid, etc., sodium hydroxide, arginine, lysine and their salts, etc. Further, as stabilizers of the drug, there can be added, for example, albumin, gelatin, citric acid, sodium ethylenediaminetetraacetate, dextrin, sodium hydrogensulfite, polyols such as polyethyleneglycol, etc., etc., and as preservatives there can be added, for example, conventional para-oxybenzoic acid esters (e.g., methylparaben, propylparaben, etc.), benzylalcohol, chlorobutanol, thimerosal, etc.

The concentration of the nonpeptidic physiologically active substance in the organic solvent solution is varied depending on a kind of the nonpeptidic physiologically active substance and a kind of the organic solvent, and is generally about 0.5 to about 70% by weight, more preferably about 1 to about 60% by weight, particularly preferably about 2 to about 50% by weight in case that the nonpeptidic physiologically active substance is an All antagonistic compound, and a mixed solution of dichloromethane and methanol is used as the organic solvent.

The concentration of the biodegradable polymer in the organic solvent solution is varied depending on a molecular weight of the biodegradable polymer and a kind of the organic solvent, and is generally about 0.5 to about 70% by weight, more preferably about 1 to about 60% by weight, particularly preferably about 2 to about 50% by weight in case of using dichloromethane as the organic solvent.

Further, when ethanol or methanol is used as the organic solvent to be mixed with dichloromethane, a ratio relative to dichloromethane in the mixed organic solvent is generally selected from about 10 to about 98% by volume, more preferably about 20 to about 95% by volume, particularly preferably about 30 to about 90% by volume.

As the order of addition of the nonpeptidic physiologically active substance, the polyvalent metal (compound) and the biodegradable polymer to the organic solvent, any one may be added first, or they may be added at the same time, or an organic solvent solution of the nonpeptidic physiologically active substance and the polyvalent metal (compound) and an organic solvent solution of the biodegradable polymer may be mixed.

In order to dissolve the nonpeptidic active substance, the polyvalent metal (compound) and the biodegradable polymer in the organic solvent, they may be allowed to stand, or they may be mixed by appropriately using outer energy. As the energy, there are ultrasonication, physical shaking, stirring and the like. The dissolution may be carried out at any temperature as far as it is not higher than the boiling point of a solvent to be used, and it is preferably 0°C to 30°C. Time required for dissolution is varied depending on kinds of the nonpeptidic physiologically active substance, the polyvalent metal (compound) and the biodegradable polymer, a blending ratio, a dissolution temperature, vibration and the like, and is preferably 1 second to 7 days, more preferably 5 seconds to 3 days, most preferably 30 seconds to 1 day at room temperature.

As a method of removing the organic solvent, a per se known method or an analogous method is used. For example, there are a method for evaporating the organic solvent at normal pressure or by gradually evacuating with stirring by a propeller-type stirrer or a magnetic stirrer, and a method for evaporating the organic solvent with regulating a degree of vacuum by using a rotary evaporator.

Treating time for removing the solvent is 0.1 to 20 hours, preferably 0.5 to 10 hours.

The organic solvent solution of the nonpeptidic physiologically active substance, the polyvalent metal (compound) and the biodegradable polymer is preferably a sterile solution, more preferably a solution prepared by filter-sterilization. When it is used as a preparation for injection, there is an advantage that a sterile bulk of the nonpeptidic physiologically active substance is not needed.

Amounts of the nonpeptidic physiologically active substance and the polyvalent metal compound to be formulated in the sustained-release solid pharmaceutical composition of the present invention are varied depending on a kind of the physiologically active compound, the desired pharmacological effect and a duration term of the effect and the like. However, when the physiologically active substance, the polyvalent metal (compound) and the biodegradable polymer are used as starting materials, relative to the total amount of these three materials, the amount of the physiologically compound is usually about 1 to about 50% by weight, more preferably 15 to 40% by weight, particularly preferably about 20 to 40% by weight and, on the other hand, the amount of the polyvalent metal (compound) is usually about 0.5 to about 20% by weight, more preferably about 1 to about 15% by weight, particularly preferably about 2 to about 10% by weight.

The dosage form of the sustained-release solid pharmaceutical composition of the present invention is not particularly limited, but a parenteral preparation is preferable and a transdermal preparation, a transmucous preparation, an implant and a microcapsule injectable preparation are contemplated. A preparation for injection using microcapsules which has a long sustained release term and has less burden to a patient is preferable.

The following will illustrate a process for producing the sustained-release solid pharmaceutical composition of the present invention, by using microcapsules (hereinafter, referred to as microspheres in some cases) as an example of a preparation.

### (I) In-water drying method

The organic solvent solution or suspension of the nonpeptidic physiologically active substance, the polyvalent metal (compound) and the biodegradable polymer obtained by the above-mentioned method is mixed with a aqueous solution in which the polyvalent metal (compound) has been dissolved, i.e., a water phase (hereinafter referred to as an outer water phase in some cases) to form an O (oil phase)/W (water phase) or S (solid phase)/O (oil phase)/W (water phase) emulsion. Then, a solvent in the oil phase is evaporated to prepare microcapsules. The polyvalent metal (compound) used here is selected from the above-mentioned ones and, for example, zinc acetate, zinc sulfate, calcium acetate, magnesium acetate or a mixture of two or more thereof is dissolved so that a concentration of the polyvalent metal ion is about 0.1 to about 80 mM. At this time, the volume of the water phase is generally selected from about 1-fold to about 10,000-fold, more preferably about 5-fold to about 5,000-fold, particularly preferably about 10-fold to about 2,000-fold volume of the oil phase. Further, In the water phase, a water miscible solvent such as alcohol (e.g. methanol or ethanol), acetone, acetonitrile, etc. may be mixed.

An emulsifying agent may be added to the outer water phase. Any emulsifying agent which can generally form a stable O/W emulsion may be added. Specifically, anionic surfactants (sodium oleate, sodium stearate, sodium laurylsulfate etc.), nonionic surfactants (polyoxyethylene sorbitan fatty acid esters [Tween 80, Tween 60, Atlas Powder], polyoxyethylene castor oil derivatives [HCO-60, HCO-50, Nikko Chemicals] etc.), polyvinylpyrrolidone, polyvinyl alcohol, carboxymethylcellulose, lecithin, gelatin and hyaluronic acid, etc., are used. These may be used alone or in combination of 2 or more thereof. Upon use, the concentration is preferably in a range of about 0.01 to 10% by weight, more preferably in a range of about 0.05 to about 5% by weight.

An osmotic pressure adjusting agent may be added to the outer water phase. Any osmotic pressure adjusting agent may be used as far as it show the osmotic pressure when formulated into an aqueous solution. Examples thereof include polyhydric alcohols, monohydric alcohols, monosaccharides, disaccharides, oligosaccharides and amino acids, and derivatives thereof.

As the polyhydric alcohols, for example, dihydric alcohols such as glycerin and the like, pentahydric alcohols such as arabitol, xylitol, adnitol and the like, and hexahydric alcohols such as mannitol, sorbitol, dulcitol and the like are used. Inter alia, hexahydric alcohols are preferable and, in particular, mannitol is suitable.

Examples of the monohydric alcohols include methanol, ethanol, isopropyl alcohol, etc., and, among them, methanol is preferable.

As the monosaccharides, for example, pentoses such as arabinose, xylilose, ribose, 2-deoxyribose and the like, and hexoses such as glucose, fructose, galactose, mannose, sorbose, rhamnose, fucose and the like are used and, among them, hexoses are preferable.

As the oligosaccharides, trioses such as maltotriose, raffinose and the like, and tetroses such as stachyose and the like are used and, among them, trioses are preferable.

As the derivatives of monosaccharides, disaccharides and oligosaccharides, for example, glucosamine, galactosamine, glucuronic acid, galacturonic acid and the like are used.

As the amino acids, any amino acids can be used as far as they are L-isomers, such as glycine, leucin, arginine and the like. Among them, L-arginine is preferable.

These osmotic pressure adjusting agents may be used alone or by mixing them.

These osmotic pressure adjusting agents are used at such a concentration that osmotic pressure of an outer water phase becomes about 1/50-fold to about 5-fold, preferably about 1/25-fold to 3-fold osmotic pressure of physiological saline.

As a method for removing the organic solvent, a per se known method or an analogous method is used. Example thereof include a method for evaporating the organic solvent at normal pressure or by gradually evacuating while stirring with a propeller-type stirrer or a magnetic stirrer, and a method for evaporating the organic solvent while regulating a degree of vacuum using a rotary evaporator.

Thus obtained microcapsules are collected by centrifugation or filtration, the physiologically active substance, a drug retaining substance and an emulsifying agent and the like adhered to the surfaces of microcapsules are repeatedly washed with distilled water several times, and the microcapsules are dispersed in distilled water, followed by lyophilization.

During the production process, an aggregation preventing agent may be added in order to prevent aggregation of particles. As the aggregation preventing agent, for example, mannitol, lactose, glucose, water soluble polysaccharides such as starches (e.g. corn starch etc.), amino acid such as glycine, and protein such as fibrin and collagen are used. Inter alia, mannitol is suitable.

Further, after lyophilization, if necessary, moisture and the organic solvent in microcapsules may be removed by warming under reduced pressure under such conditions that microcapsules are not fused together. Preferably, warming is performed at a temperature slightly higher than the intermediate point glass transition temperature of the biodegradable polymer obtained by a differential scanning calorimeter, preferably under conditions of a temperature raising rate of 10 to 20°C per minute. More preferably, warming is performed at a temperature range from the intermediate point glass transition temperature of the biodegradable polymer to a temperature about 30°C higher than it. Inter alia, when a lactic acid-glycolic acid copolymer is used as the biodegradable polymer, warming is performed in a temperature range, preferably, from its intermediate point glass transition temperature to a temperature 10°C higher than the intermediate point glass transition temperature, more preferably in a temperature range from an intermediate point glass transition temperature to a temperature 5°C higher than the intermediate point glass transition temperature.

Heating time is varied depending on an amount of microcapsules, etc., and is generally about 12 hours to about 168 hours, preferably about 24 hours to about 120 hours, particularly preferably about 48 hours to about 96 hours after a temperature of microcapsules themselves has reached a predetermined temperature.

The warming method is not particularly limited as far as it can warm collected microcapsules uniformly.

As the warming drying method, for example, a method for warming and drying in a constant temperature bath, a fluidizing bed, a moving bath or a kiln, and a method for warming and drying with a microwave are used. Among them, a method of warming and drying in a constant temperature bath is preferable.

Alternatively, removal of moisture and the organic solvent in microcapsules may be performed by a method using a supercritical fluid (CO₂ etc.) or carbon dioxide in a high pressure gaseous state. Preferably, a method using carbon dioxide in a high pressure gaseous state is adopted.

### (II) Phase separation method

For producing microcapsules by this method, an coacervating agent in which one or more polyvalent metals are dissolved is gradually added to the organic solvent solution or suspension of the nonpeptidic physiologically active substance, the polyvalent metal (compound) and the biodegradable polymer obtained by the above-mentioned method with stirring to precipitate and solidify microcapsules. The polyvalent metal is selected from the above-mentioned ones and, for example, zinc caprylate, zinc enanthate, zinc caprate, zinc benzoate, zing slicylate or a mixture of two or more thereof is preferred and is dissolved at a concentration of about 0.1 to about 80 mM. The volume of the coacervating agent is selected from the range of about 0.01 to about 1,000-fold, more preferably about 0.05 to about 500-fold, and in particular, about 0.1 to about 200-fold volume of the oil phase.

Any coacervating agent is acceptable as long as it is a polymer, mineral oil, vegetable oil, etc., that is miscible in the organic solvent and does not dissolve both of the physiologically active compound and the biodegradable polymer. For example, silicone oil, sesame oil, soybean oil, corn oil, cotton seed oil, coconut oil, linseed oil, mineral oil, n-hexane, n-heptane, etc. are employed. These may be used in combination of two or more thereof.

Thus-obtained microcapsules are separated, repeatedly washed with heptane, etc. to remove the coacervating agent, other than the physiologically active substance and the biodegradable polymer, and then dried under reduced pressure. Alternatively, the microcapsules may be washed by the method similar to that described in the above (I) in-water drying, and then subjected to freeze-drying, and further to removal of the solvent by warming and drying, using a supercritical fluid or carbon dioxide in a high pressure gaseous state, etc.

The release of the drug from the thus obtained microcapsules or fine powders can be controlled by degradation rate of the biodegradable polymer used and a kind and/or amount of the polyvalent metal (compound).

The sustained-release solid pharmaceutical composition of the present invention can be administered as it is, or can be used for the production of various preparations as a raw material and administered as medicine such as injections or implants intramuscularly, subcutaneously, into organs, etc.; transmucosal preparations into nasal cavity, rectum, uterus, etc.; oral preparations (e.g., capsules (e.g., hard capsules, soft capsules, etc.), solid preparations such as granules, powders, etc., liquid preparations such as syrups, emulsions, suspensions, etc.); etc. Also, the sustained-release solid pharmaceutical composition of the present invention can be administered using a needleless injector.

For example, when the sustained-release solid preparation obtained by the process of the present invention is to be processed into a preparation for injection, it is dispersed together with a dispersing agent (e.g., surfactants such as Tween 80, HCO-60, etc., polysaccharides such as sodium hyaluronate, carboxymethylcellulose, sodium alginate, etc., etc.), a preservative (e.g., methylparaben, propylparaben, etc.), an isotonizing agent (e.g., sodium chloride, mannitol, sorbitol, glucose, proline, etc.), etc. to form an aqueous suspension, or it is dispersed together with vegetable oil such as sesame oil, corn oil, etc. to form an oily suspension, said suspensions being actually used as sustained-release preparations for injection.

The particle size of the sustained-release solid pharmaceutical composition obtained by the process of the present invention is selected from the range satisfying the dispersibility and needle-passability requirements when it is used as an injectable suspension. For example, the average diameter ranges from about 0.1 to about 300 µm, preferably from about 0.5 to about 150 µm, more preferably from about 1 to about 100 µm.

In order to prepare a sterile preparation of the sustained-release solid preparation obtained by the process of the present invention, a method for sterilizing all production steps, a method for sterilizing with γ-ray, a combined method thereof, a method for adding a preservative, etc. are employed, and there is no limitation to a specific method.

With low toxicity, the sustained-release solid preparation obtained by the process of the present invention can be used in mammals (e.g., human, bovine, swine, dog, cat, mouse, rat, rabbit, etc.) as safe medicine, etc.

The dose of the sustained-release solid pharmaceutical composition obtained by the process of the present invention is within the range of an effective amount of the physiologically active substance, varying depending on type, content and dosage form of the nonpeptidic physiologically active substance as the active component; duration of release of the nonpeptidic physiologically active substance; target disease; subject animal; etc. For example, the dose per administration of the active component, the nonpeptidic physiologically active substance, is preferably chosen within the range from about 0.01 mg to about 10 mg/kg body weight per adult, more preferably from about 0.05 mg to about 5 mg/kg body weight per adult, when the sustained-release solid preparation is 1-month preparation.

The dose per administration of the sustained-release solid preparation is preferably chosen within the range from about 0.05 mg to about 50 mg/kg body weight per adult, more preferably from about 0.1 mg to about 30 mg/kg body weight per adult.

Number of administrations can be appropriately selected from once per a few weeks, once per a month, or once per a few months (e.g., 3 months, 4 months, 6 months, etc.), etc., depending on type, content and dosage form of the active component, the nonpeptidic physiologically active substance, duration of release of the nonpeptidic physiologically active substance, target diseases, subject animals, etc.

Further, the sustained-release solid preparation of the present invention can advantageously be used for patients who are bedridden, are suffered from dementia, pharynic and/or espophageal diseases, diseases of digestive organs, and disorder of eating and swallowing, and are difficult to be medicated by oral administration such as at the time of surgery, or the like.

In case that the nonpeptidic physiologically active substance is a compound having All antagonistic activity, the compound possesses high safety and therefore acute increase of a blood level just after administration of it does not cause excess reduction of blood pressure. Thus, the sustained-release solid pharmaceutical composition of the present invention can be used for the diseases described below and can maintain a constant drug level in blood during day and night. Therefore, the dose and numbers of administration can be reduced, compared with administration of conventional oral preparations. Moreover, the change of blood drug level is not remarkable and condition of the patient does not change due to interruption of taking the drug, etc. Therefore, it is expected that the treatment effect of the drug become clearer by administration of the sustained-release solid preparation of the present invention.

As for the safety, the risk of excess decrease in blood pressure is low as compared with that in case of oral administration as described above in the situation of normal use. Even if excess pressure decrease takes place in case of an incident such as a traffic accident, or the like owing to loss of a large quantity of body fluids, pressure increase can immediately be done by intravenous administration of not only angiotensin II but also a medicine (such as catechol amine preparation) to be used usually in an emergency medical care field and continuous pressure increase is also possible by oral administration of a hypotension curing agent, so that not only acute caring treatment at the time of emergency but also long term treatment can be possible.

Examples of the objective diseases of the compound having angiotensin II antagonistic activity as the nonpeptidic physiologically active substance include diseases which are caused or promoted to be caused by vascular contraction, proliferation, and organ dysfunction manifested through an angiotensin II receptor, existence of angiotensin II or factors induced by the presence of angiotensin II.

Examples of such diseases include systemic diseases such as hypertension, abnormality of diurnal blood pressure variation, heart diseases (e.g., cardiac hypertrophy, acute heart failure, chronic heart failure including congestive heart failure, cardiomyopathy, angina pectoris, myocarditis, arrhythmia, tachycardia, myocardial infarction, etc.), cerebrovascular disorder (e.g., asymptomatic cerebrovascular disorder, transient cerebral ischemia, cerebral apoplexy, cerebrovascular dementia, hypertensive cerebropathy, etc.), cerebral edema, cerebral circulatory disorder, recurrence and sequela of cerebrovascular disorder (neurotic symptom, psychic symptom, subjective symptom, disorder in daily living activities, etc.), ischemic peripheral circulatory disorder, myocardial ischemia, venous insufficiency, progression of cardiac insufficiency after myocardial infarction, diabetes mellitus, diabetic complications (diabetic retinopathy, diabetic nephropathy, diabetic neuropathy, etc.), renal diseases (nephritis, glomerulonephritis, glomerulosclerosis, renal failure, thrombotic vasculopathy, complications of dialysis, organ dysfunction including nephropathy by radiation damage, etc.) arteriosclerosis including atherosclerosis (aneurysm, coronary arteriosclerosis, cerebral arteriosclerosis, peripheral arteriosclerosis, etc.), vascular hypertrophy, vascular hypertrophy or obliteration and organ failure after intervention (percutaneous transluminal coronary angioplasty, stenting, coronary angioscopy, intravascular ultrasound, douche thrombolytic therapy, etc.), vascular re-obliteration and restenosis after bypass, polycythemia, hypertension, organ failure and vascular hypertrophy after transplantation, rejection after transplantation, ocular diseases (glaucoma, ocular hypertension, etc.), thrombosis, multiple organ failure, endothelial dysfunction, hypertensive tinnitus, other cardiovascular diseases (deep vein thrombosis, obstructive impairment, arteriosclerosis obliteran, obliterative thromboangiitis, transient cerebral circulation disorder, Raynaud's disease, Berger disease, etc.), metabolic and/or nutritional disorder (obesity, hyperlipemia, hypercholesterolemia, diabetes mellitus, impaired glucose tolerance, hyperuricacidemia, hyperkalemia, hypernatremia, etc.), nerve degeneration diseases (Alzheimer's disease, Parkinson's disease, amyotrophic lateral sclerosis, AIDS related cerebral symptom, etc.), central nervous system disorder (cerebral hemorrhage, cerebral infarct, their sequela, complication, head injury, spinal injury, cerebral edema, sensory malfunction, sensory functional disorder, autonomic nervous system disorder, autonomic nervous system malfunction, multiple sclerosis, etc.), dementia, defects of memory, disorder of consciousness, amnesia, anxiety symptom, catatonic symptom, disconform mental state, psychopathy, (depression, epilepsy, alcoholism, etc.), inflammatory diseases (diabetic complication such as retinopathy, nephropathy, neuropathy great vessel dysfunction; arthritis such as rheumatoid arthritis, osteoarthritis, rheumatoid myelitis, periostitis; inflammation after operation and injury; remission of swelling; pharyngitis; cystitis; pneumonia; atopic dermatitis; inflammatory intestinal diseases such as Crohn's disease, ulcerative colitis; meningitis; inflammatory ocular disease; inflammatory pulmonary diseases such as pneumonia, pulmonary silicosis, pulmonary sarcoidosis, pulmonary tuberculosis, etc.), allergic diseases (allergic coryza, conjunctivitis, gastrointestinal allergy, pollinosis, anaphylaxis, etc.), chronic obstructive pulmonary disease, interstitial pneumonia, pneumocytis carinni pneumonia, collagen diseases (e.g., systemic lupus erythematosus, scleroderma, polyarteritis, etc.), hepatic diseases (hepatitis including chronic hepatitis, hepatic cirrhosis, etc.), portal hypertension, digestive system disorder (gastritis, gastric ulcer, stomach cancer, stomach disorder after operation, dyspepsia, esophageal ulcer, pancreatitis, colon polyp, cholelithiasis, hemorrhoidal disease, etc.), blood and/or myelopoietic diseases (erythrocytosis, vascular purpura, autoimmune hemolytic anemia, disseminated intravascular coagulation, multiple myelopathy, etc.), bone diseases (fracture, refracture, osteoporosis, osteomalacia, bone Behcet's disease, sclerosing myelitis, rheumatoid arthritis, osteoarthrosis of the knee and joint tissue distruction owing to similar diseases and disorder, etc.), solid ulcer, ulcer [malignant melanoma, malignant lymphoma, cancer of digestive organs (e.g., cancer of stomach, intestine, etc.)], cancer and cachexia following cencer, metastasis cancer, endocrinopathy (Addison's disease, Cushing's syndrome, pheochromocytoma, primary aldosteronism and the like), Creutzfeldt-Jakob disease, urinary organ and/or male genital diseases (cystitis, prostatic hypertrophy, prostatic cancer, sex infectious disease, etc.), female disorder (climacteric disorder, gestosis, endometriosis, hysteromyoma, ovarian disease, breast disease, sex infectious disease, etc.), diseases and disorder related to environment and occupational factors (radiation hazard, hazard by ultraviolet, infrared, or laser beam, altitude sickness, etc.), respiratory diseases (cold syndrome, pneumonia, asthma, pulmonary hypertension, pulmonary thromboembolism, pulmonary thrombosis, etc.), infectious diseases (viral infectious disease by cytomegalovirus, influenza virus, herpes virus, etc., rikettsiosis, bacterial infectious diseases, etc.), toxemia (sepsis, sepsis shock, endotoxin shock, Gram-negative supsis, toxin shock syndrome, etc.), rhinolarygological diseases (Meniere's syndrome, tinnitus, dysgeusia, vertigo, disequilibrium, dysphagia, etc.), skin diseases (keloid, hemangioma, psoriasis, etc.), intradialytic hypotension, myasthenia gravis, chronic fatigue syndrome, and the like.

In case the nonpeptidic physiologically active substance is a compound having angiotensin II antagonistic activity, by suppressing the action of angiotensin II for a long term, disorder and abnormality of biological and physiological functions which cause a variety of diseases accompanying adult disorder and aging can be improved, or accentuation of such disorder and abnormality can be controlled, thereby achieving primary and secondary prophylactic effects or controlling development of disease conditions caused by those diseases and symptoms. Such disorder and abnormality of biological and physiological functions include disorder and abnormality of automatic controlling capability of cerebral circulation and/or renal circulation, disorder of circulation (peripheral, cerebral, microcirculation and the like), disorder of blood-brain-barrier, insulin susceptibility deterioration, salt susceptibility deterioration, abnormal state of coagulation and fibrinolysis system, abnormal state of blood and blood cell components (accentuation of platelet agglutinability, abnormality of erythrocyte transformation, accentuation of leukocyte agglutininig function, rise of blood viscosity, etc.), production and function accentuation of growth factor and cytokine (PDGF, VEGF, FGF, interleukin, TNF-α, MCP-1, etc.), accentuation of proliferation and infiltration of inflammatory cells, accentuation of production of free radical, liposteatosis accentuation, endothelial function disorder, endothelial, cell, and organ dysfunction, cell morphogenesis change of edema, smooth muscle, and the like (morphogenesis to proliferation type), accentuation of production and function of vasoactive substance and thrombosis inducers (endothelin, thromboxane A₂, etc.), abnormal vasoconstriction, impaired glucose tolerance, metabolic disorder (serum lipid disorder, blood sugar disorder, etc.), abnormal cell propagation, vascular rebirth (including abnormal vasa vasorum formation in adventitial coat of atherosclerosis, abnormal capillary reticular formation), and the like. Among them, the compound can be advantageously used as a primary and secondary prophylactic drug for organ dysfunction accompanied with a variety of diseases (e.g. cerebrovascular disorder and organ dysfunction following the cerebrovascular disorder, organ dysfunction following cardiovascular diseases, organ dysfunction following diabetes mellitus, organ dysfunction after intervention).

In the sustained-release solid pharmaceutical composition of the present invention, when the nonpeptidic physiologically active substance is a compound having angiotensin II antagonistic activity (especially, candesartan cilexetil, candesartan, etc.), it can be advantageously used as a prophylactic and therapeutic drug for portal hypertension. It is well known that esophagovaritosis occurs frequently during night (Hepatology 1994; 19: 595-601), and since the preparation of the present invention is capable of maintaining a constant blood level all day long, the preparation of the present invention is not only capable of decreasing the dosage and the number of administration as compared with those in case of a preparation for oral administration, but also expected to realize stable lowering of portal vein pressure owing to the slight fluctuation of the blood level of the drug. The above characteristics of the preparation show usefulness as a preventive drug for rupture of varicose vein in esophagus and stomach. Further, since no symptom change is caused owing to interruption of the agent administration, it is also expected to clarify the therapeutic effect. Further, a compound having angiotensin II antagonistic activity as the physiologically active compound (especially, candesartan cilexetil, candesartan) is expected to be efficacious on the production and promotion of HGF (hepatocyte growth factor) and to attribute to liver regeneration and liver function restitution.

Further, it is expected to be able to further clarify the prophylactic and therapeutic efficacy on the cerebrovascular disorder such as cerebral infarct, etc. by maintaining a constant blood level of a compound having angiotensin II antagonistic activity as the nonpeptidic physiologically active substance (especially, candesartan cilexetil, candesartan) all day long. In case the nonpeptidic physiologically active substance is a compound having angiotensin II antagonistic activity, as a method for treating a patient, it can also be thought that a preparation for oral administration of the angiotensin II antagonist is firstly administered to the patient for a certain period to confirm the reaction of the patient, and then the sustained-release preparation of the present invention is administered. The angiotensin II antagonist to be used for the preparation for the oral administration may be the same as or different from the angiotensin II antagonist contained in the sustained-release preparation. In addition, a hypotensive other than the angiotensin II antagonist (e.g. calcium antagonist, diuretic, β-blocker, etc.) is firstly administered orally to confirm the reaction of a patient, and then the sustained-release preparation of the present invention can be administered. Further, a diuretic hypotensive drug (a preparation for oral administration) which is commonly used in combination with the angiotensin II antagonist may be used in combination with the sustained-release solid pharmaceutical composition of the present invention.

Further, the sustained-release solid preparation may be used in combination with other medical components including lipid decreasing drug or cholesterol decreasing drug, HMG-CoA reductase (3-hydroxy-3-methylglutaryl coenzyme A reductase) inhibitor, insulin sensitivity improving drug, bone disease therapeutic drug, myocardial protection drug, coronary arterial disease therapeutic drug, other hypertension therapeutic drug, chronic heart failure therapeutic drug, diabetic mellitus therapeutic drug, liver disease therapeutic drug, intestine and/or duodenal ulcer therapeutic drug, biliary disorder therapeutic drug, dysthyroidism therapeutic drug, nephrosis syndrome therapeutic drug, chronic renal failure therapeutic drug, female disease therapeutic drug, urinary organs and/or male genital disease therapeutic drug, and infection therapeutic drug. In this case, these compounds may be administered in the form of an oral preparation and in some cases, in the form of a rectal preparation or suppository. In this case, examples of components which can be used in combination include fibrates (e.g., clofibrate, benzafibrate, GM fibrozyl, etc.), nicotinic acid and its derivatives and analogues (e.g., acipimox, probucol), bile acid-conjugated resin (e.g. cholestyramine, questinol, etc.), compounds suppressing cholesterol absorption (e.g., sitosterol, neomycin, etc.) squalene epoxidase inhibitor (e.g., NB-598 and analogous compounds, etc.), and the like.

Examples of other components which can be used in combination include oxidosqualene-lanosterolcyclase such as decaline derivatives, azadecaline derivatives, and indane derivatives.

Furthermore, combinations with the following various therapeutic drugs are also possible.
Hypertension therapeutic drugs: diuretic [e.g., furosemide (Lasix), bemetanid (Lunetoron), azosemido (Diart)]; hypotensive drug [e.g., ACE inhibitor (enalapril maleate (Renivace), etc.), Ca antagonist (manidipine, amlodipine), α- or β-receptor inhibitor, etc.] and the like;
Chronic heart failure therapeutic drugs: cardiotonic [e.g., cardiotonic glycoside (Digoxin, etc.), β-receptor stimulus agent (catecholamin preparation such as denopamine and dobutamine), PDE inhibitor. etc.], diuretic agent [e.g., furosemide (Lasix), spironolactone (Aldacton), etc.], ACE inhibitor [e.g. enalapril maleate (Renivace), etc.], Ca antagonist (e.g., amlodipine), β-receptor inhibitor, and the like;
Antiarryhthmic drugs: disopyramide, lidocaine, quinidine sulfate, flecainide acetate, mexiletine hydrochloride, amiodarone, β-blocker, Ca antagonist, and the like;
Bone disease therapeutic drugs: calcium preparation (e.g. calcium carbonate, etc.), calcitonine preparation, activated type vitamin D₃ preparation [e.g., alfacalcidol (Alfarol), calcitriol (Rocaltrol), etc.], sex hormones (e.g., estrogen, estradiol, etc.), hormone preparation [e.g., estrogen conjugated (Premarin), etc.], ipriflavone preparation (Osten, etc.), vitamin K₂, vitamin K₂ preparation [e.g., menatetrenone (Glakay), etc.], bisphosphonate preparation (etidronate, etc.), prostaglandin E2, fluorine compound (e.g., sodium fluoride, etc.), bone matrix protein (BMP), fibroblast growth Factor (FGF), platelet-derived growth factor (PDGF), transforming growth factor (TGF-β), insulin-like growth factor 1 and 2 (IGF-1, -2), parathyroid hormone (PTH), compounds disclosed in EP-A1-376197, EP-A1-460488, and EP-A1-719782 [e.g., (2R,4S)-(-)-N-[4-(diethoxyphosphorylmethyl)phenyl]-1,2,4,5-tetrahydro-4-methyl-7,8-methylenedioxy-5-oxo-3-benzothiepin-2-carboxyamide, etc.], and the like;
Diabetes mellitus therapeutic drugs; Actos, rosiglitazone, Kinedak, Penfill, Humulin, Euglucon, Glimicron, Daonil, Novolin, Monotard, insulins, Glucobay, Dimelin, Rastinon, versylcone, Deamelin-S, Iszilin, and the like;
Liver disease therapeutic drugs: glycyrrhizin preparation (e.g., Strong Minophagen, etc.), liver lysosome, SH compound (e.g., glutathione, etc.), special amino acid preparation (e.g., Aminoleban, etc.), phospholipid (e.g., polyenephosphatidyl choline, etc.), vitamins (e.g., vitamin B₁, B₂, B₆, B₁₂, C, etc.), adrenocortical hormone (e.g., dexamethasone, betamethasone, etc.), interferon (e.g., interferon α, β, etc.), hepatic cerebropathy therapeutic drug (e.g., lactulose, etc.), hemostatic to be employed in esophagus and/or gastric varicosis rupture (e.g., vasopressin, somatostatin, etc.), and the like;
Gastric and/or duodenal ulcer therapeutic drugs: antiacid [e.g., histamine H2 receptor antagonists (cimetidine, etc.), proton pump inhibitor (lansoprazole, etc.), and the like;
Biliary tract therapeutic drugs: choleretic (e.g., dehydrocholic acid, etc.), cholekinetic (e.g., magnesium sulfate, etc.), and the like;
Hypothyroidism therapeutic drugs: dry thyroidea (Thyreoid), levothyroxine sodium (Thyradin-S), liothyronine sodium (Thyronin, Thyronamin), and the like;
Nephritic syndrome therapeutic drugs: for steroid therapy which is normally employed as primarily selection, prednisolon (Predonine), prednisolone sodium succinate (Predonine), methylprednisolone sodium succinate Solumedrol), betamethasone (Rinderone), and the like are used; and for anticoagulation therapy, dipyridamole (Persantin), dilazep dihydrochloride (Comelian), teclopidine, clopidogrel, antiplatelet drug such as Fxa inhibitor, etc. and anticoagulant are used;
HMG-CoA reductase inhibitors: cerivastatin, atorvastatin, provastatin, simvastatin, itavastatin, lovastatin, fluvastatin, (+)-3R,5S-7-(4-(4-fluorophenyl)-6-isopropyl-2-(N-methyl-N-methanesulfonylamino)pyridin-5-yl)-3,5-dihydroxy-6(E)-heptenoic acid, and the like;
Chronic renal failure therapeutic drugs: usable in combination with diuretic agent [e.g., furosemide (Lasix), bumetanid (Lunetoron), azosemido (Diart)], hypotensive drug [e.g., ACE inhibitor, enalapril maleate (Renivace)], calcium antagonist (manidipine) and α-adrenergic receptor antagonist, preferably by oral administration at the time of administration;
Thrombosis prophylactic drugs: anticoagulant [e.g., heparin sodium, heparin calcium, warfarin potassium (warfarin), blood coagulation factor Xa inhibitor and drugs capable of correcting balancing function of coagulation system], thrombolytic (e.g., tPA, urokinase), antiplatelet [e.g., aspirin, sulfinpyrazon (Anturan), dipyridamole (Persantin), ticropidine (Panaldine), cilostazol (Pletaal), GPIIb/IIIa antagonist (ReoPro)], and the like;
Coronary vasodilators: nifedipine, diltiazem, nicorandil, nitrite agent, and the like;
Cardioplegia drugs: opening drug for heart ATP-K, Na-H antiporters inhibitor, endothelin antagonist, urotensin antagonist, and the like;
Anti-inflammatory drugs: aspirin, acetaminophen, nonsteroidal anti-inflammatory drug (e.g., indometacine, etc.), steroid agent (e.g., dexamethasone, etc.), and the like;
Antiallergic drugs: antihistamine drug (e.g., chloropheniramine maleate, etc.), drug for stimulus therapy (e.g., bucillamine, etc.), azelastine hydrochloride, seratrodast, tranilast, oxatomide, Stronger Neo Minophagen C, tranexamic acid, ketotifen fumarate, and the like;
Antineoplastic drugs: alkylating agent, metabolic antagonist, antitumor antibiotic preparation, antitumor plant-derived component preparation, other antitumor drugs, and the like,
Central nervous system active drugs: anxiolytic, sleep sedative, anesthetic, antispasmodic, autonomic drug, drug for Parkinson's disease, drug for psychoneurosis, and the like;
Therapeutic drugs for female diseases: [e.g., therapeutic drug for climacteric disorder (estrogen conjugated, estradiol, testosterone enanthate, estradiol valerate, etc.), breast cancer therapeutic drug (tamoxifen citrate, etc.), endometriosis and/or myeoma uteri therapeutic drug (leuprorelin acetate, danazol, etc.)], and the like;
Urinary organ and/or male genital disease therapeutic drugs: [e.g., prostatic hypertrophy (tamsulosin hydrochloride, prazosin hydrochloride, chlormadinone acetate, etc.), prostatic cancer (leuprorelin acetate, goserelin acetate, chlormadinone acetate, etc.)], and the like;
Infectious disease therapeutic drugs: [e.g., antibiotic preparation (cefotiam dihydrochloride, cefozopran hydrochloride, ampicillin, etc.), chemotherapeutic drug (sulfonamido agent, synthetic antibacterial agent, antiviral substance, etc.), biological preparation (vaccines, blood derivatives such as immunogloblin, etc.), and the like]; and others.

Further, there are medical care by introduction of living body-derived various factors and their gene (e.g. ischemic disease therapeutic by vascular regeneration promoting factors such as HGF, VEGF and their gene introduction, etc.), care by decoy nucleic acids and the like.

In case of using the sustained-release solid pharmaceutical composition of the present invention in combination with these medical components, respective drugs may be incorporated into the single sustained-release preparation. Alternatively, the above-described medical components can be compounded with pharmacologically acceptable carriers, fillers, binders, diluent and the like to prepare respective preparations, and they are separately or simultaneously administered in combination with the sustained-release solid pharmaceutical composition of the present invention. In case preparations of these medical components are separately produced, the respective preparations can be mixed using a diluent upon administration, but the respective separate preparations can also be administered to a patient simultaneously or at certain time intervals.

Hereinafter, the present invention will be illustrated more in details with the reference to Reference Example, Examples and Experiments, but the present invention is not limited thereto.

### Examples

### Reference Example 1

Increase in solubility of poorly water-soluble nonpeptidic physiologically active compound by addition of metal compound

An amount of a solvent (dichloromethane/methanol=7/3) which can dissolve 0.1g of 2-ethoxy-1-[[2'-(1H-tetrazol-5-yl)biphenyl-4-yl]methyl]benzimidazole-7-carboxylic acid (hereinafter, abbreviated as Compound A) is shown in Table 1.

**Table 1**

| Metal compound added | Molecular weight of metal compound | Amount added | Amount of solvent |
|---|---|---|---|
| - | - | - | 3 mL |
| Zinc acetate dihydrate | 219.51 | 49.8 mg | 0.5 mL |
| Magnesium acetate tetrahydrate | 214.46 | 48.7 mg | 0.5 mL |

### Example 1

Two g of Compound A and 0.996 g of zinc acetate dihydrate (manufactured by Wako Pure Chemical Industries, Ltd.) were added to a solution in which 3.67 g of polylactic acid (weight average molecular weight 14,500; manufactured by Wako Pure Chemical Industries, Ltd.) had been dissolved in 7.5 ml of dichloromethane and 3.5 ml of methanol, and the mixture was shaken and stirred at room temperature for 2 hours to obtain a homogeneous solution. This solution was poured into 800 ml of a 0.1% by weight aqueous polyvinyl alcohol solution which had been regulated at 15°C, and an O/W emulsion was formed therefrom at 7,000 rpm using a turbine-type homomixer. This O/W emulsion was stirred at room temperature for 3 hours to volatilize dichloromethane and methanol, the oil phase was solidified to prepare microcapsules, which were collected them at 2,000 rpm using a centrifuge. They were dispersed again into distilled water, and centrifuged to wash a released drug, etc. The collected microcapsules were dispersed by addition of distilled water in which a small amount of mannitol had been dissolved, and lyophilized to obtain a powder. The encapsulation ratio of Compound A into the microcapsules was 101.6%, and the content of Compound A in the microcapsules was 26.6%.

### Example 2

According to the same manner as that of Example 1, microcapsules were obtained except that 2 g of Compound A, 0.757 g of zinc acetate dihydrate (manufactured by Wako Pure Chemical Industries, Ltd.) and 0.277 g of zinc oxide (TYPE V manufactured by Wako Pure Chemical Industries, Ltd.) were added to a solution in which 3.64 g of polylactic acid (weight average molecular weight 14,500; manufactured by Wako Pure Chemical Industries, Ltd.) had been dissolved in 7.5 ml of dichloromethane and 3.5ml of methanol, to obtain a homogeneous solution. The encapsulation ratio of Compound A into the microcapsules was 101.9%, and the content of Compound A in the microcapsules was 25.9%.

### Example 3

Two g of Compound A and 0.996 g of zinc acetate (manufactured by Wako Pure Chemical Industries, Ltd.) were added to a solution in which 3 g of a lactic acid-glycolic acid copolymer (lactic acid/glycolic acid 75/25 (mol%), weight average molecular weight 10,600; manufactured by Wako Pure Chemical Industries, Ltd.) had been dissolved in 7 ml of dichloromethane and 3 ml of methanol, to obtain a homogeneous solution. This was cast into a circle of 5 cm radius on a flat plate, and dried at room temperature for 16 hours under reduced pressure to obtain a dried material. This dried material was coarsely ground and classified on a sieve having a pore diameter of 150 mm to obtain a dried material, 3.6 g of which and 0.4 g of mannitol were mixed, and air-ground at air pressure of 2 kg/cm² with a jet mill apparatus (A-OJET; manufactured by Seishinkigyo) to obtain fine particles having an average particle diameter of 21 mm. The content of Compound A in the fine particles was 29.1%.

### Experimental Example 1

Twenty five mg of respective microcapsules obtained in Examples 1 and 2 were dispersed into 0.15 ml of a dispersing medium (solution in which 5 mg of sodium carboxymethylcellulose, 1 mg of Polysorbate 80 and 50 mg of mannitol were dissolved in 1 ml of distilled water), and the dispersion was subcutaneously administered to 7 week-old male SD rats at the neck back sites with a 20G injection needle. After administration, rats were slaughtered by bleeding from abdominal aorta in course of time, the microcapsules remaining at the administration site were taken out, and the compound therein was quantitated to calculate a remaining ratio of the compound. The remaining ratio is shown in Table 2.

**Table 2**

| Average remaining ratio of compound after subcutaneous administration of microcapsules (n=2 to 3) | | | | | |
|---|---|---|---|---|---|
| | After 1 day | After 1 week | After 2 weeks | After 3 weeks | After 4 weeks |
| Example 1 | 90.0% | 70.3% | 52.7% | 40.4% | 22.2% |
| Example 2 | 96.6% | 83.0% | 56.3% | 24.1% | 10.8% |

### Comparative Example 1

Two g of 2-ethoxy-1-[[2'-(1H-terazol-5-yl)diphenyl-4-]methyl]benzimidazole-7-carboxylic acid (hereinafter, abbreviated as Compound A) and 3.97 g of a lactic acid-glycolic acid copolymer (lactic acid/glycolic acid 75/25 (mol%), weight average molecular weight 8,700; manufactured by Wako Pure Chemical Industries, Ltd.) were added to a mixed solution of 12.75 ml of dichloromethane, 2.25 ml of methanol and 0.136 mL of acetic acid, and the mixture was shaken and stirred at room temperature overnight to obtain a suspension. This suspension was poured into 800 ml of a 0.1% by weight aqueous polyvinyl alcohol solution which had been regulated at 18°C, and an S/O/W emulsion was formed therefrom at 7,000 rpm using a turbine-type homomixer. This S/O/W emulsion was stirred at room temperature for 3 hours to volatilize dichloromethane, methanol and acetic acid, whereby, the polymer was solidified to prepare microcapsules, which were collected at 3,000rpm using a centrifuge. They were dispersed again into distilled water, and were further centrifuged to wash a released drug, etc. The collected microcapsules were lyophilized to obtain a powder. The encapsulation ratio of Compound A into the microcapsules was 100%.

### Comparative Example 2

Two g of Compound A, 0.37 g of zinc oxide (manufactured by Hakusui Kagaku Kougyo) and 3.6 g of a lactic acid-glycolic acid copolymer (lactic acid-glycolic acid 75/25 (mol%), weight average molecular weight 8,700; manufactured by Wako Pure Chemical Industries, Ltd.) were added to a mixed solution of 12.75 ml of dichloromethane, 2.25 ml of methanol and 0.136 mL of acetic acid, and shaken and stirred at room temperature overnight to obtain a homogeneous solution. This solution was poured into 800 ml of a 0.1% by weight aqueous polyvinyl alcohol solution which had been regulated at 18°C, and an O/W emulsion was formed therefrom at 7,000 rpm using a turbine-type homomixer. This O/W emulsion was stirred at room temperature for 3 hours to volatilize dichloromethane, methanol and acetic acid, whereby, the polymer was solidified to prepare microcapsules, which were collected at 3,000rpm using a centrifuge. They were dispersed again into distilled water and further centrifuged to wash a released drug, etc. The collected microcapsules were dispersed again by addition of distilled water in which 0.8 g of mannitol had been dissolved, and lyophilized to obtain a powder. The encapsulation ratio of Compound A into the microcapsules was 90.5%, and the content of Compound A in the microcapsule/mannitol powder was 26.4%.

### Comparative Example 3

One-point-five g of Compound A, 0.278 g of zinc oxide (manufactured by Hakusui Kagaku Kogyo) and 2.7 g of a lactic acid-glycolic acid copolymer (lactic acid/glycolic acid 75/25 (mol%), weight average molecular weight 10,500; manufactured by Wako Pure Chemical Industries, Ltd.) was added to a mixed solution of 11.25 ml of dichloromethane, 1.69 ml of methanol and 0.102 mL of acetic acid, the mixture was shaken and stirred at room temperature overnight to obtain a homogeneous solution. This solution was poured into 800 ml of a 0.1% by weight aqueous polyvinyl alcohol solution which had been regulated at 18°C, and an O/W emulsion was formed therefrom at 7,000 rpm using a turbine-type homomixer. This O/W emulsion was stirred at room temperature for 3 hours to volatilize dichloromethane methanol and acetic acid and solidify the polymer to prepare microcapsules, which were collected at 3,000 rpm using a centrifuge. They were dispersed again into distilled water, the dispersion was centrifuged, and a released drug and the like were washed. The collected microcapsules were dispersed again by addition of distilled water in which 0.8 g of mannitol had been dissolved, and lyophilized to obtain a powder. The encapsulation ratio of Compound A into the microcapsules was 93.1%, and the content of Compound A in the microcapsule/mannitol powder was 26.8%.

### Example 4

Two g of Compound A and 3.97 g of a lactic acid-glycolic acid copolymer (lactic acid/glycolic acid 75/25 (mol%), weight average molecular weight 8,700; manufactured by Wako Pure Chemical Industries, Ltd.) were added to a mixed solution of 12.75 ml of dichloromethane, 2.25 ml of methanol and 0.136 mL of acetic acid, and the mixture was shaken and stirred at room temperature overnight to obtain a suspension. This suspension was poured into 800ml of a 0.1% by weight aqueous polyvinyl alcohol solution to which 30 mM zinc acetate had been added and which had been regulated at 18°C, and the mixture was formulated into an S/O/W emulsion at 7,000rpm using a turbine-type homomixier. This S/O/W emulsion was stirred at room temperature for 3 hours to volatilize dichloromethane, methanol and acetic acid, to solidify a polymer to prepare microcapsules, which were collected at 3,000 rpm using a centrifuge. They were dispersed again into distilled water, the dispersion was further centrifuged, and a released drug and the like were washed. The collected microcapsule was lyophilized to obtain a powder. The encapsulation ratio of Compound A into the microcapsules was 94.9%.

### Example 5

Two g of Compound A, 0.37 g of zinc oxide (manufactured by Hakusui Kagaku Kogyo) and 3.6 g of a lactic acid-glycolic acid copolymer (lactic acid/glycolic acid 75/25 (mol%), weight average molecular weight 8,700; manufactured by Wako Pure Chemical Industries, Ltd.) were added to a mixed solution of 12.75 ml of dichloromethane, 2.25 ml of methanol and 0.136 mL of acetic acid, and the mixture was shaken and stirred at room temperature overnight to obtain a uniform solution. This solution was poured into 800 ml of a 0.1% by weight aqueous polyvinyl alcohol solution to which 10 mM zinc acetate has been added and which had been regulated at 18°C, and an O/W emulsion was formed therefrom at 7,000 rpm using a turbine-type homomixer. This O/W emulsion was stirred at room temperature for 3 hours to volatilize dichloromethane, methanol and acetic acid to solidify an oil phase to prepare microcapsules, which were collected at 3,000 rpm using a centrifuge. They were dispersed again into distilled water, the dispersion was further centrifuge, and a released drug and the like were washed. The collected microcapsules were dispersed again by addition of distilled water in which 0.8g of mannitol had been dissolved, and lyophilized to obtain a powder. The encapsulation ratio of Compound A into the microcapsules was 90.7%, and the content of Compound A in the microcapsule/mannitol powder was 26.4%.

### Example 6

Two g of Compound A, 0.37 g of zinc oxide (manufactured by Hakusui Kagaku Kogyo) and 3.6 g of a lactic acid-glycolic acid copolymer (lactic acid/ glycolic acid 75/25 (mol%), weight average molecular weight 8,700; manufactured by Wako Pure Chemical Industries, Ltd.) were added to a mixed solution of 12.75 ml of dichloromethane, 2.25 ml of methanol and 0.136 mL of acetic acid, and the mixture was shaken and stirred at room temperature overnight to obtain a homogeneous solution. This solution was poured into 800ml of a 0.1% by weight aqueous polyvinyl alcohol solution to which 30 mM zinc acetate had been added and which had been regulated at 18°C, and an O/W emulsion was formed therefrom at 7,000rpm using a turbine-type homomixer. This O/W emulsion was stirred at room temperature for 3 hours to volatilize dichloromethane, methanol and acetic acid, to solidify an oil phase to prepare microcapsules, which were collected at 3,000rpm using a centrifuge. They were dispersed again into distilled water, the dispersion was further centrifuged, and a released drug and the like were washed. The collected microcapsules were dispersed again by addition of distilled water in which 0.8 g of mannitol had been dissolved, and lyophilized to obtain a powder. The encapsulation ratio of Compound A into the microcapsules was 92.2%, and the content of Compound A in the microcapsule/mannitol powder was 26.6%.

### Example 7

One-point-five g of Compound A, 0.278 g of zinc oxide (manufactured by Hakusui Kagaku Kogyo) and 2.7 g of a lactic acid-glycolic acid copolymer (lactic acid/glycolic acid 75/25 (mol%), weight average molecular weight 10,500; manufactured by Wako Pure Chemical Industries, Ltd.) were added to a mixed solution of 11.25 ml of dichloromethane, 1.69 ml of methanol and 0.102 mL of acetic acid, and the mixture was shaken and stirred at room temperature overnight to obtain a homogeneous solution. This solution was poured into 800ml of a 0.1% by weight aqueous polyvinyl alcohol solution to which 30 mM zinc acetate had been added and which had been regulating at 18°C, and an O/W emulsion was formed therefrom at 7,000 rpm using a turbine-type homomixer. This O/W emulsion was stirred at room temperature for 3 hours to volatilize dichloromethane, methanol and acetic acid, to solidify an oil phase to prepare microcapsules, which were collected at 3,000rpm using a centrifuge. This was dispersed again into distilled water, the dispersion was further centrifuged, and a released drug and the like were washed. The collected microcapsules were dispersed again by addition of distilled water in which 0.8 g of mannitol had been dissolved, and lyophilized to obtain a powder. The encapsulation ratio of Compound A into the microcapsules was 88.0%, and the content of Compound A in the microcapsule/mannitol powder was 25.4%.

### Example 8

Two g of Compound A, 0.37 g of zinc oxide (manufactured by Hakusui Kagaku Kogyo) and 3.6 g of a lactic acid-glycolic acid copolymer (lactic acid/glycolic acid 75/25 (mol%), weight average molecular weight 8,700; manufactured by Wako Pure Chemical Industries, Ltd.) were added to a mixed solution of 12.75 ml of dichloromethane, 2.25 ml of methanol and 0.136 mL of acetic acid, and the mixture was shaken and stirred at room temperature overnight to obtain a homogeneous solution. This solution was poured into 800 ml of a 0.1% by weight aqueous polyvinyl alcohol solution to which 10 mM zinc acetate had been added and which had been regulated at 18°C, and an O/W emulsion was formed therefrom at 7,000 rpm using a turbine-type homomixer. This O/W emulsion was stirred at room temperature for 3 hours to volatilize dichloromethane, methanol and acetic acid, to solidify the oil phase to prepare microcapsules, which were collected at 3,000 rpm using a centrifuge. They were dispersed again into distilled water, the dispersion was further centrifuged, and a released drug and the like were washed. The collected microcapsules were dispersed again by addition of distilled water in which 0.8 g of mannitol had been dissolved, and lyophilized to obtain a powder. The encapsulation rate of Compound A into the microcapsules was 89.1%, and the content of Compound A in the microcapsule/mannitol powder was 26.2%.

### Experimental Example 2

The zinc contents of respective microcapsules obtained in Comparative Examples 1, 2 and 3 and Examples 4, 5, 6 and 7 were measured by the following method. Namely, the microcapsules were ashed by heating and the ash was dissolved in hydrochloric acid to obtain a solution. The concentration of zinc in the solution was measured with an atomic-absorption spectrometer (Z-8000 polarized Zeeman atomic-absorption spectrometer; Hitachi Seisakusho). The value obtained by the measurement was corrected by the weighed microcapsule weight to calculate the content of zinc in the microcapsules. The content of zinc in the microcapsules is shown in Table 3.

**Table 3**

| | Content of zinc (%) |
|---|---|
| Comparative Example 1 | 0.0 |
| Example 4 | 1.4 |
| Comparative Example 2 | 4.4 |
| Example 5 | 4.7 |
| Example 6 | 4.8 |
| Comparative Example 3 | 4.6 |
| Example 7 | 4.7 |

In the microcapsules prepared by adding zinc acetate to the outer water phase, increase in the content of zinc by at least 0.1% or more was observed as compared with the microcapsules prepared without addition of zinc acetate.

### Experimental Example 3

About 5mg of respective microcapsules obtained in Comparative Example 1 and Example 4 were added to 10 mL of a release test solution (1/150 M phosphate buffer). The mixture was stirred and mixed at room temperature for 1 minute, and centrifuged at 3000 rpm for 10 minutes to obtain the supernatant. The content of Compound A in the resultant supernatant was measured by liquid chromatography. The in vitro initial release rate from respective microcapsules was calculated by dividing the amount of Compound A released into the release test solution which was calculated from the concentration of Compound A in the supernatant by the amount of Compound A in the microcapsules. The resulting in vitro initial release rate is shown in Table 4.

**Table 4**

| In vitro initial release rate from microcapsules | |
|---|---|
| | Initial release rate (%) |
| Comparative Example 1 | 15.8 |
| Example 4 | 4.3 |

Drug initial release from the microcapsules prepared by adding zinc acetate to the outer water phase was decreased to about 30% or less as compared with the microcapsule prepared by without addition of zinc acetate, and the characteristics possessed by the composition of the present invention were shown.

### Experimental Example 4

Twenty five mg of respective microcapsules obtained in Comparative Example 2 and Example 5 were dispersed in 0.2 ml of a dispersing medium (solution in which 5 mg of sodium carboxymethylcellulose, 1 mg of Polysorbate 80, and 50 mg of mannitol were dissolved in 1 ml of distilled water), and the dispersion was administered to 7 week-old male SD rats subcutaneously at the neck back sites with a 22G injection needle. After administration, the blood was taken from the rat's tail vein with time, and the concentration of the drug in the resulting plasma was measured. The area under the curve of drug vs time until 24 hours from administration of the microcapsules is shown in Table 5.

**Table 5**

| Area under the curve of blood-concentration vs time after subcutaneous administration of microcapsules (n=4) | |
|---|---|
| | AUC 0 to 24 h (ng hr/mL) |
| Comparative Example 2 | 19944 |
| Example 5 | 3432 |

Drug initial burst release from the microcapsules prepared by adding zinc acetate to the outer water phase was decreased to about 20% or less as compared with the microcapsule prepared by without addition of zinc acetate, and the characteristics possessed by the composition of the present invention were shown.

### Experimental Example 5

Twenty five mg of respective microcapsules obtained in Comparative Example 3 and Example 7 were dispersed into 0.2 ml of a dispersing medium (a solution in which 5 mg of sodium carboxymethylcellulose, 1 mg of Polysorbate 80, and 50 mg of mannitol were dissolved in 1 ml of distilled water), and the dispersion was administered to 7 weeks-old male SD rats subcutaneously at the neck back sites with a 22G injection needle. After administration, the blood was taken from the rat's tail vein with time, and the concentration of the drug in the obtained plasma was measured. The area under the curve of drug vs time until 24 hours from administration of the microcapsules is shown in Table 6.

**Table 6**

| Area under the curve of blood-level vs time after subcutaneous administration of microcapsules (n=4) | |
|---|---|
| | AUC 0 to 24 h (ng hr/mL) |
| Comparative Example 3 | 6772 |
| Example 7 | 2512 |

Drug initial burst release from the microcapsules prepared by adding zinc acetate to the outer water phase was decreased to about 40% or less as compared with the microcapsules prepared by without addition of zinc , and the characteristics possessed by the composition of the present invention were shown.

### Industrial applicability

(1) The process of the present invention is characterized by using the organic solvent solution containing the poorly water-soluble nonpeptidic physiologically active compound, the polyvalent metal compound and the biodegradable polymer, wherein the physiologically active compound is in an amount exceeding the solubility thereof in the biodegradable polymer and organic solvent in the absence of the polyvalent metal compound, and sterilization by filtration with a filter can be readily performed during the production process. Then, the process is suitable for producing a preparation for injection. In addition, since distribution of the poorly water-soluble nonpeptidic physiologically active compound in the resulting sustained-release preparation is uniform, the stable sustained-release effect can be obtained.
   In the case that the poorly water-soluble nonpeptidic physiologically active compound is a compound having angiotensin II antagonistic activity, a constant blood level thereof can be maintained and therefore the fluctuation of blood level thereof is less than the case of oral administration. Then, stable and continuous pharmacological activity can be expected. Accordingly, disease conditions hardly take a turn for the worse even in case of a group of patients with slight subjective symptoms who intentionally refuse to take a medicine such as taking at inconstant time, interruption of taking, etc. Thus, more clear therapeutic effect can be expected on not only hypertension, abnormality of diurnal blood pressure variation, heart diseases, (cardiac hypertrophy, heart failure, myocardial infraction, etc.), cerebrovascular disorder (asymptomatic cerebral infarction, transient cerebral ischemia, cerebral apoplexy, cerebrovascular dementia, hypertensive cerebropathy. etc.), ischemic peripheral circulatory disorder, cardiomyopathy, venous insufficiency, progression of cardiac insufficiency after myocardial infarction, sequelae of cerebrovascular disorder, and the like, but also diabetic complication, diabetic retinopathy, diabetic nephropathy, nephritis, glomerulonephritis, nephropathy owing to radiation, atherosclerosis, arteriosclerosis, vascular hypertrophy, vascular hypertrophy and obliteration after intervention, vascular reobstruction after bypass, polycythemia, hypertension, organ failure, vascular hypertrophy after transplantation, rejection after transplantation, high aldosteronism, glomerulosclerosis, renal failure, glaucoma, ocular hyperlipemia, hyperlipidemia, angina pectoris, aneurysm, coronary arteriosclerosis, cerebral arteriosclerosis, peripheral arteriosclerosis, thrombosis, central nervous system disorder, Alzheimer's disease, defects of memory, depression, amnesia, senile dementia, sensory disturbance, multiple organ failure, endothelial dysfunction, hypertensive tinnitus, Meniere's syndrome, following vertigo or scleroderma, or anxiety, tense and uncomfortable psychological state, digestion disturbance, autonomic nervous system disorder, myasthenia gravis, cancer and cancer-related diseases, and the like.
(2) In the sustained-release solid pharmaceutical composition of the present invention comprising the nonpeptidic physiologically active substance and the biodegradable polymer, when about 0.05% by weight or more based on the weight of the composition of a polyvalent metal is present on the surface of the composition, the initial burst release can be suppressed to 60% or less as compared with that in the absence of the polyvalent metal on the surface. When an excessive amount is released at an early stage, disadvantageous effects on a living body such as blood glucose reduction or blood pressure reduction is manifested in some cases, depending on a particular nonpeptidic physiologically active substance. Then, the suppression of such release is very advantageous.

In particular, when the nonpeptidic physiologically active substance is a compound having angiotensin II antagonistic activity, there is a possibility that a blood pressure is excessively reduced due to excessive drug release at an early stage and, thus, the initial burst release suppression has the great meaningfulness in view of inhibition of such excessive reduction of a blood pressure. In the case that the pharmaceutical composition of the present invention contains a compound having angiotensin II antagonistic activity, a constant blood level thereof can be maintained and therefore the fluctuation of blood level thereof is less than the case of oral administration. Then, stable and continuous pharmacological activity can be expected. Accordingly, disease conditions hardly take a turn for the worse even in case of a group of patients with slight subjective symptoms who intentionally refuse to take a medicine such as taking at inconstant time, interruption of taking, etc. Thus, more clear therapeutic effect can be expected on not only hypertension, abnormality of diurnal blood pressure variation, heart diseases, (cardiac hypertrophy, heart failure, myocardial infraction. etc.), cerebrovascular disorder (asymptomatic cerebral infarction, transient cerebral ischemia, cerebral apoplexy, cerebrovascular dementia, hypertensive cerebropathy. etc.), ischemic peripheral circulatory disorder, cardiomyopathy, venous insufficiency, progression of cardiac insufficiency after myocardial infarction, sequelae of cerebrovascular disorder, and the like, but also diabetic complication, diabetic retinopathy, diabetic nephropathy, nephritis, glomerulonephritis, nephropathy owing to radiation, atherosclerosis, arteriosclerosis, vascular hypertrophy, vascular hypertrophy and obliteration after intervention, vascular reobstruction after bypass, polycythemia, hypertension, organ failure, vascular hypertrophy after transplantation, rejection after transplantation, high aldosteronism, glomerulosclerosis, renal failure, glaucoma, ocular hyperlipemia, hyperlipidemia, angina pectoris, aneurysm, coronary arteriosclerosis, cerebral arteriosclerosis, peripheral arteriosclerosis, thrombosis, central nervous system disorder, Alzheimer's disease, defects of memory, depression, amnesia, senile dementia, sensory disturbance, multiple organ failure, endothelial dysfunction, hypertensive tinnitus, Meniere's syndrome, following vertigo or scleroderma, or anxiety, tense and uncomfortable psychological state, digestion disturbance, autonomic nervous system disorder, myasthenia gravis, cancer and cancer-related diseases, and the like.

## Claims

1. A process for producing a sustained-release preparation, which comprises removing a solvent from an organic solvent solution containing a poorly water-soluble nonpeptidic physiologically active compound, a polyvalent metal compound and a biodegradable polymer, said physiologically active compound dissolved in an amount exceeding the solubility thereof in the organic solvent solution of biodegradable polymer in the absence of the polyvalent metal compound.

2. The process according to claim 1, wherein a molecular weight of the poorly water-soluble nonpeptidic physiologically active compound is about 2,000 or lower.

3. The process according to claim 1, wherein the poorly water-soluble nonpeptidic physiologically active compound has a functional group which can form a complex salt with the polyvalent metal compound.

4. The process according to claim 3, wherein the functional group is a group which can donate a covalent electron pair.

5. The process according to claim 3, wherein the functional group is a group having one or more hetero atom(s) selected from an oxygen atom, a nitrogen atom and a sulfur atom.

6. The process according to claim 3, wherein the functional group is a group selected from (1) a carboxyl group, (2) an imidazolyl group, (3) a mercapto group, (4) an amino group, (5) a tetrazolyl group, (6) a trifluoromethanesulfonamido group, (7) a phosphono group, (8) a sulfo group and (9) an optionally substituted 5 to 7-membered monocyclic heterocyclic residue, which has one or more hetero atom(s) selected from an oxygen atom, a nitrogen atom and a sulfur atom.

7. The process according to claim 1, wherein the poorly water-soluble nonpeptidic physiologically active compound has a functional group having a pKa of about 2.5 to about 6.

8. The process according to claim 1, wherein the poorly water-soluble nonpeptidic physiologically active compound is a compound having angiotensin II antagonistic activity, a prodrug thereof, or a salt thereof.

9. The process according to claim 8, wherein the compound having angiotensin II antagonistic activity is a compound having an oxygen atom in the molecule.

10. The process according to claim 8, wherein the compound having angiotensin II antagonistic activity is a compound having an ether linkage or a carbonyl group.

11. The process according to claim 8, wherein the compound having angiotensin II antagonistic activity is a compound represented by the formula (I): 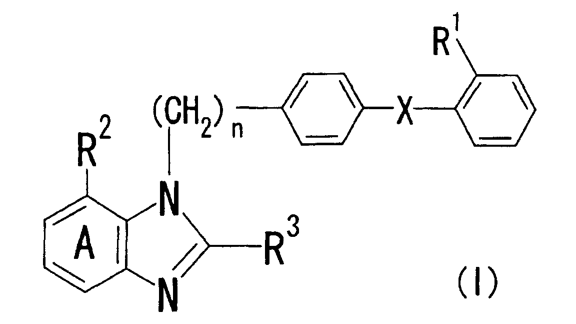 wherein R¹ denotes a group which can form an anion or a group which can be changed into such a group, X denotes a bond or a spacer having 2 or less atoms at the straight chain part thereof, n denotes 1 or 2, a ring A denotes a benzene ring which may be further substituted, R² denotes a group which can form an anion or a group which can be changed into such a group, and R³ denotes an optionally substituted hydrocarbon residue, wherein the hydrocarbon residue may bind via a hetero atom.

12. The process according to claim 8, wherein the compound having angiotensin II antagonistic activity, a prodrug thereof, or a salt thereof is Losartan, Losartan potassium, Eprosartan, Candesartan cilexetil, Candesartan, Valsartan, Telmisartan, Irbesartan, Olmesartan, Olmesartan medoxomil or Tasosartan.

13. The process according to claim 8, wherein the compound having angiotensin II antagonistic activity is 2-ethoxy-1-[[2'-(1H-tetrazol-5-yl)biphenyl-4-yl]methyl]benzimidazole-7-carboxylic acid.

14. The process according to claim 8, wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate.

15. The process according to claim 8, wherein the compound having angiotensin II antagonistic activity is 2-ethoxy-1-[[2'-(2,5-dihydro-5-oxo-1,2,4-oxadiazol-3-yl)biphenyl-4-yl]methyl]benzimidazole-7-carboxylic acid.

16. The process according to claim 1, wherein the biodegradable polymer is an α-hydroxycarboxylic acid polymer.

17. The process according to claim 16, wherein the α-hydroxycarboxylic acid polymer is a lactic acid-glycolic acid copolymer.

18. The process according to claim 17, wherein a molar ratio of lactic acid and glycolic acid in the lactic acid-glycolic acid copolymer is 100/0 to 40/60.

19. The process according to claim 16, wherein a weight average molecular weight of the polymer is 3,000 to 50,000.

20. The process according to claim 1, wherein the sustained-release preparation is for injection.

21. The process according to claim 1, wherein the polyvalent metal is zinc.

22. The process according to claim 21, wherein the polyvalent metal compound comprises one or two of zinc acetate and zinc oxide.

23. The process according to claim 1, wherein a molar ratio of the polyvalent metal compound relative to the poorly water-soluble nonpeptidic physiologically active compound in the organic solvent solution is 1/10 to 10/1.

24. The process according to claim 1, wherein the concentration of the poorly water-soluble nonpeptidic physiologically active compound in the organic solvent solution is about 0.5 to about 70% by weight.

25. The process according to claim 1, wherein the concentration of the biodegradable polymer in the organic solvent solution is about 0.5 to about 70% by weight.

26. A sustained-release preparation obtainable by the process according to claim 1.

27. The sustained-release preparation according to claim 26, wherein the poorly water-soluble nonpeptidic physiologically active compound is a compound having angiotensin II antagonistic activity, a prodrug thereof, or a salt thereof.

28. A medicine comprising the sustained-release preparation according to claim 26.

29. The medicine according to claim 28, which is an agent for preventing or treating circulatory diseases.

30. The medicine according to claim 28, which is an agent for preventing or treating hypertension, abnormality of diurnal blood pressure variation or organ dysfunction.

31. An organic solvent solution, which comprises a poorly water-soluble nonpeptidic physiologically active compound, a polyvalent metal compound and a biodegradable polymer, said physiologically active compound dissolved in an amount exceeding the solubility thereof in the organic solvent solution of a biodegradable polymer in the absence of the polyvalent metal compound.

32. Use of a polyvalent metal compound for increasing the solubility of a poorly water-soluble nonpeptidic physiologically active compound in an organic solvent solution of a biodegradable polymer.

33. A method for increasing the solubility of a poorly water-soluble nonpeptidic physiologically active compound in an organic solvent solution containing a biodegradable polymer, an organic solvent and the poorly water-soluble nonpeptidic physiologically active compound, which comprises using a polyvalent metal compound.

34. A sustained-release solid pharmaceutical composition comprising a nonpeptidic physiologically active substance and a biodegradable polymer, wherein about 0.05% by weight based on the weight of the composition, or more of a polyvalent metal is present on the surface of the composition.

35. The sustained-release solid pharmaceutical composition according to claim 34, wherein the initial burst release of the nonpeptidic physiologically active substance is suppressed at about 60% or less as compared with the absence of the polyvalent metal on the surface.

36. The sustained-release solid pharmaceutical composition according to claim 34, wherein a molecular weight of the nonpeptidic physiologically active substances is about 2,000 or less.

37. The sustained-release solid pharmaceutical composition according to claim 34, wherein the nonpeptidic physiologically active substance is a poorly water-soluble compound.

38. The sustained-release solid pharmaceutical composition according to claim 34, wherein the nonpeptidic physiologically active substance is a compound having angiotensin II antagonistic activity, a prodrug thereof, or a salt thereof.

39. The sustained-release solid pharmaceutical composition according to claim 38, wherein the compound having angiotensin II antagonistic activity is a compound having an oxygen atom in a molecule.

40. The sustained-release solid pharmaceutical composition according to claim 38, wherein the compound having angiotensin II antagonistic activity is a compound having an ether linkage or a carboxyl group.

41. The sustained-release solid pharmaceutical composition according to claim 38, wherein the compound having angiotensin II antagonistic activity is a compound represented by the formula (I): 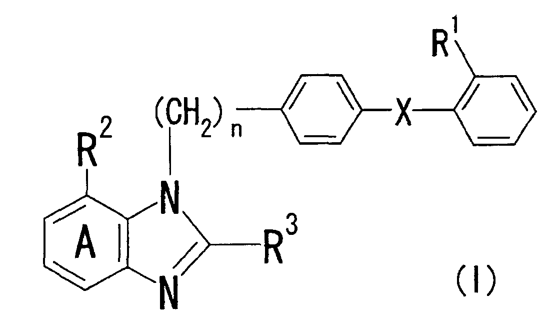 wherein R¹ denotes a group which can form an anion or a group which can be changed into such a group, X denotes a bond or a spacer having 2 or less atoms at the straight chain part thereof, n denotes 1 or 2, a ring A denotes a benzene ring which may be further substituted, R² denotes a group which can form an anion or a group which can be changed into such a group, and R³ denotes an optionally substituted hydrocarbon residue, wherein the hydrocarbon residue may bind via a hetero atom.

42. The sustained-release solid pharmaceutical composition according to claim 38, wherein the compound having angiotensin II antagonistic activity, a prodrug thereof, or a salt thereof is Losartan, Losartan potassium, Eprosartan, Candesartan cilexetil, Candesartan, Valsartan, Telmisartan, Irbesartan, Ormesartan, Ormesartan medoxomil or Tasosartan.

43. The sustained-release solid pharmaceutical composition according to claim 38, wherein the compound having angiotensin II antagonistic activity is 2-ethoxy-1-[[2'-(1H-tetrazol-5-yl)biphenyl-4-yl]methyl]benzimidazole-7-carboxylic acid.

44. The sustained release solid pharmaceutical composition according to claim 38, wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate.

45. The sustained-release solid pharmaceutical composition according to claim 38, wherein the compound having angiotensin II antagonistic activity is 2-ethoxy-1-[[2'-(2,5-dihydro-5-oxo-1,2,4-oxadiazol-3-yl)biphenyl-4-yl]methyl]benzimidazole-7-carboxylic acid.

46. The sustained-release solid pharmaceutical composition according to claim 34, wherein the biodegradable polymer is an α-hydroxycarboxylic acid polymer.

47. The sustained-release solid pharmaceutical composition according to claim 46, wherein the α-hydroxycarboxylic acid polymer is a lactic acid-glycolic acid copolymer.

48. The sustained-release solid pharmaceutical composition according to claim 47, wherein a molar ratio of lactic acid and glycolic acid in the lactic acid-glycolic acid copolymer is 100/0 to 40/60.

49. The sustained-release solid pharmaceutical composition according to claim 46, wherein a weight average molecular weight of the polymer is about 3,000 to about 50,000.

50. The sustained-release solid pharmaceutical composition according to claim 34, the polyvalent metal is a divalent metal.

51. The sustained-release solid pharmaceutical composition according to claim 50, wherein the divalent metal is zinc.

52. The sustained-release solid pharmaceutical composition according to claim 34, which is for injection.

53. The sustained-release solid pharmaceutical composition according to claim 34, which is microcapsules.

54. A process for producing the sustained-release solid pharmaceutical composition according to claim 34, which comprises removing an organic solvent from an emulsion obtained by mixing an organic solvent solution or suspension containing a nonpeptidic physiologically active substances and a biodegradable polymer, and a water phase containing polyvalent metal ion in concentration of about 0.1 to about 80 mM.

55. The process for producing the sustained-release solid pharmaceutical composition according to claim 34, which comprises removing an organic solvent from an emulsion obtained by mixing an organic solvent solution containing a nonpeptidic physiologically active substance, a polyvalent metal and a biodegradable polymer, said physiologically active substance dissolved in an amount exceeding the solubility thereof in the organic solvent solution of the biodegradable polymer in the absence of the polyvalent metal, and a water phase containing polyvalent metal ion in concentration of about 0.1 to about 80 mM.

56. The process according to claim 54 or 55, wherein the polyvalent metal ion is zinc.

57. The process according to claim 55, wherein the polyvalent metal is used as one or two of polyvalent metal compound(s) selected from zinc acetate and zinc oxide.

58. A sustained-release solid pharmaceutical composition obtainable by the process according to claim 54 or 55.

59. The sustained-release solid pharmaceutical composition according to claim 38, which is an agent for preventing or treating circulatory disease.

60. The sustained-release solid pharmaceutical composition according to claim 38, which is an agent for preventing or treating hypertension, abnormality of diurnal blood pressure variation or organ dysfunction.

61. A method of suppressing the initial burst release of a nonpeptidic physiologically active substance, which comprises, in a solid pharmaceutical composition containing a nonpeptidic physiologically active substance and a biodegradable polymer, allowing about 0.05% by weight, based on the weight of the composition, or more of a polyvalent metal to be present on the surface of the composition.
